# EUROPEAN PATENT APPLICATION

(11) **EP 4 446 812 A1**
(43) Date of publication of application: **16.10.2024**
(21) Application number: 24165781.6
(22) Date of filing: 25.03.2024
(51) Int. Cl.: G03F 7/004, G03F 7/039

(54) **ONIUM SALT, CHEMICALLY AMPLIFIED POSITIVE RESIST COMPOSITION, AND RESIST PATTERN FORMING PROCESS**

(30) Priority: 28.03.2023 JP 2023051139
(71) Applicant: Shin-Etsu Chemical Co., Ltd., Tokyo (JP)
(72) Inventor: FUKUSHIMA, Masahiro, Joetsu-shi (JP)
(74) Representative: Ter Meer Steinmeister & Partner

(57) **Abstract**

An alkanesulfone type onium salt whose anion has a bulky substituent at α-position of a sulfo group and a bulky aromatic ring structure is capable of generating an acid having an adequate acid strength and controlled diffusion. A chemically amplified positive resist composition comprising the onium salt is provided.

## Description

### TECHNICAL FIELD

This invention relates to an onium salt, a chemically amplified positive resist composition, and a resist pattern forming process.

### BACKGROUND ART

Pattern formation to a smaller feature size is required to meet the recent demand for higher integration in integrated circuits. Acid-catalyzed chemically amplified resist compositions are most often used in forming resist patterns with a feature size of 0.2 µm or less. High-energy radiation such as UV, deep-UV or EB is used as the energy source for exposure of these resist compositions. In particular, the EB lithography, which is utilized as the ultra-fine microfabrication technique, is also indispensable in processing a photomask blank into a photomask for use in the fabrication of semiconductor devices. Resist compositions for use in the EB lithography include positive ones wherein exposed regions are dissolved away to form a pattern and negative ones wherein exposed regions are retained to form a pattern. Either one which is easier to use is chosen in accordance with the morphology of the necessary resist pattern.

In general, the EB lithography is by writing an image with EB, without using a mask. In the case of positive resist, those regions of a resist film other than the regions to be retained are successively irradiated with EB having a minute area. In the case of negative resist, those regions of a resist film to be retained are successively irradiated with EB. The operation of successively scanning all finely divided regions on the work surface takes a long time as compared with full wafer exposure through a photomask. To prevent any throughput decline, a resist film having a high sensitivity is required. Because of a long image writing time, it is likely that a difference arises between an initially imaged portion and a lately imaged portion. The stability with time of the exposed portion in vacuum is one of the important performance factors. One of the important applications of chemically amplified resist material resides in processing of photomask blanks. Some photomask blanks have a surface material that can have an impact on the pattern profile of the overlying chemically amplified resist film, for example, a layer of a chromium compound, typically chromium oxide deposited on a photomask substrate. For high resolution and profile retention after etching, it is one important performance factor to maintain the profile of a resist film pattern rectangular independent of the type of substrate.

Attempts were made to ameliorate resist sensitivity and pattern profile in a controlled way by properly selecting and combining components used in resist compositions and adjusting processing conditions. One outstanding problem is the diffusion of acid, which has a significant impact on the resolution of a chemically amplified resist film. In the processing of photomasks, it is required that the profile of the resist pattern resulting from exposure does not change depending on the time taken until PEB. The major cause for time-dependent changes is the diffusion of acid generated upon exposure. Since the problem of acid diffusion has large impacts on sensitivity and resolution not only in the photomask processing, but also in general resist compositions, many studies are made thereon.

Patent Documents 1 and 2 describe acid generators capable of generating bulky acids upon exposure, for thereby controlling acid diffusion and reducing roughness. Since these acid generators are still insufficient to control acid diffusion, it is desired to have an acid generator with more controlled diffusion.

Patent Document 3 discloses a resist composition comprising a base polymer having bound thereto an acid generator capable of generating a sulfonic acid upon light exposure whereby acid diffusion is controlled. This approach of controlling acid diffusion by binding repeat units capable of generating acid upon exposure to a base polymer is effective in forming a pattern with reduced LER. However, the base polymer having bound therein repeat units capable of generating acid upon exposure encounters a problem with respect to its solubility in organic solvent, depending on the structure and proportion of the relevant units.

Polymers comprising a major proportion of aromatic structure having an acidic side chain, for example, polyhydroxystyrene are useful in resist materials for the KrF lithography. These polymers are not used in resist materials for the ArF lithography since they exhibit strong absorption at a wavelength of around 200 nm. These polymers, however, are expected to form useful resist materials for the EB and EUV lithography for forming patterns of smaller size than the processing limit of ArF lithography because they offer high etching resistance.

Often used as the base polymer in positive resist compositions for EB and EUV lithography is a polymer having an acidic functional group on phenol side chain masked with an acid labile group (or acid-decomposable protective group). Upon exposure to high-energy radiation, the acid labile group is deprotected by the catalysis of an acid generated from a photoacid generator so that the polymer may turn soluble in alkaline developer. Typical of the acid labile group are tertiary alkyl, tert-butoxycarbonyl, and acetal groups. The use of protective groups (e.g., acetal groups) requiring a relatively low level of activation energy for deprotection offers the advantage that a resist film having a high sensitivity is obtainable. However, if the diffusion of generated acid is not fully controlled, deprotection reaction can occur even in the unexposed region of the resist film, giving rise to problems like a degradation of LER and a lowering of in-plane uniformity (CDU) of pattern line width.

Patent Document 4 describes a resist composition comprising a polymer comprising repeat units having an acetal group and a sulfonium salt capable of generating an acid having a high acid strength and low pKa such as fluoroalkanesulfonic acid. The composition forms a pattern with noticeable LER. This is because the acid strength of fluoroalkanesulfonic acid is too high for the deprotection of the acetal group requiring a relatively low level of activation energy for deprotection. Even if acid diffusion is controlled, deprotection reaction can be promoted in the unexposed region by a minor amount of acid that has diffused thereto.

Patent Documents 5 and 6 describe photoacid generators capable of generating a non-fluorinated aromatic sulfonic acid having a plurality of bulky alkyl substituents. Since a plurality of alkyl substituents are introduced, the generated acid has a higher molecular weight, which is effective for suppressing acid diffusion. The control of acid diffusion is insufficient for the purpose of forming small-size patterns. There remains room for further improvement.

### Citation List

Patent Document 1: JP-A 2009-053518
Patent Document 2: JP-A 2010-100604
Patent Document 3: JP-A 2011-022564
Patent Document 4: JP 5083528
Patent Document 5: JP 6248882
Patent Document 6: JP-A 2019-202974

### DISCLOSURE OF INVENTION

Resist compositions are recently demanded which are capable of forming not only line- and-space (LS), isolated line (IL) and isolated space (IS) patterns of satisfactory profile, but also hole patterns of satisfactory profile. Patent Document 5 describes an acid generator capable of generating a bulky acid with controlled diffusion, which ensures to form patterns having satisfactory resolution and roughness, but the formation of hole patterns is accompanied with corner rounding.

An object of the invention is to provide an onium salt capable of generating an acid having an adequate acid strength and low diffusion, a chemically amplified positive resist composition comprising the onium salt, and a resist pattern forming process using the composition.

The inventors have found that when an alkanesulfone type onium salt containing an anion having a bulky substituent at α-position of a sulfo group and a bulky aromatic ring structure is added to a resist composition as an acid generator, the onium salt generates an acid having an adequate acidity. Since the anion structure is bulky enough to hinder rotation of the linking group, the generated acid is restrained from excessive diffusion. A pattern with satisfactory resolution and minimal LER is obtainable from the resist composition. A pattern of rectangular profile is obtainable by virtue of properly inhibited dissolution.

Accordingly, the invention provides an onium salt, chemically amplified positive resist composition, and resist pattern forming processs, as defined below.
1. An onium salt having the formula (A): 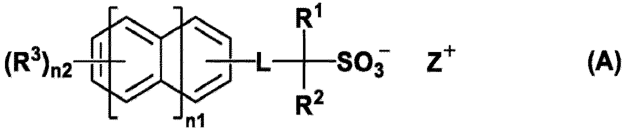 wherein n1 is an integer of 0 to 2, n2 is an integer of 2 to 5 in case of n1=0, an integer of 2 to 7 in case of n1=1, and an integer of 2 to 9 in case of n1=2,
   L is a single bond, ether bond, ester bond, sulfonic ester bond, carbonate bond or carbamate bond,
   R¹ and R² are each independently hydrogen or a C₃-C₂₀ branched or cyclic hydrocarbyl group which may contain a heteroatom, excluding that both R¹ and R² are hydrogen, R¹ and R² may bond together to form a ring with the carbon atom to which they are attached,
   R³ is each independently iodine or a C₃-C₂₀ branched or cyclic hydrocarbyl group which may contain a heteroatom, at least one R³ is attached to a carbon atom adjoining the carbon atom to which L is attached, and
   Z⁺ is an onium cation.
2. The onium salt of 1 having the formula (A1): 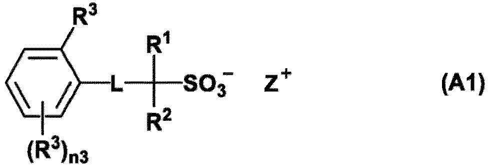 wherein L, R¹, R², R³, and Z⁺ are as defined above, and n3 is an integer of 1 to 4.
3. The onium salt of 2 having the formula (A2): 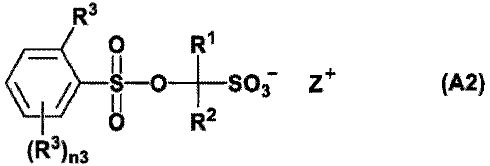 wherein n3, R¹, R², R³, and Z⁺ are as defined above.
4. The onium salt of 1 wherein Z⁺ is an onium cation having the formula (cation-1) or (cation-2): 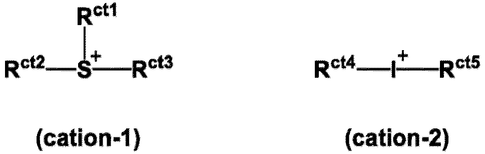 wherein R^{ct1} to R^{ct5} are each independently a C₁-C₃₀ hydrocarbyl group which may contain a heteroatom, and R^{ct1} and R^{ct2} may bond together to form a ring with the sulfur atom to which they are attached.
5. A photoacid generator comprising the onium salt of any one of 1 to 4.
6. A chemically amplified positive resist composition comprising the photoacid generator of 5.
7. The chemically amplified positive resist composition of 6, further comprising a base polymer containing a polymer which is decomposed under the action of acid to increase its solubility in alkaline developer.
8. The chemically amplified positive resist composition of 7 wherein the polymer comprises repeat units having the formula (B1): 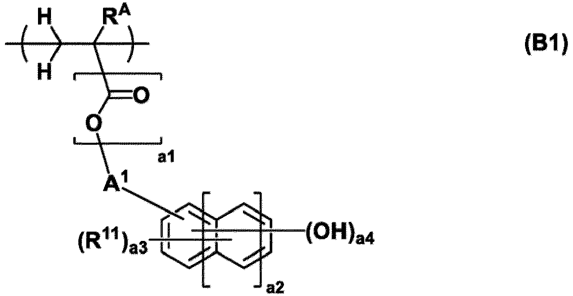 wherein a1 is 0 or 1, a2 is an integer of 0 to 2, a3 is an integer satisfying 0 ≤ a3 ≤ 5+2(a2)-a4, a4 is an integer of 1 to 3,
   R^{A} is hydrogen, fluorine, methyl or trifluoromethyl,
   R¹¹ is halogen, an optionally halogenated C₂-C₈ saturated hydrocarbylcarbonyloxy group, optionally halogenated C₁-C₆ saturated hydrocarbyl group, or optionally halogenated C₁-C₆ saturated hydrocarbyloxy group, and
   A¹ is a single bond or C₁-C₁₀ saturated hydrocarbylene group in which some constituent - CH₂- may be replaced by -O-.
9. The chemically amplified positive resist composition of 7 or 8 wherein the polymer further comprises repeat units having the formula (B2-1): 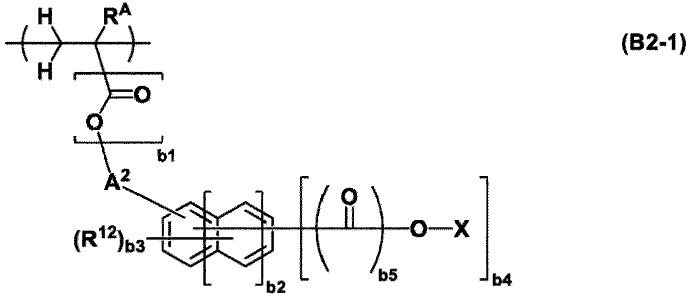 wherein b1 is 0 or 1, b2 is an integer of 0 to 2, b3 is an integer satisfying 0 ≤ b3 ≤ 5+2(b2)-b4, b4 is an integer of 1 to 3, b5 is 0 or 1,
   R^{A} is hydrogen, fluorine, methyl or trifluoromethyl,
   R¹² is halogen, an optionally halogenated C₂-C₈ saturated hydrocarbylcarbonyloxy group, optionally halogenated C₁-C₆ saturated hydrocarbyl group, or optionally halogenated C₁-C₆ saturated hydrocarbyloxy group,
   A² is a single bond or C₁-C₁₀ saturated hydrocarbylene group in which some constituent - CH₂- may be replaced by -O-,
   X is an acid labile group when b4 is 1, and X is each independently hydrogen or an acid labile group, at least one being an acid labile group, when b4 is 2 or 3.
10. The chemically amplified positive resist composition of any one of 7 to 9 wherein the polymer further comprises repeat units having the formula (B2-2): 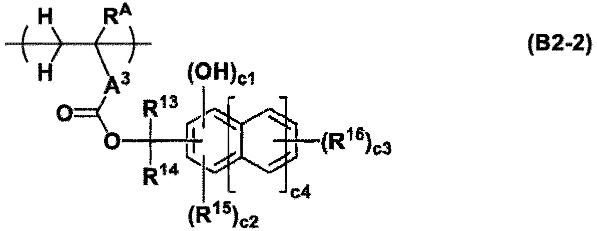 wherein c1 is an integer of 0 to 2, c2 is an integer of 0 to 2, c3 is an integer of 0 to 5, c4 is an integer of 0 to 2,
   R^{A} is hydrogen, fluorine, methyl or trifluoromethyl,
   R¹³ and R¹⁴ are each independently a C₁-C₁₀ hydrocarbyl group which may contain a heteroatom, R¹³ and R¹⁴ may bond together to form a ring with the carbon atom to which they are attached,
   R¹⁵ is each independently fluorine, C₁-C₅ fluorinated alkyl group or C₁-C₅ fluorinated alkoxy group,
   R¹⁶ is each independently a C₁-C₁₀ hydrocarbyl group which may contain a heteroatom, and
   A³ is a single bond, phenylene group, naphthylene group, or *-C(=O)-O-A³¹-, wherein A³¹ is a C₁-C₂₀ aliphatic hydrocarbylene group which may contain hydroxy, ether bond, ester bond or lactone ring, or a phenylene or naphthylene group, * designates a point of attachment to the carbon atom in the backbone.
11. The chemically amplified positive resist composition of any one of 7 to 10 wherein the polymer further comprises repeat units of at least one type selected from repeat units having the formula (B3), repeat units having the formula (B4), and repeat units having the formula (B5): 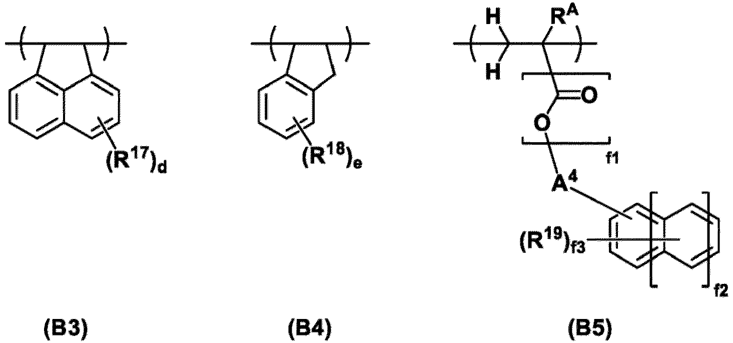 wherein d is an integer of 0 to 6, e is an integer of 0 to 4, f1 is 0 or 1, f2 is an integer of 0 to 2, and f3 is an integer of 0 to 5,
   R^{A} is hydrogen, fluorine, methyl or trifluoromethyl,
   R¹⁷ and R¹⁸ are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
   R¹⁹ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro group, or cyano group, R¹⁹ may be a hydroxy group when f2 is 1 or 2, and
   A⁴ is a single bond or C₁-C₁₀ saturated hydrocarbylene group in which some constituent - CH₂- may be replaced by -O-.
12. The chemically amplified positive resist composition of any one of 7 to 11 wherein the polymer further comprises repeat units of at least one type selected from repeat units having the formulae (B6) to (B13): 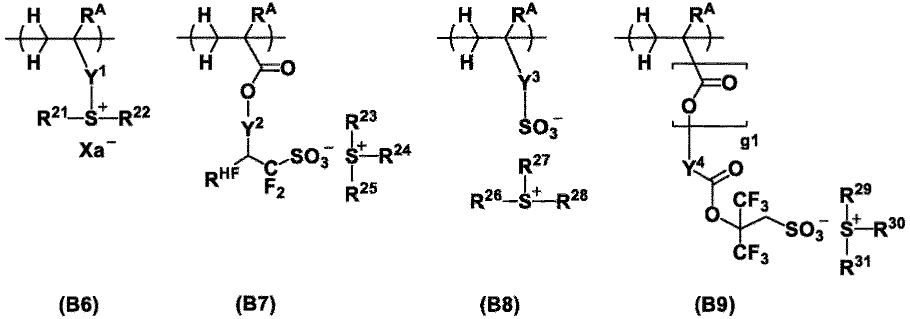 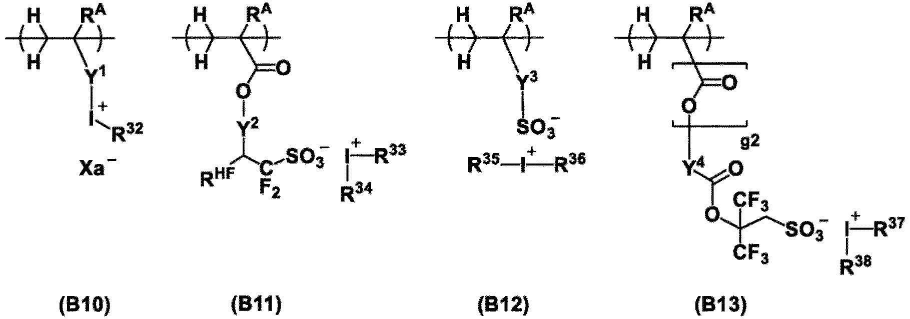 wherein R^{A} is each independently hydrogen, fluorine, methyl or trifluoromethyl,
   Y¹ is a single bond, a C₁-C₆ aliphatic hydrocarbylene group, phenylene group, naphthylene group or C₇-C₁₈ group obtained by combining the foregoing, *-O-Y¹¹-, *-C(=O)-O-Y¹¹-, or *-C(=O)-NH-Y¹¹-, Y¹¹ is a C₁-C₆ aliphatic hydrocarbylene group, phenylene group, naphthylene group or C₇-C₁₈ group obtained by combining the foregoing, which may contain a carbonyl moiety, ester bond, ether bond or hydroxy moiety,
   Y² is a single bond or **-Y²¹-C(=O)-O-, Y²¹ is a C₁-C₂₀ hydrocarbylene group which may contain a heteroatom,
   Y³ is a single bond, methylene, ethylene, phenylene, fluorinated phenylene, trifluoromethyl-substituted phenylene, *-O-Y³¹-, *-C(=O)-O-Y³¹-, or *-C(=O)-NH-Y³¹-, Y³¹ is a C₁-C₆ aliphatic hydrocarbylene group, phenylene group, fluorinated phenylene group, trifluoromethyl-substituted phenylene group, or C₇-C₂₀ group obtained by combining the foregoing, which may contain a carbonyl moiety, ester bond, ether bond or hydroxy moiety,
   * designates a point of attachment to the carbon atom in the backbone, ** designates a point of attachment to the oxygen in the formula,
   Y⁴ is a single bond or C₁-C₃₀ hydrocarbylene group which may contain a heteroatom,
   g1 and g2 are each independently 0 or 1, g1 and g2 are 0 when Y⁴ is a single bond,
   R²¹ to R³⁸ are each independently halogen or a C₁-C₂₀ hydrocarbyl group which may contain a heteroatom, R²¹ and R²² may bond together to form a ring with the sulfur atom to which they are attached, R²³ and R²⁴, R²⁶ and R²⁷, or R²⁹ and R³⁰ may bond together to form a ring with the sulfur atom to which they are attached,
   R^{HF} is hydrogen or trifluoromethyl, and
   Xa⁻ is a non-nucleophilic counter ion.
13. The chemically amplified positive resist composition of any one of 7 to 12, further comprising an organic solvent.
14. The chemically amplified positive resist composition of any one of 7 to 13, further comprising a fluorinated polymer comprising repeat units of at least one type selected from repeat units having the formula (D1), repeat units having the formula (D2), repeat units having the formula (D3) and repeat units having the formula (D4) and optionally repeat units of at least one type selected from repeat units having the formula (D5) and repeat units having the formula (D6): 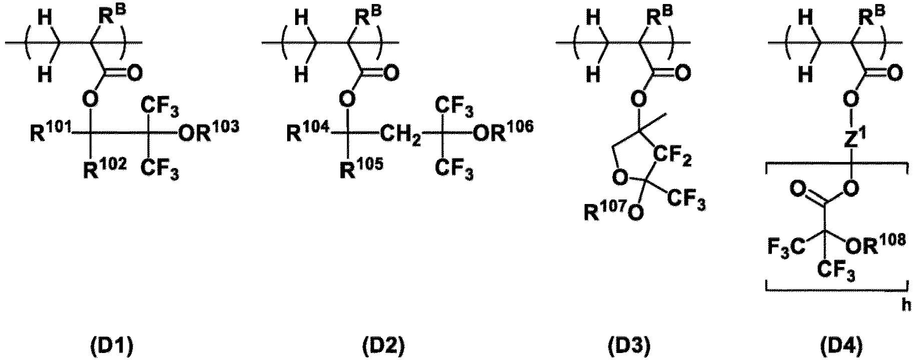 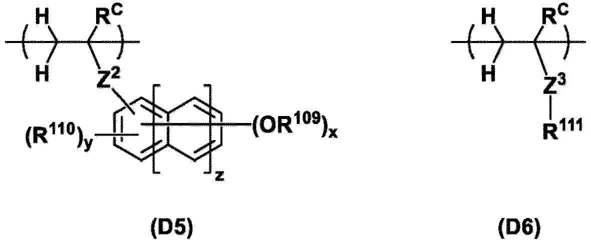 wherein x is an integer of 1 to 3, y is an integer satisfying 0 ≤ y ≤ 5+2z-x, z is 0 or 1, h is an integer of 1 to 3,
   R^{B} is each independently hydrogen, fluorine, methyl or trifluoromethyl,
   R^{C} is each independently hydrogen or methyl,
   R¹⁰¹, R¹⁰², R¹⁰⁴ and R¹⁰⁵ are each independently hydrogen or a C₁-C₁₀ saturated hydrocarbyl group,
   R¹⁰³, R¹⁰⁶, R¹⁰⁷ and R¹⁰⁸ are each independently hydrogen, a C₁-C₁₅ hydrocarbyl group, C₁-C₁₅ fluorinated hydrocarbyl group, or acid labile group, and when R¹⁰³, R¹⁰⁶, R¹⁰⁷ and R¹⁰⁸ each are a hydrocarbyl or fluorinated hydrocarbyl group, an ether bond or carbonyl moiety may intervene in a carbon-carbon bond,
   R¹⁰⁹ is hydrogen or a C₁-C₅ straight or branched hydrocarbyl group in which a heteroatom-containing moiety may intervene in a carbon-carbon bond,
   R¹¹⁰ is a C₁-C₅ straight or branched hydrocarbyl group in which a heteroatom-containing moiety may intervene in a carbon-carbon bond,
   R¹¹¹ is a C₁-C₂₀ saturated hydrocarbyl group in which at least one hydrogen is substituted by fluorine, and in which some constituent -CH₂- may be replaced by an ester bond or ether bond,
   Z¹ is a C₁-C₂₀ (h+1)-valent hydrocarbon group or C₁-C₂₀ (h+1)-valent fluorinated hydrocarbon group,
   Z² is a single bond, *-C(=O)-O- or *-C(=O)-NH-, * designates a point of attachment to the carbon atom in the backbone,
   Z³ is a single bond, -O-, *-C(=O)=O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond, or sulfonamide bond, and * designates a point of attachment to the carbon atom in the backbone.
15. The chemically amplified positive resist composition of any one of 7 to 14, further comprising a quencher.
16. The chemically amplified positive resist composition of any one of 7 to 15, further comprising a photoacid generator other than the photoacid generator of 5.
17. A resist pattern forming process comprising the steps of:
   applying the chemically amplified positive resist composition of any one of 7 to 16 onto a substrate to form a resist film thereon,
   exposing the resist film to high-energy radiation, and
   developing the exposed resist film in an alkaline developer.
18. The process of 17 wherein the high-energy radiation is EUV or EB.
19. The process of 17 o 18 wherein the substrate has the outermost surface of a chromium-containing material.
20. The process of any one of 17 to 19 wherein the substrate is a photomask blank.

### ADVANTAGEOUS EFFECTS OF INVENTION

When processed by the microfabrication technology, especially EB and EUV lithography processes, a chemically amplified positive resist composition comprising an onium salt within the scope of the invention as a photoacid generator can form a resist pattern having a very high resolution and reduced LER. A pattern of rectangular profile is obtainable by virtue of properly inhibited dissolution.

### DESCRIPTION OF PREFERRED EMBODIMENTS

As used herein, the singular forms "a," "an" and "the" include plural referents unless the context clearly dictates otherwise. "Optional" or "optionally" means that the subsequently described event or circumstances may or may not occur, and that description includes instances where the event or circumstance occurs and instances where it does not. The notation (Cn-Cm) means a group containing from n to m carbon atoms per group. In chemical formulae, the broken line (---) and asterisk (*) each designate a point of attachment, namely valence bond. Me stands for methyl and Ac for acetyl. As used herein, the term "fluorinated" refers to a fluorine-substituted or fluorine-containing compound or group. The terms "group" and "moiety" are interchangeable.

The abbreviations and acronyms have the following meaning.
PAG: photoacid generator
Mw: weight average molecular weight
Mn: number average molecular weight
Mw/Mn: molecular weight distribution or dispersity
GPC: gel permeation chromatography
PEB: post-exposure baking
LER: line edge roughness
CDU: critical dimension uniformity

It is understood that for some structures represented by chemical formulae, there can exist enantiomers and diastereomers because of the presence of asymmetric carbon atoms. In such a case, a single formula collectively represents all such isomers. The isomers may be used alone or in admixture.

### Onium salt

One embodiment of the invention is an onium salt having the formula (A).

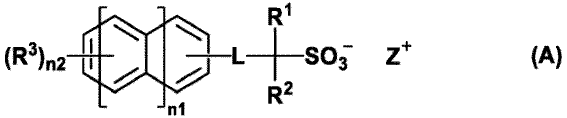

In formula (A), n1 is an integer of 0 to 2. The relevant structure represents a benzene ring in case of n1=0, a naphthalene ring in case of n1=1, and an anthracene ring in case of n1=2. From the aspect of solvent solubility, a benzene ring corresponding to n1=0 is preferred. The subscript n2 is an integer of 2 to 5 in case of n1=0, an integer of 2 to 7 in case of n1=1, and an integer of 2 to 9 in case of n1=2.

In formula (A), L is a single bond, ether bond, ester bond, sulfonic ester bond, carbonate bond or carbamate bond. Inter alia, an ester bond and sulfonic ester bond are preferred, with sulfonic ester bond being more preferred.

In formula (A), R¹ and R² are each independently hydrogen or a C₃-C₂₀ branched or cyclic hydrocarbyl group which may contain a heteroatom, excluding that both R¹ and R² are hydrogen. When both R¹ and R² are hydrogen or a straight hydrocarbyl group, the structure in proximity to -SO₃⁻ group becomes less bulky, suggesting that apparent acid diffusion is not restrained and lithography performance is degraded.

The hydrocarbyl groups R¹ and R² may be saturated or unsaturated. Examples thereof include, but are not limited to, C₃-C₂₀ branched alkyl groups such as isopropyl, sec-butyl, tert-butyl, tert-pentyl, and 2-ethylhexyl; C₃-C₂₀ cyclic aliphatic hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, oxanorbomyl, tricyclo[5.2.1.0^{2,6}]decyl, and adamantyl; C₆-C₂₀ aryl groups such as phenyl, naphthyl and anthracenyl, and combinations thereof. In the hydrocarbyl group, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, cyano moiety, fluorine, chlorine, bromine, iodine, carbonyl moiety, ether bond, ester bond, sulfonic ester bond, carbonate bond, lactone ring, sultone ring, carboxylic anhydride (-C(=O)-O-C(=O)-) or haloalkyl moiety. Suitable halogen atoms include fluorine, chlorine, bromine, and iodine, with fluorine and iodine being preferred.

R¹ and R² may bond together to form a ring with the carbon atom to which they are attached. Examples of the ring include cyclopropane, cyclobutane, cyclopentane, cyclohexane, norbornane, and adamantane rings. In the ring, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the ring may contain a hydroxy moiety, cyano moiety, fluorine, chlorine, bromine, iodine, carbonyl moiety, ether bond, ester bond, sulfonic ester bond, carbonate bond, lactone ring, sultone ring, carboxylic anhydride (-C(=O)-O-C(=O)-) or haloalkyl moiety.

In formula (A), R³ is each independently iodine or a C₃-C₂₀ branched or cyclic hydrocarbyl group which may contain a heteroatom. At least one R³ is attached to a carbon atom adjoining the carbon atom to which L is attached. Examples of the hydrocarbyl group are as exemplified above for the hydrocarbyl groups R¹ and R².

Examples of the structure that R³ forms with the aromatic ring in formula (A) are shown below, but not limited thereto. Herein, the broken line designates a point of attachment to L.

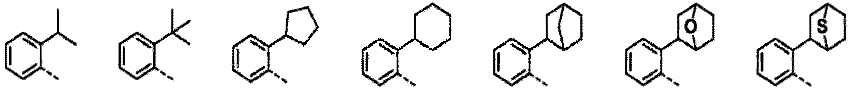

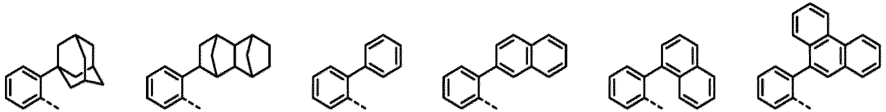

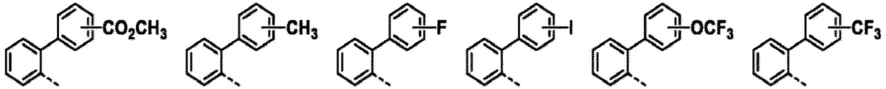

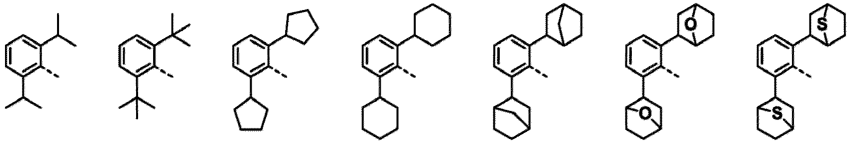

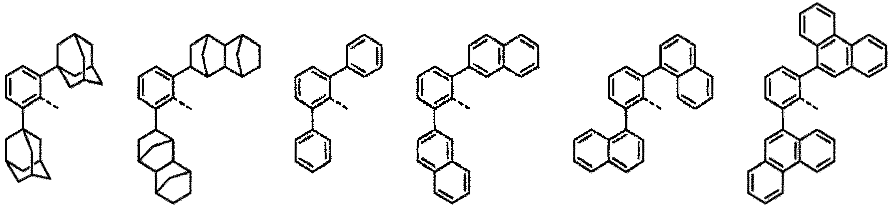

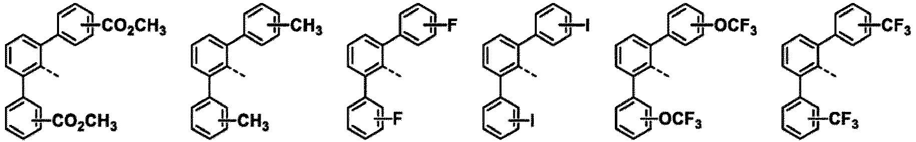

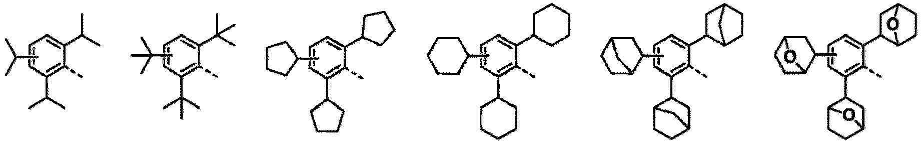

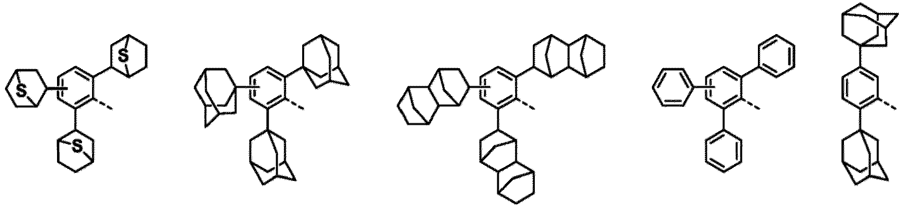

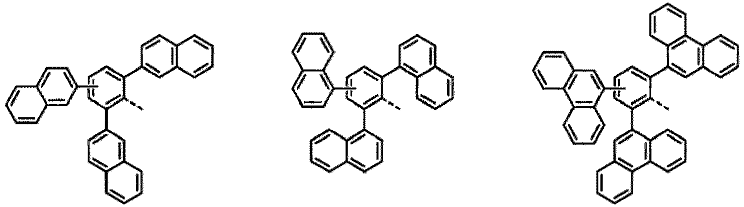

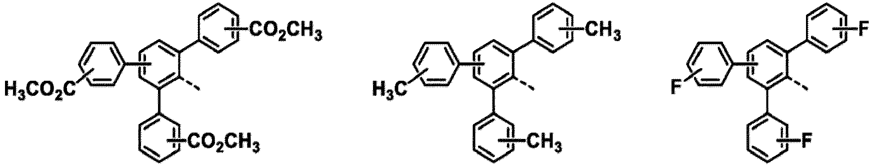

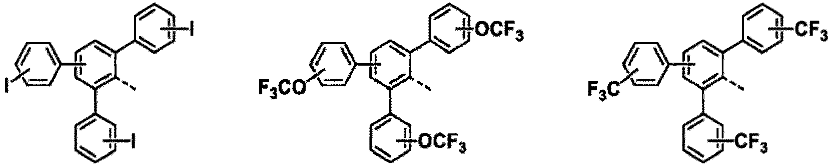

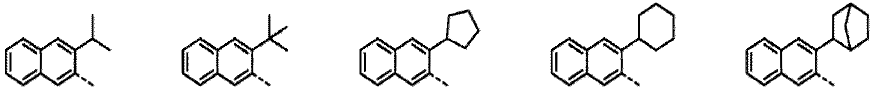

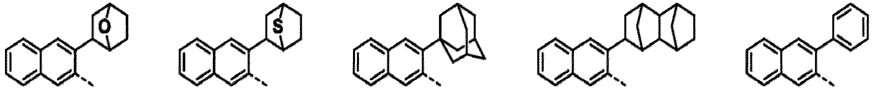

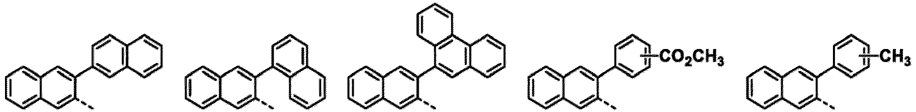

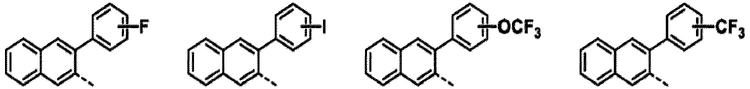

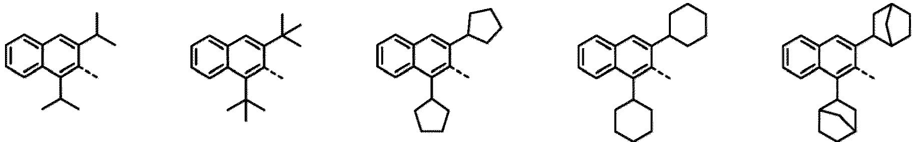

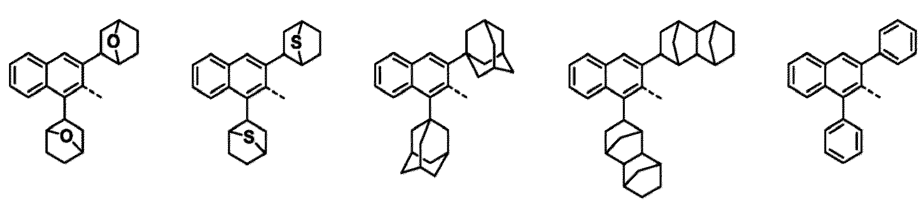

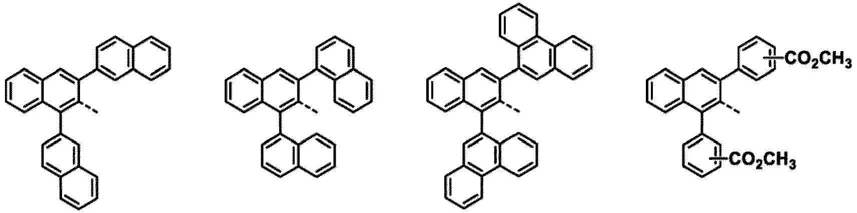

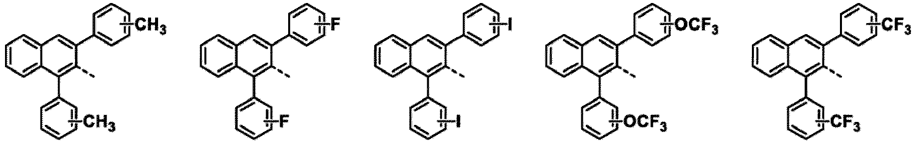

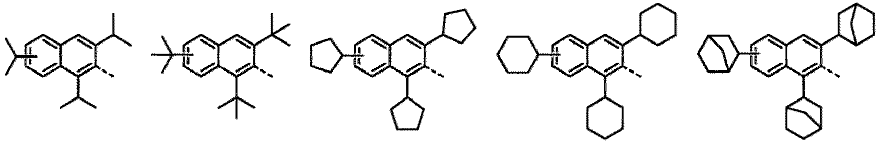

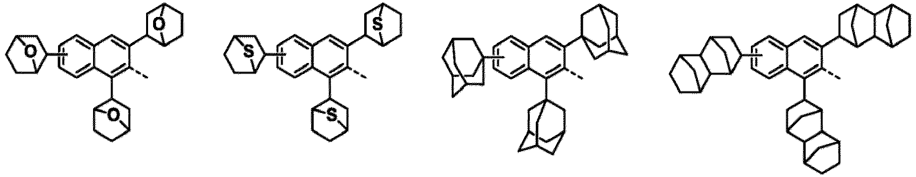

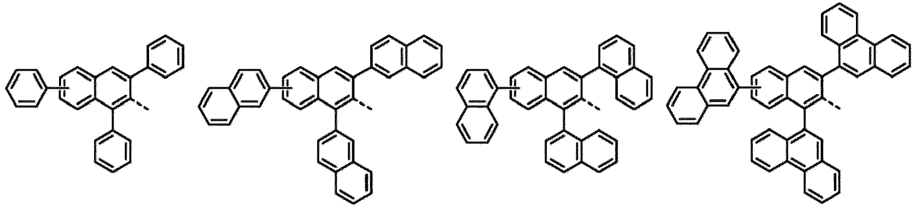

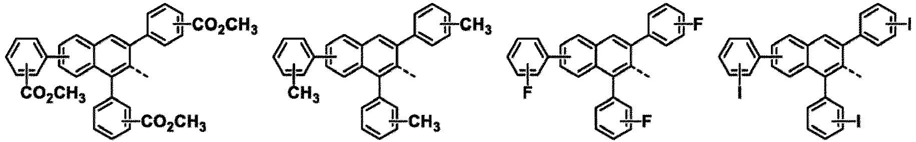

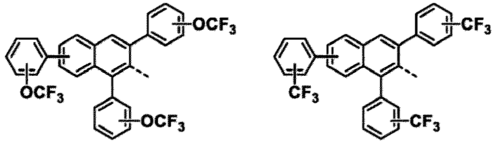

Preferably, the onium salt of formula (A) has the formula (A1). 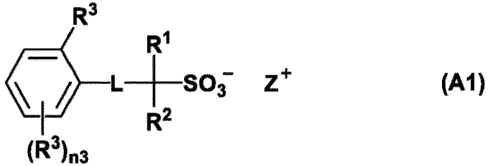 Herein L, R¹, R², R³, and Z⁺ are as defined above, and n3 is an integer of 1 to 4.

More preferably, the onium salt of formula (A1) has the formula (A2). 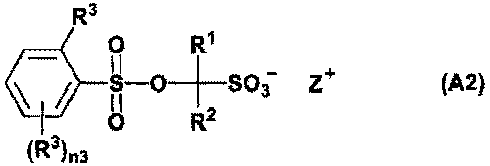 Herein n3, R¹, R², R³, and Z⁺ are as defined above.

Preferred examples of the anion in the onium salt having formula (A) are shown below, but not limited thereto.

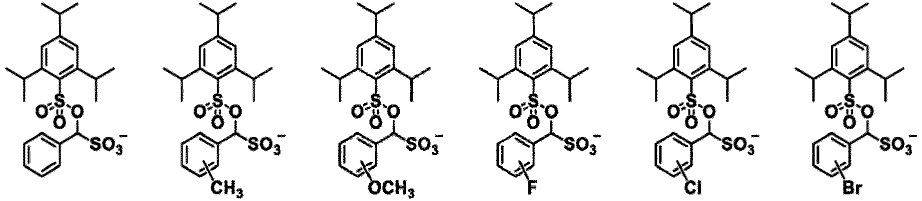

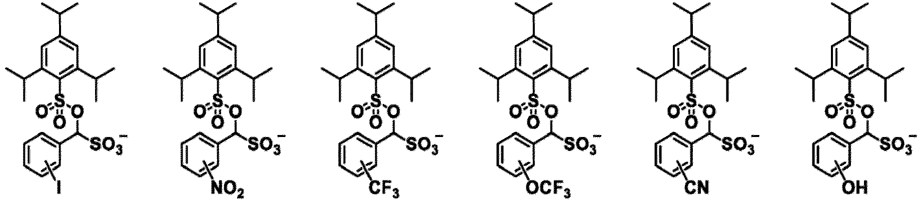

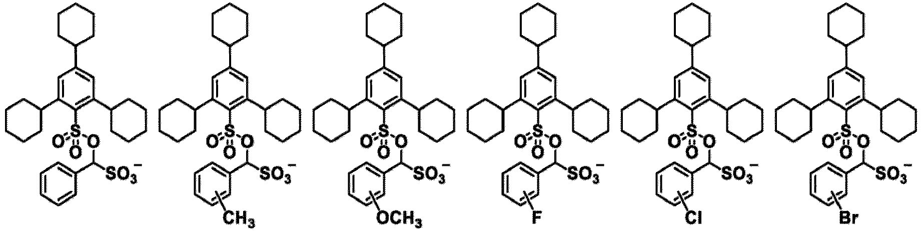

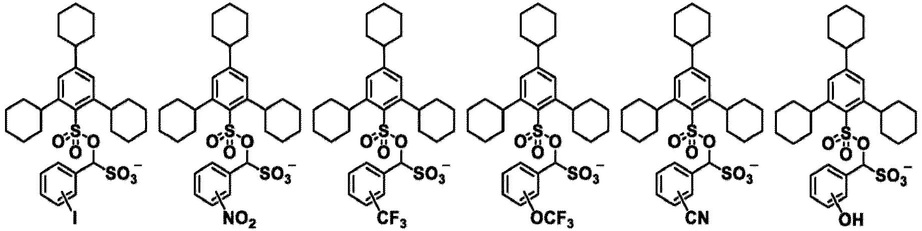

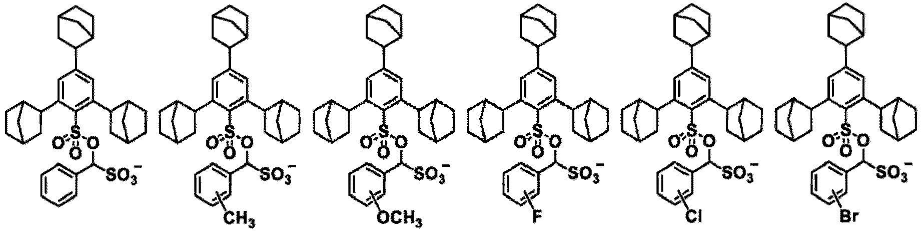

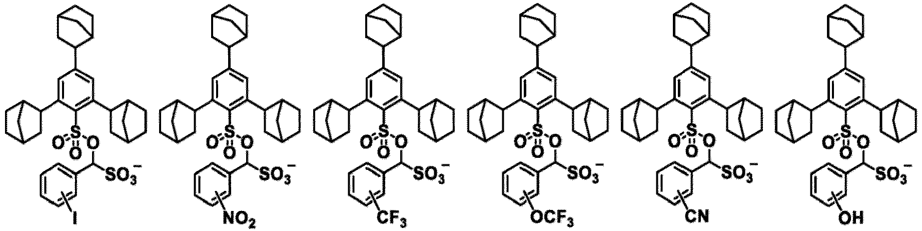

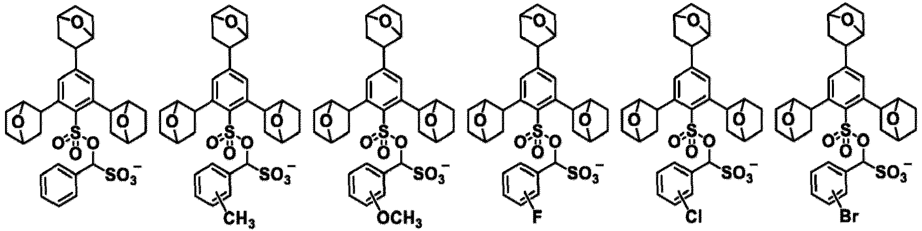

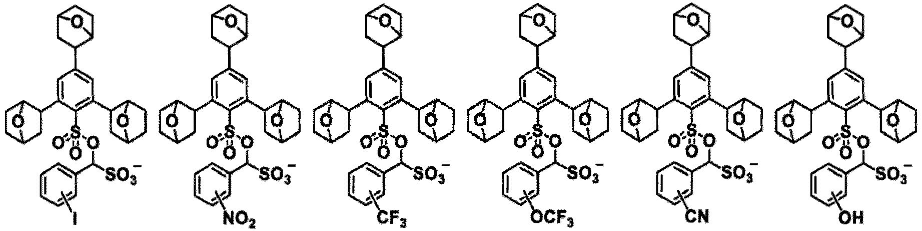

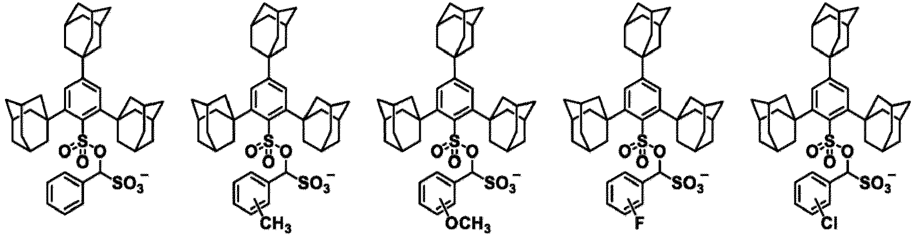

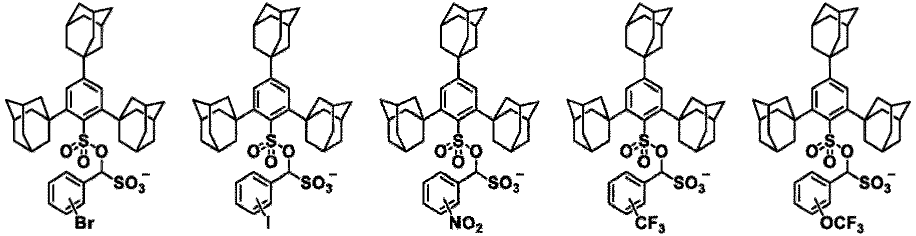

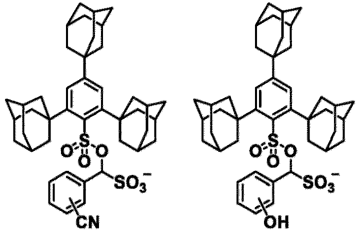

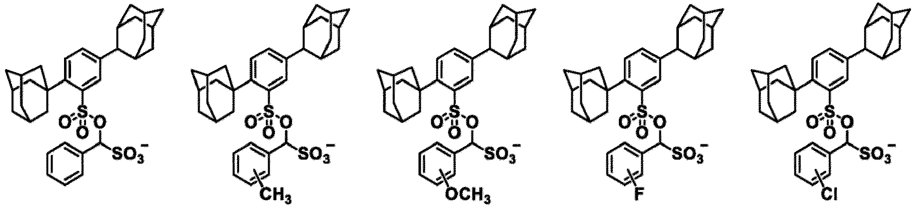

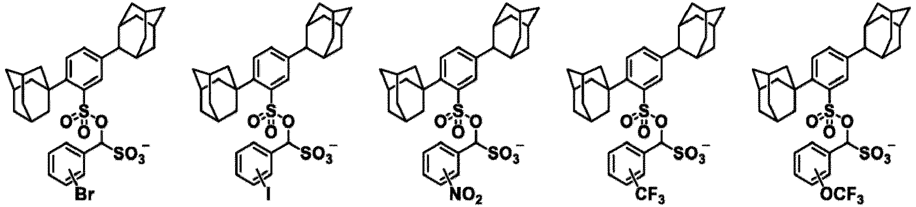

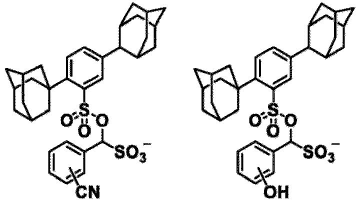

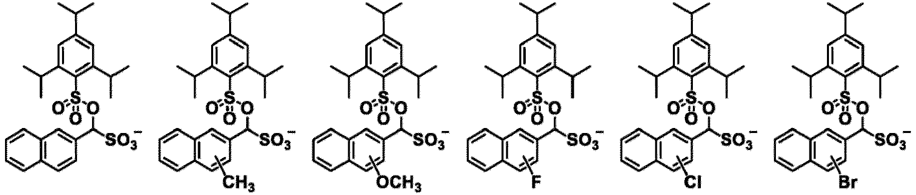

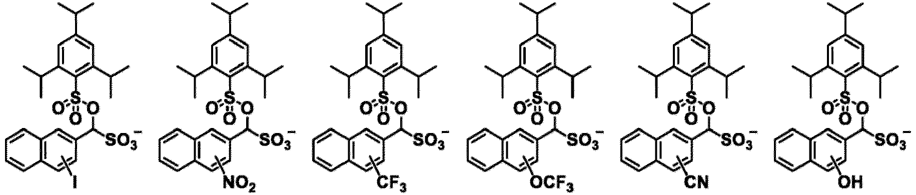

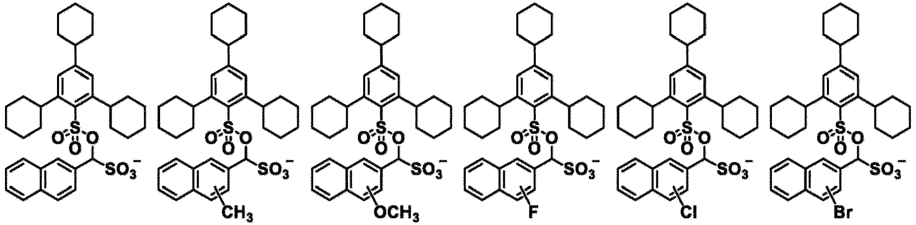

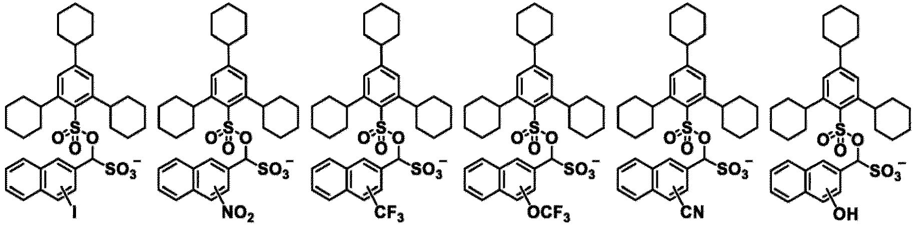

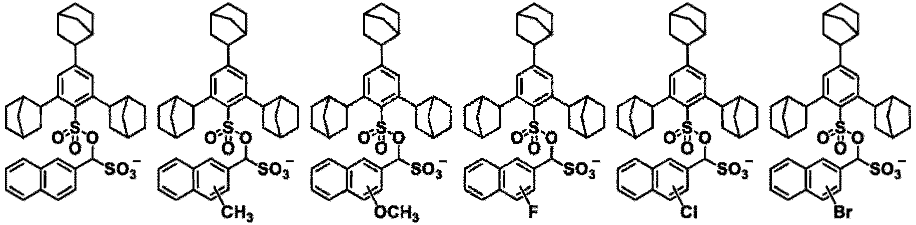

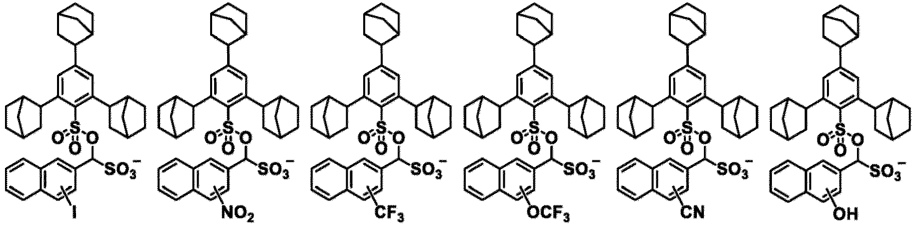

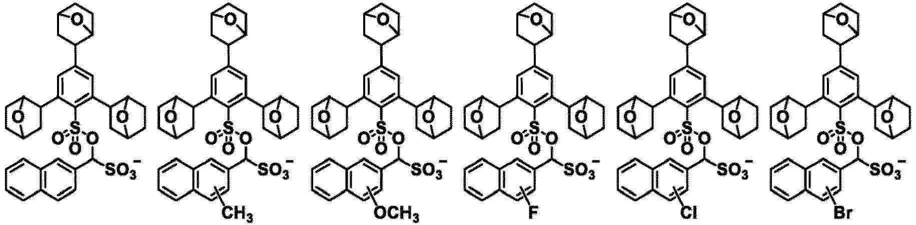

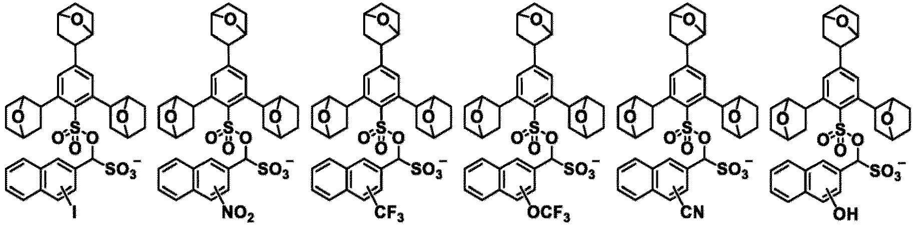

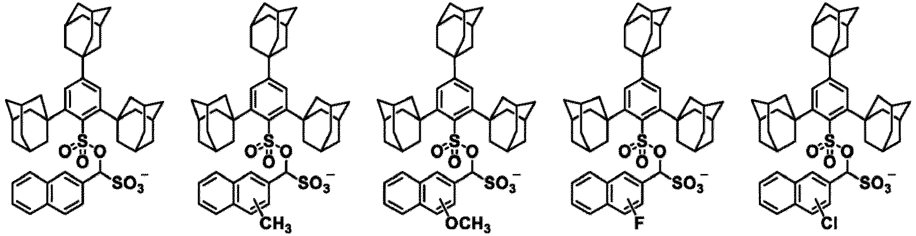

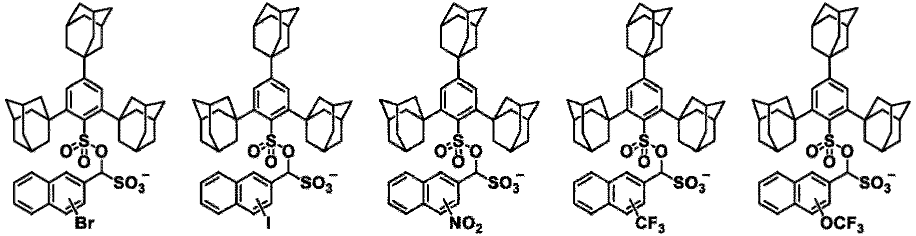

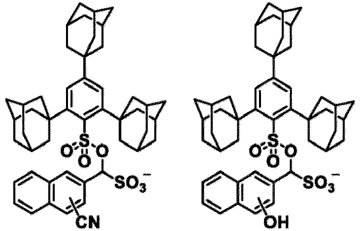

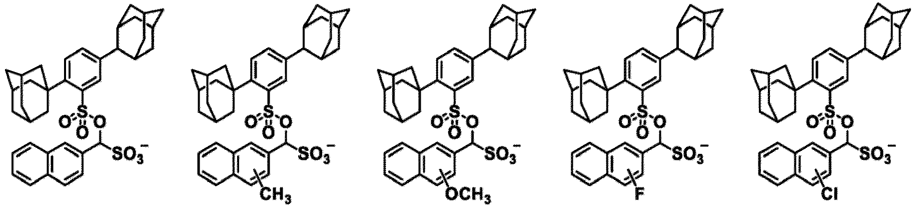

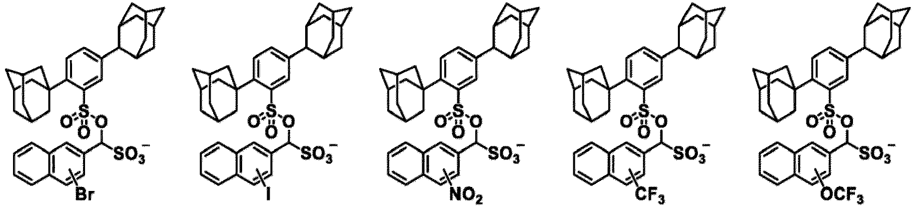

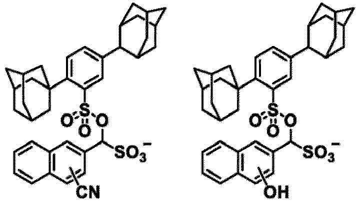

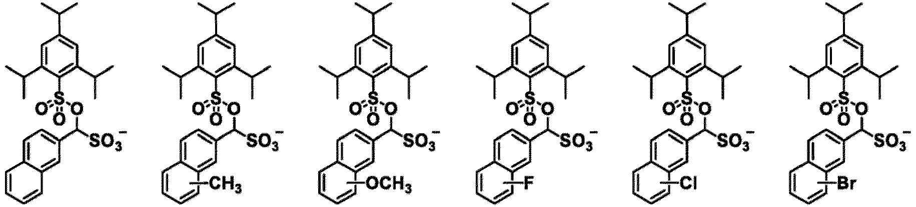

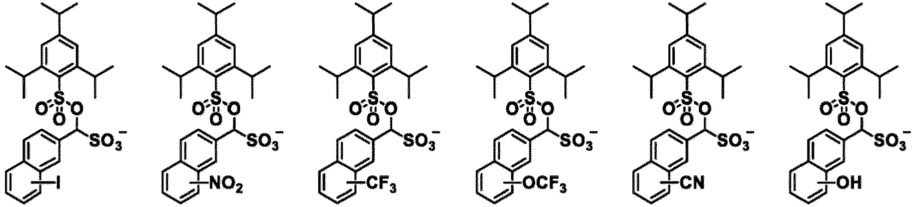

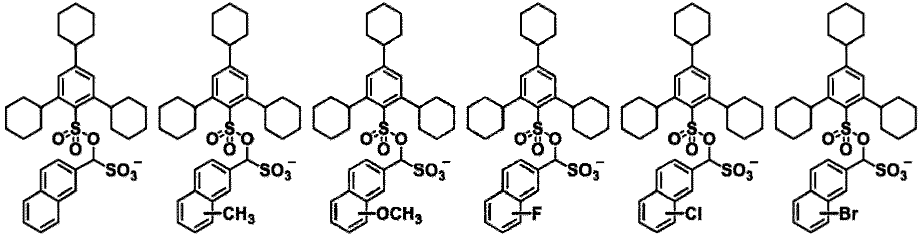

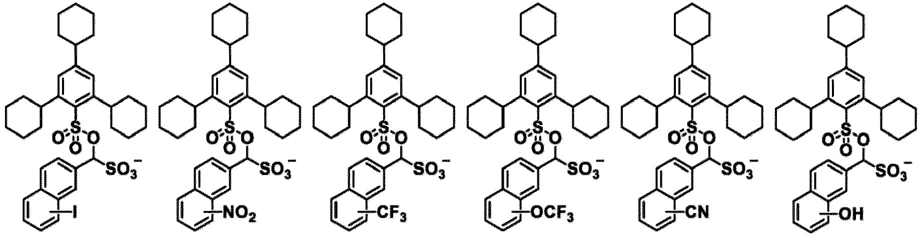

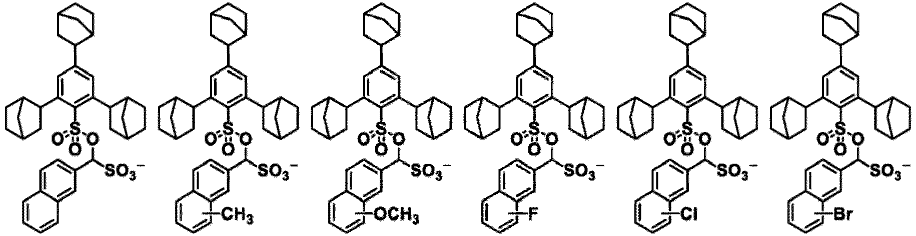

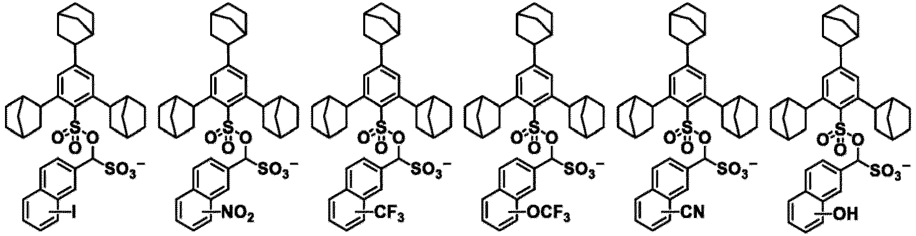

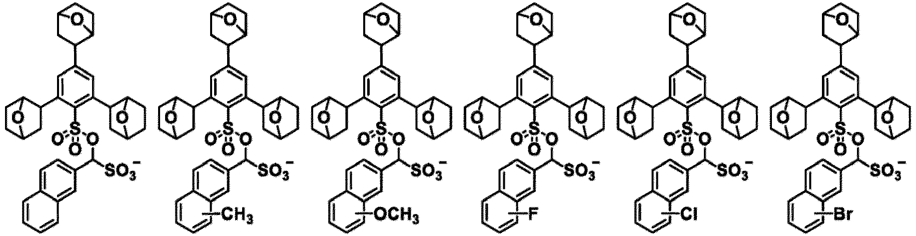

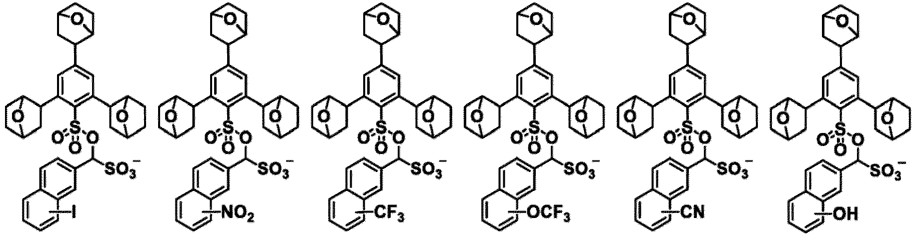

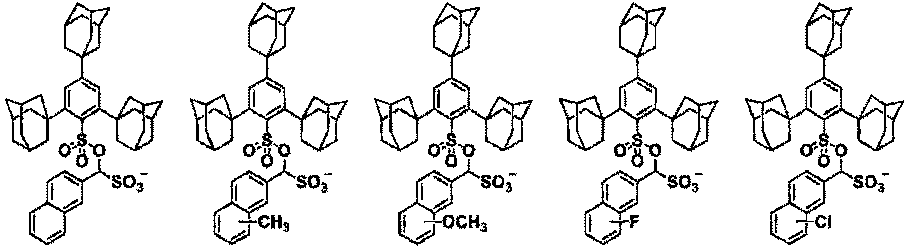

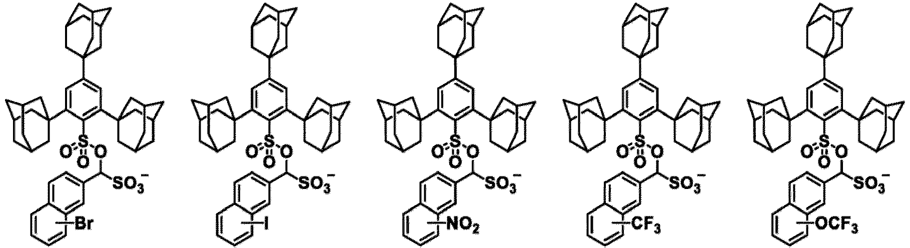

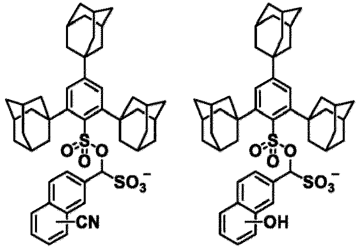

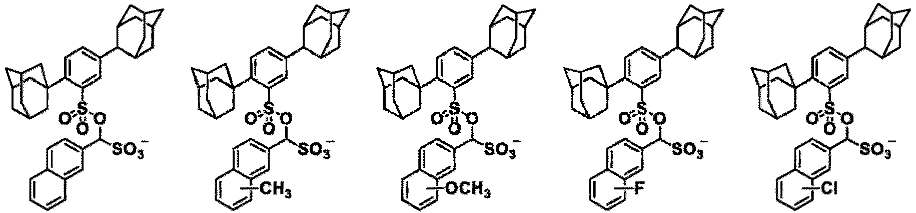

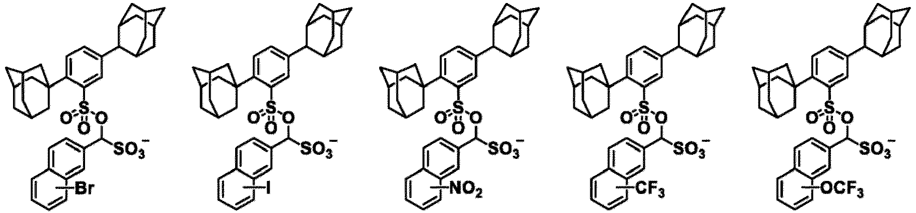

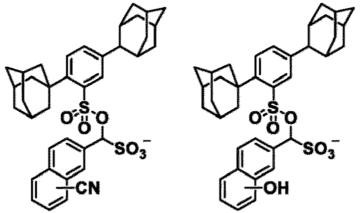

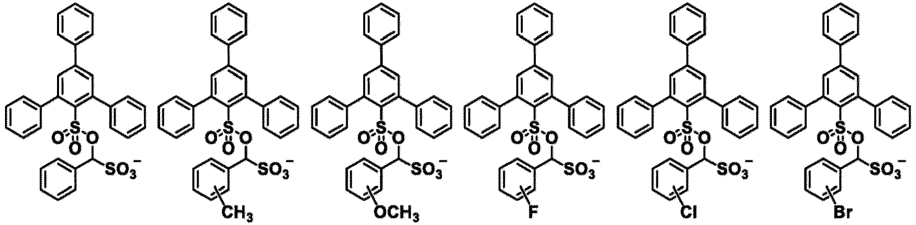

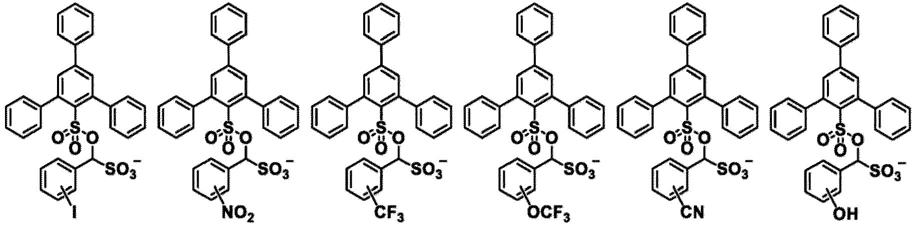

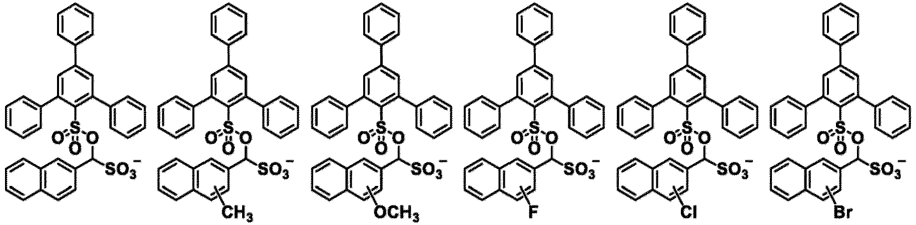

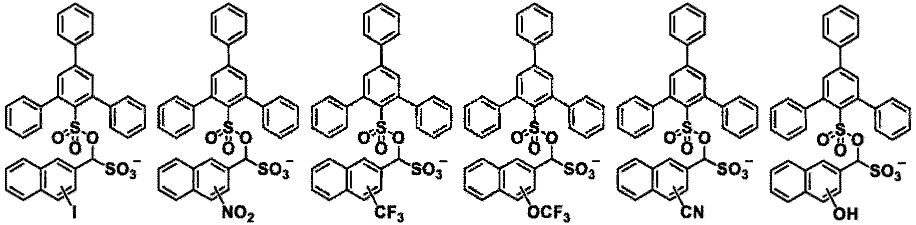

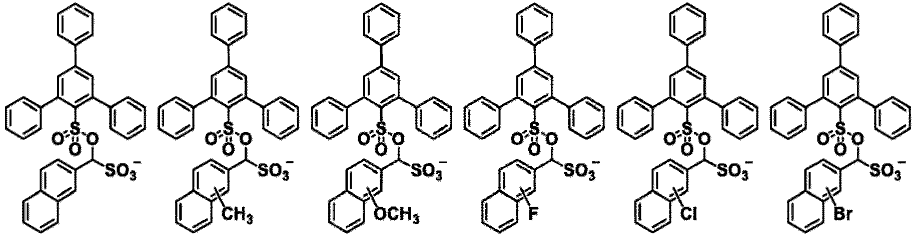

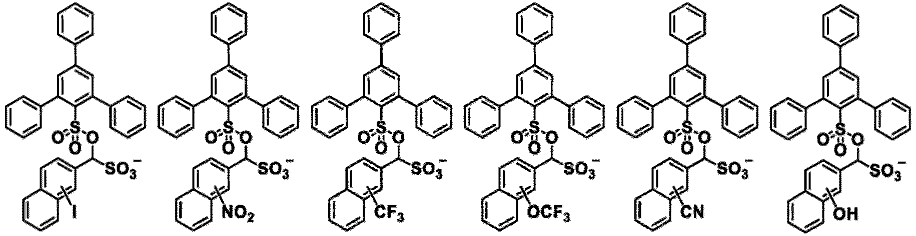

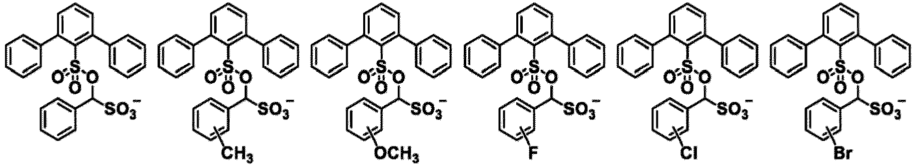

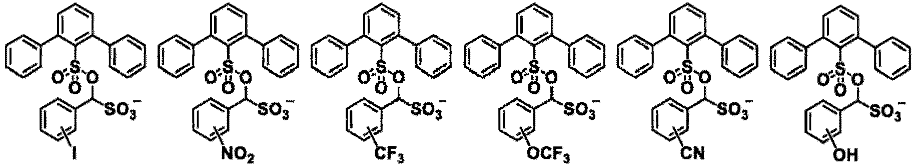

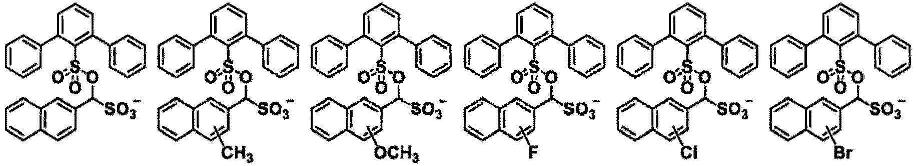

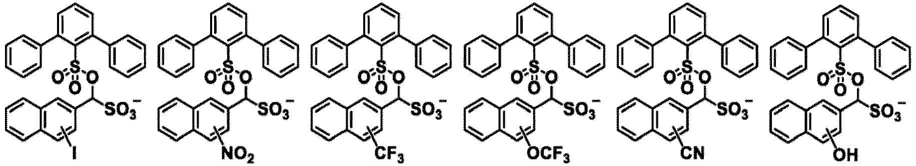

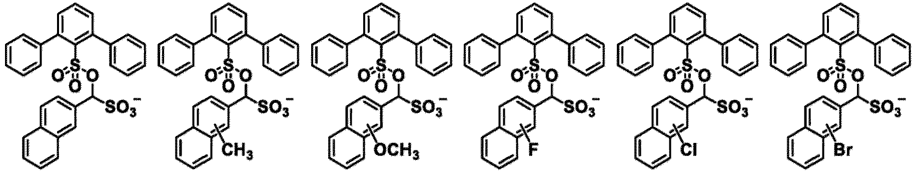

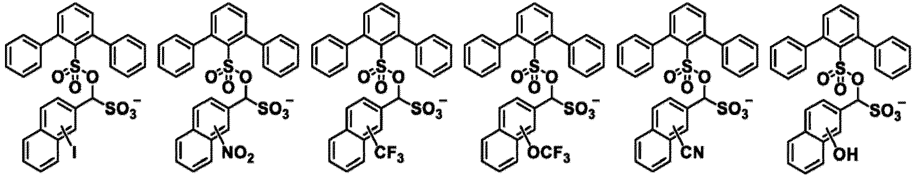

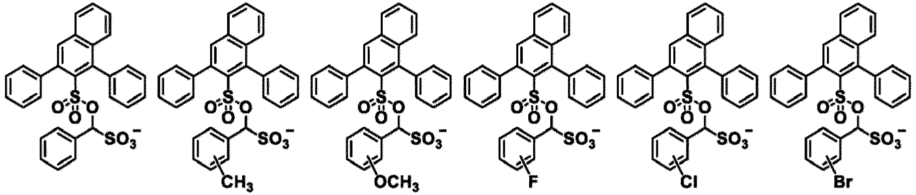

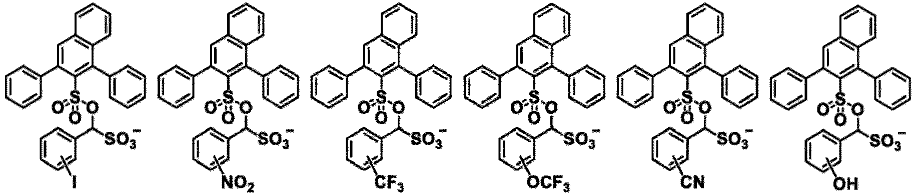

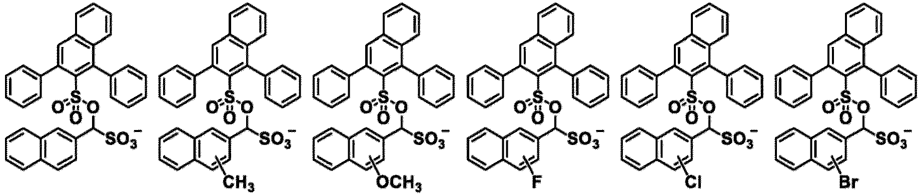

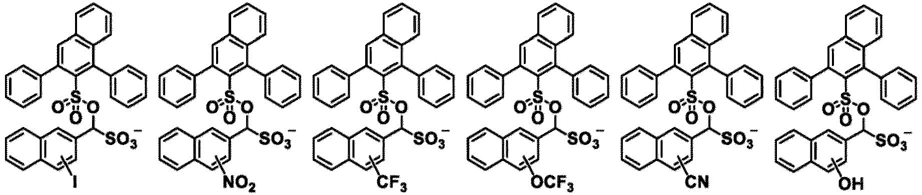

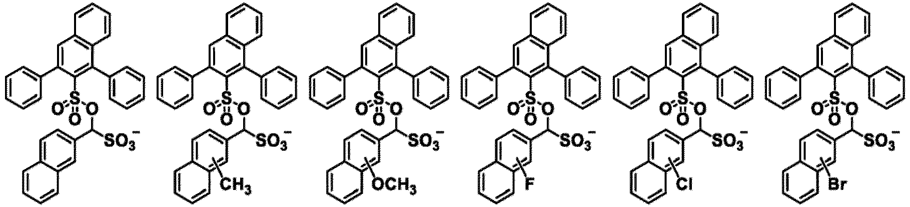

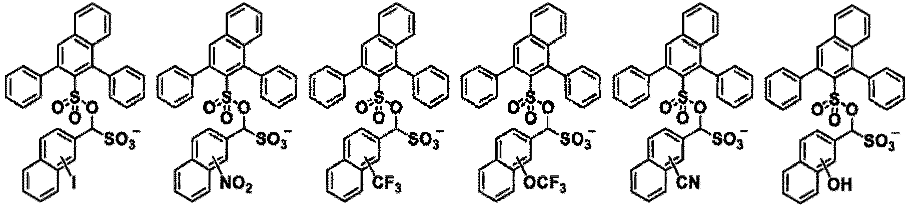

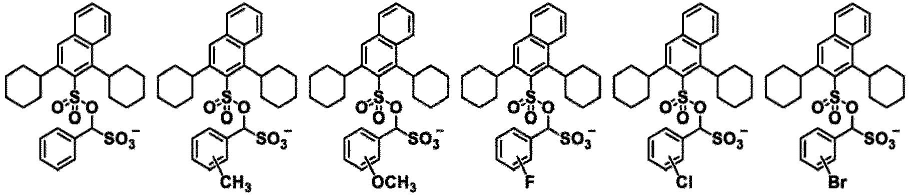

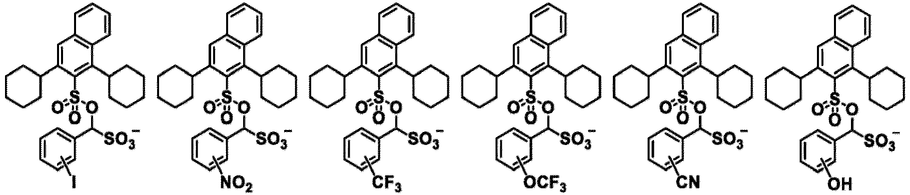

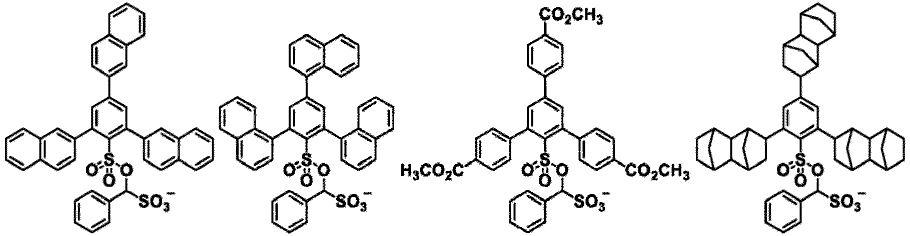

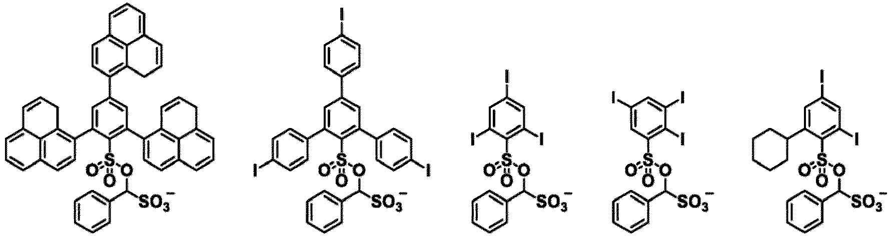

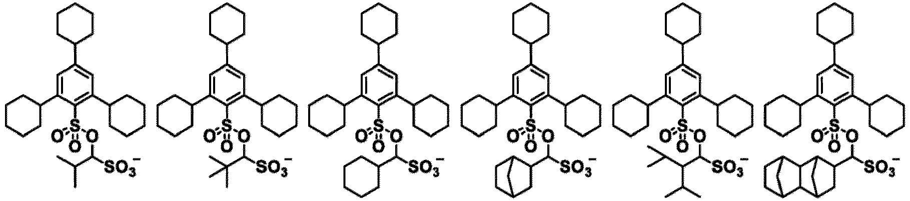

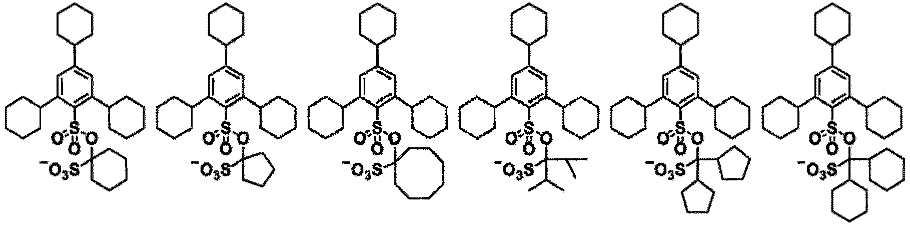

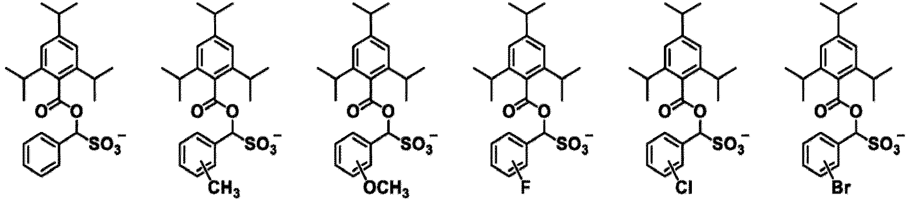

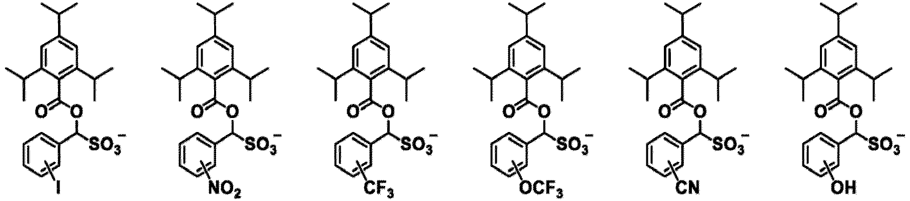

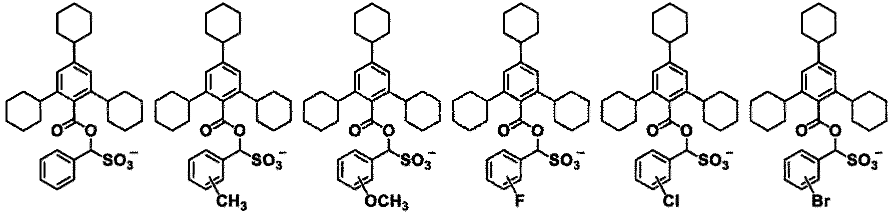

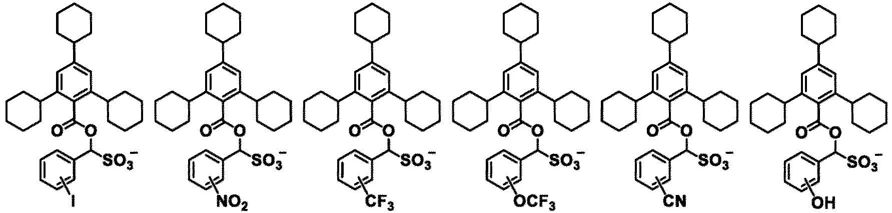

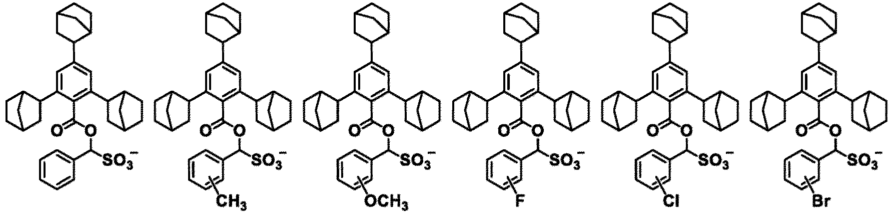

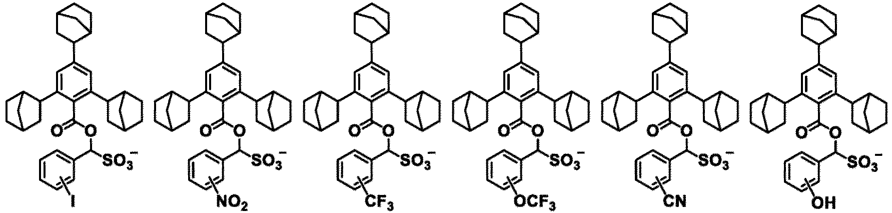

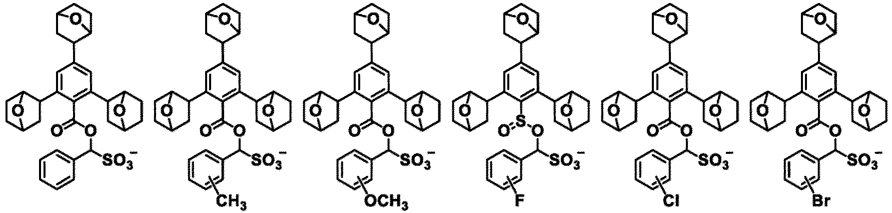

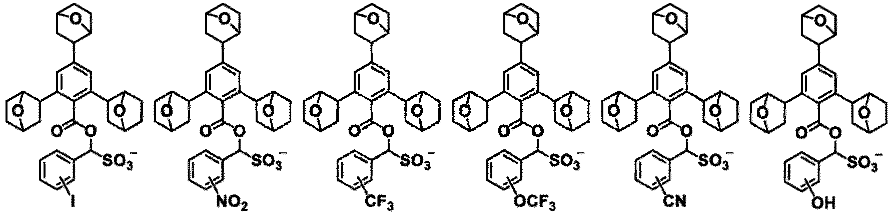

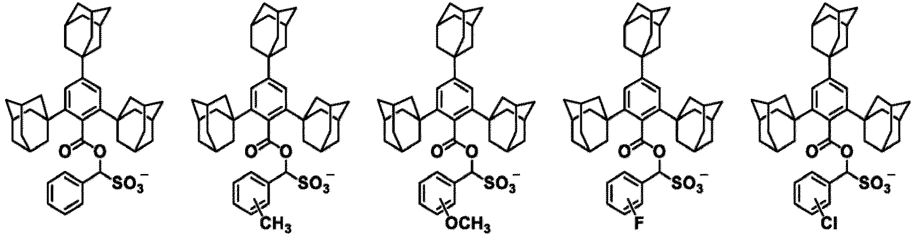

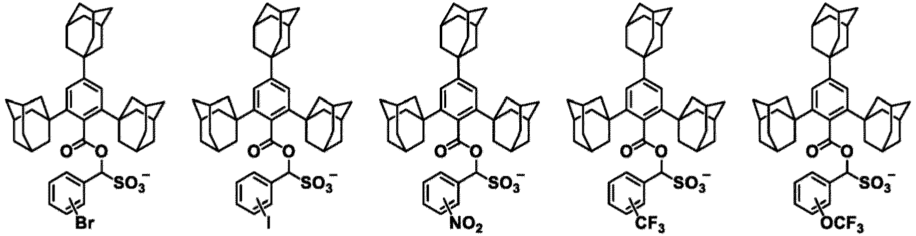

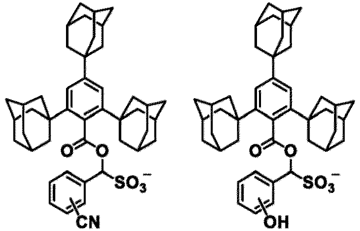

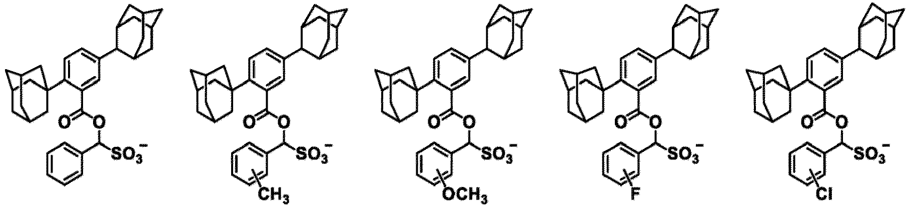

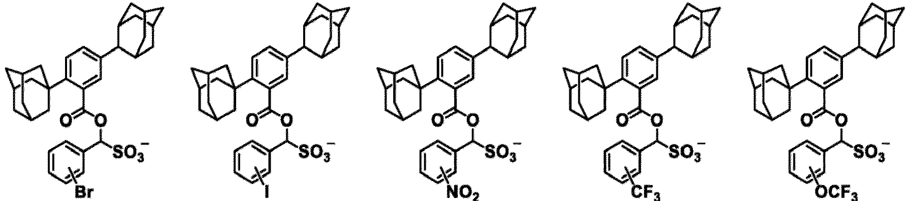

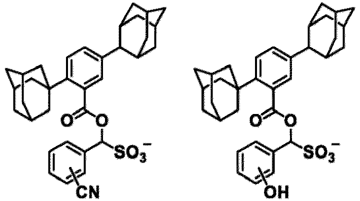

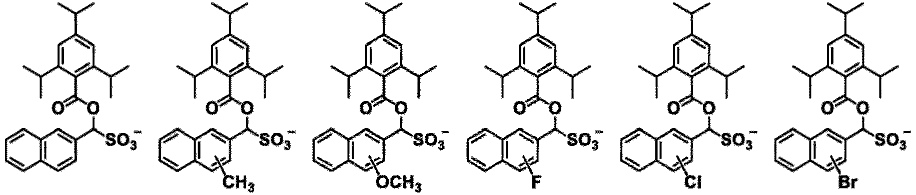

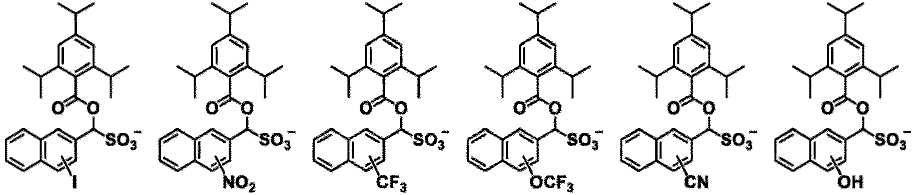

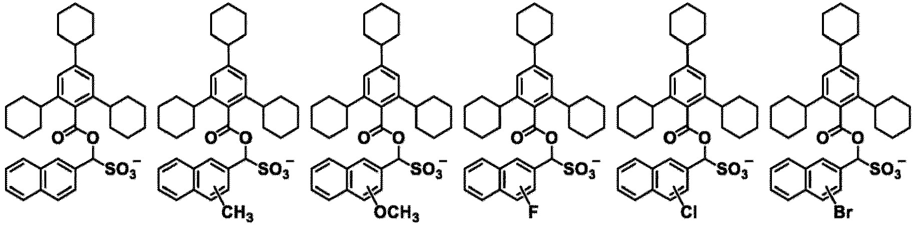

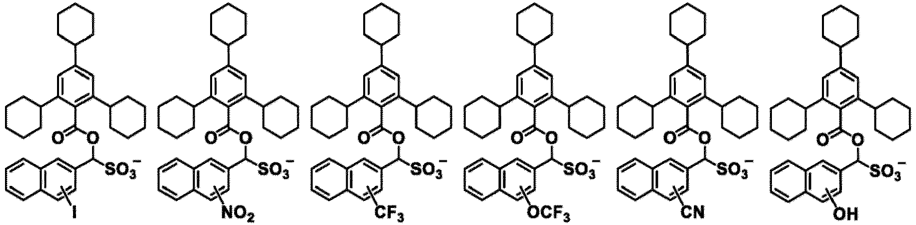

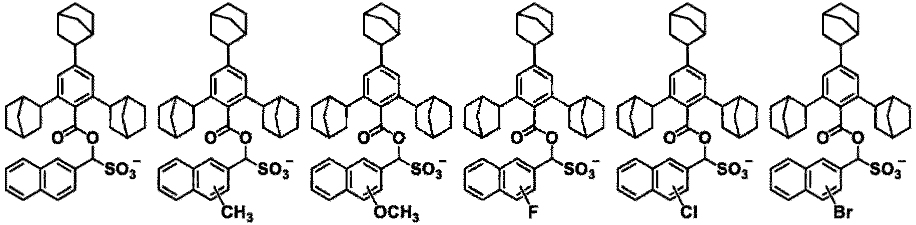

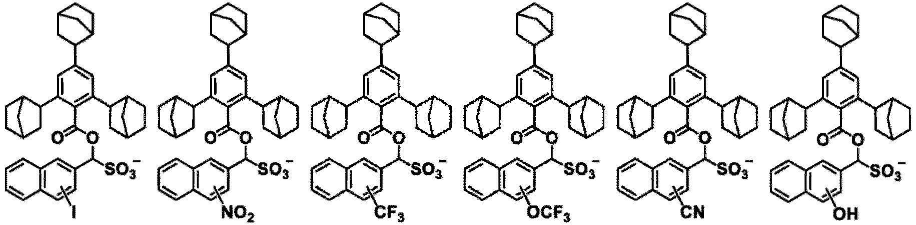

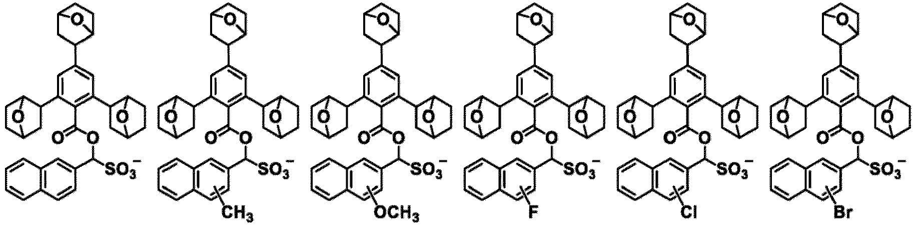

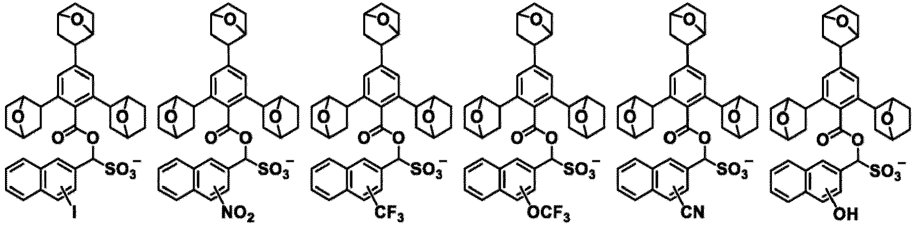

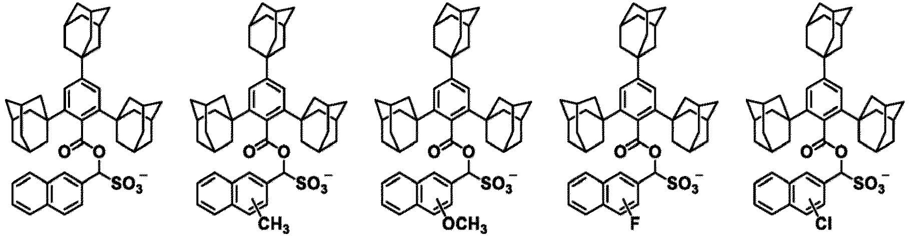

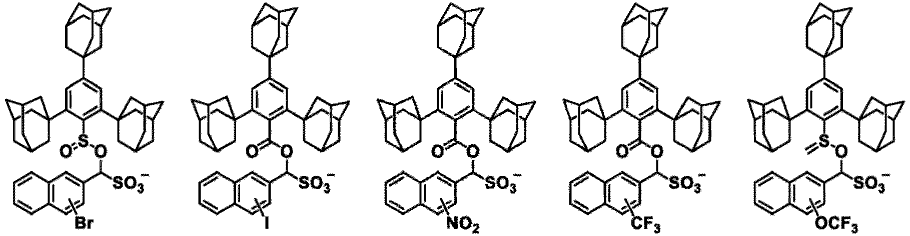

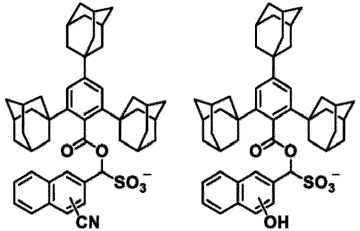

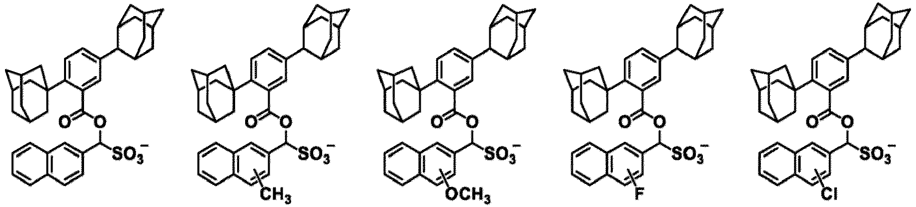

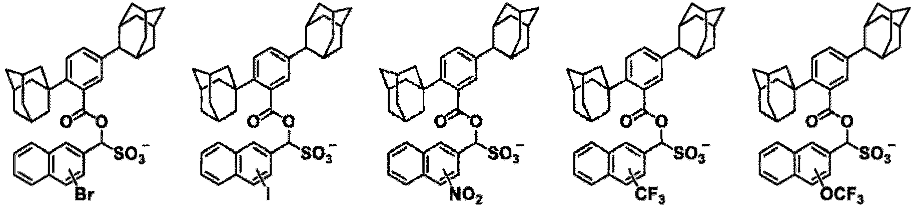

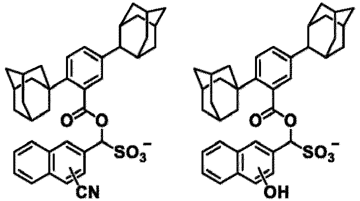

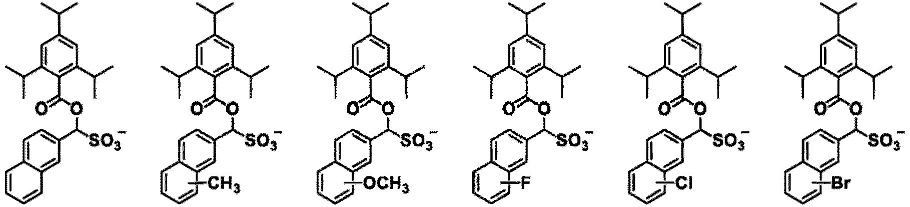

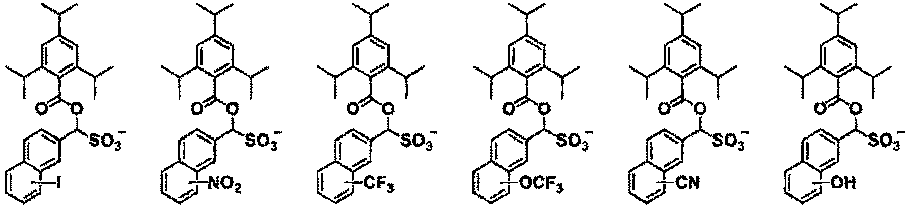

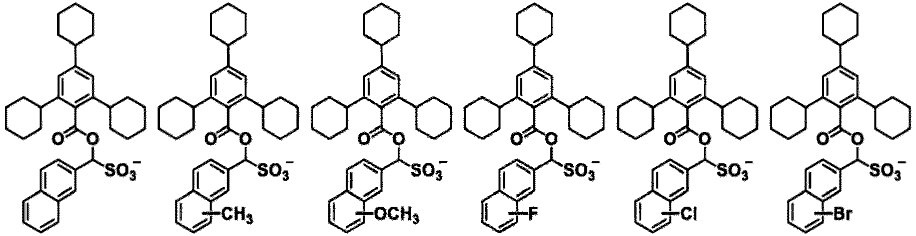

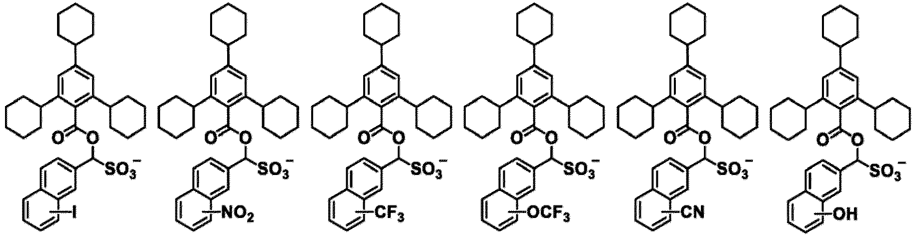

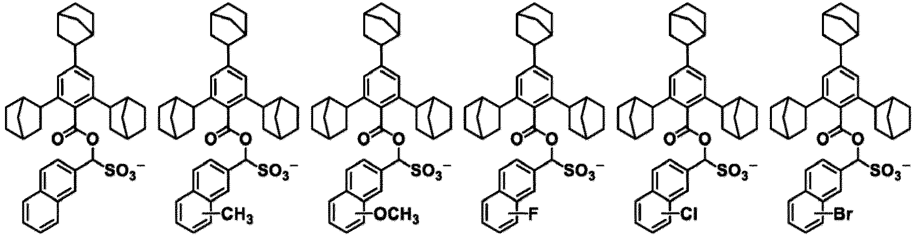

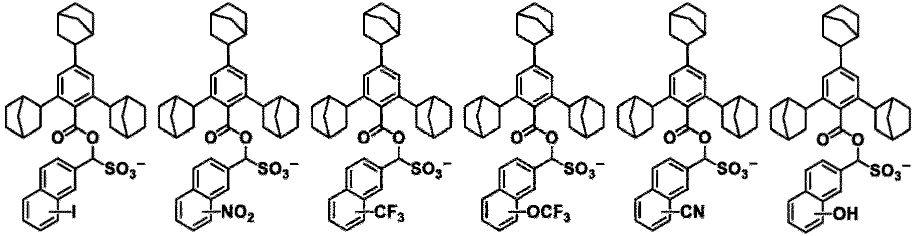

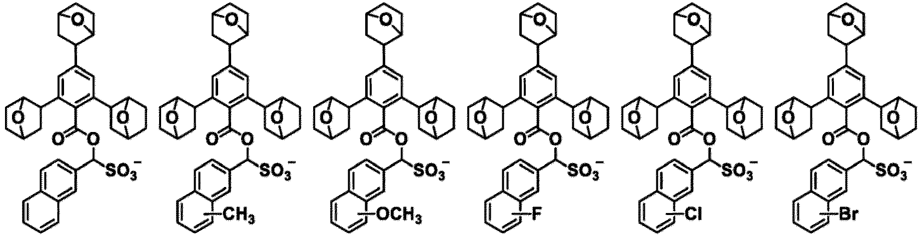

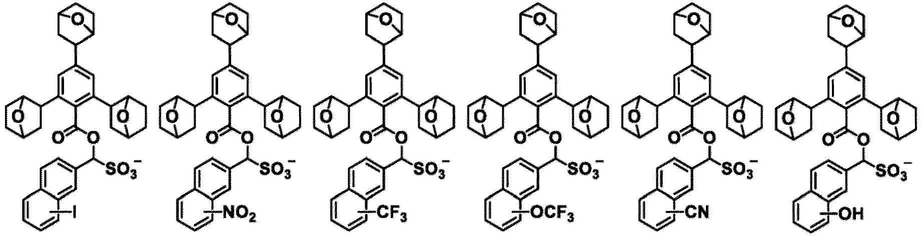

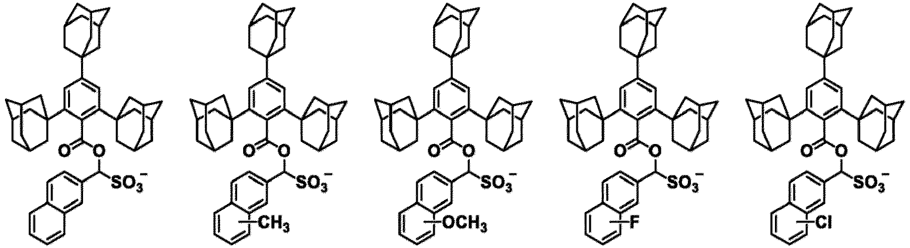

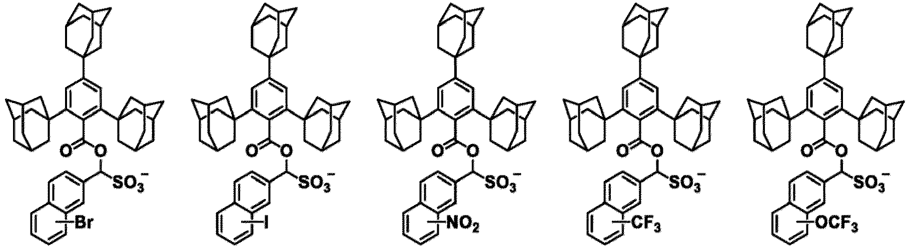

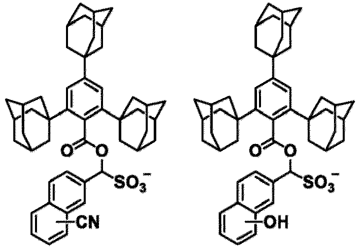

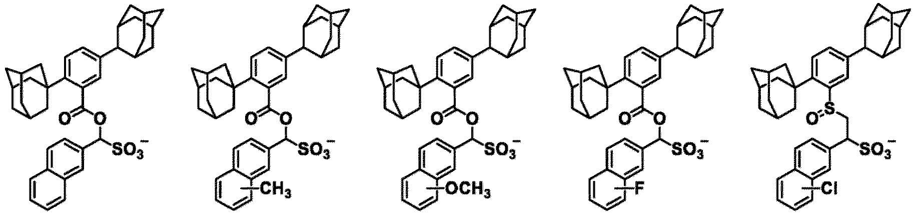

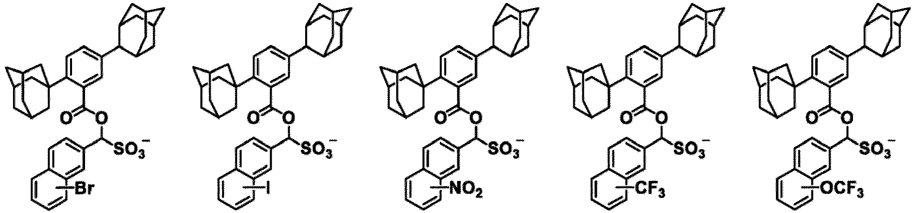

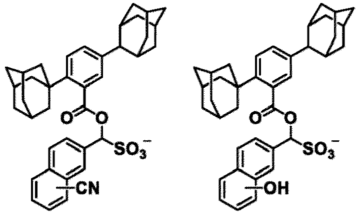

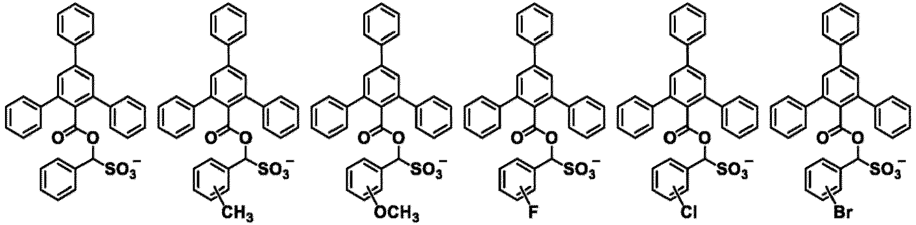

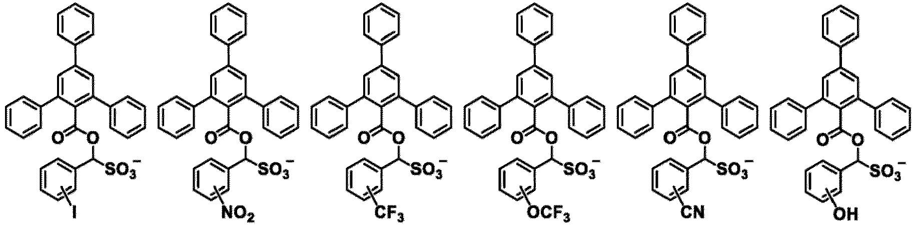

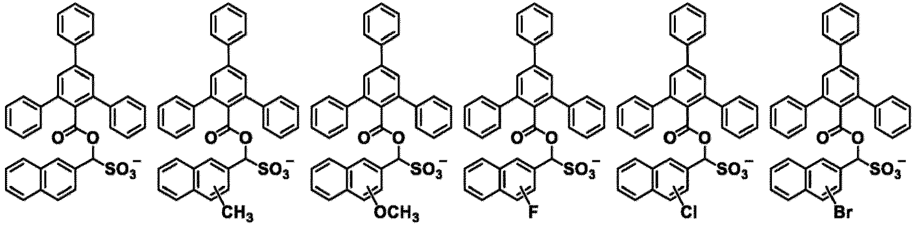

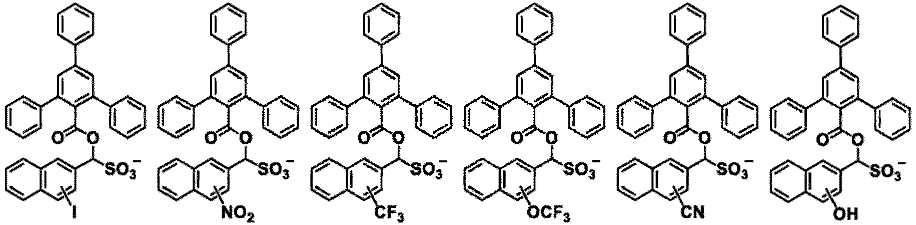

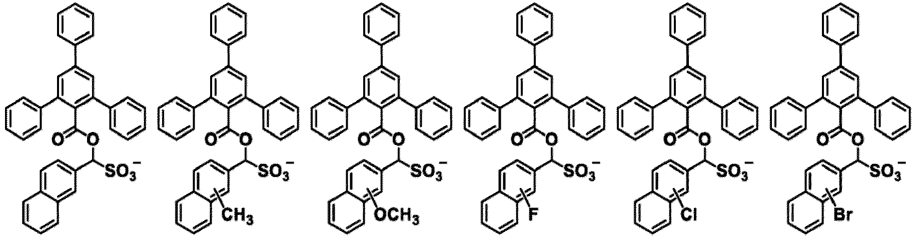

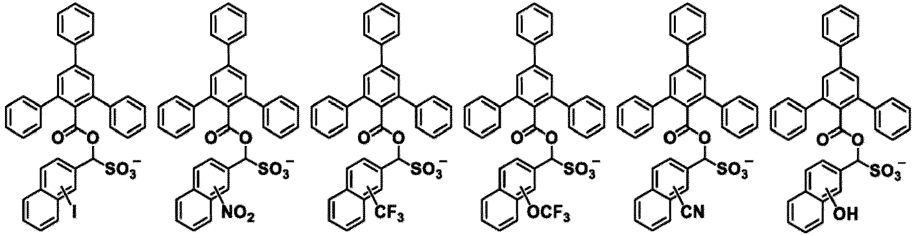

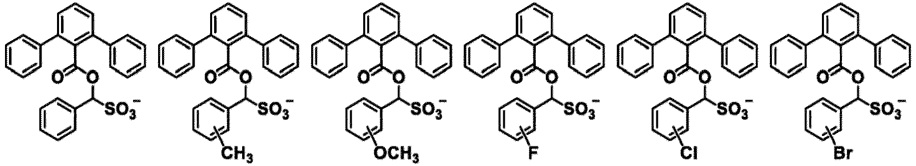

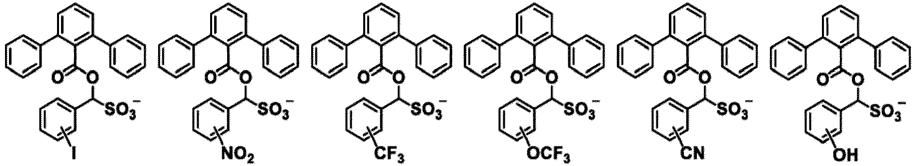

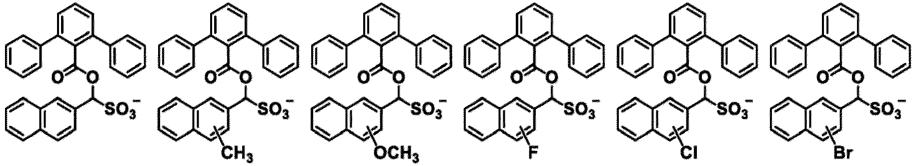

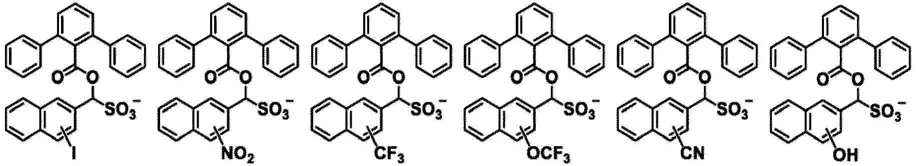

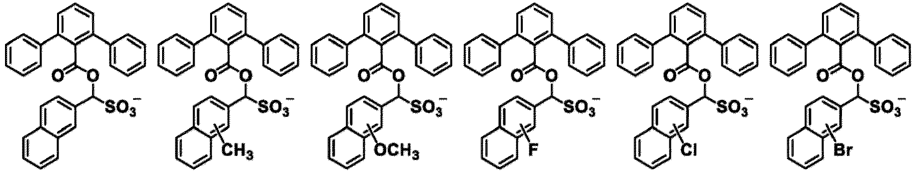

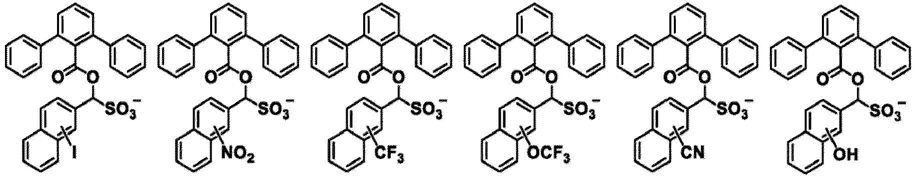

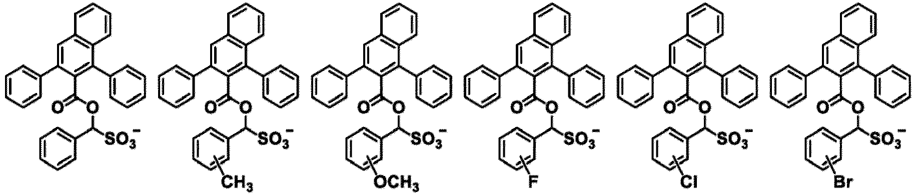

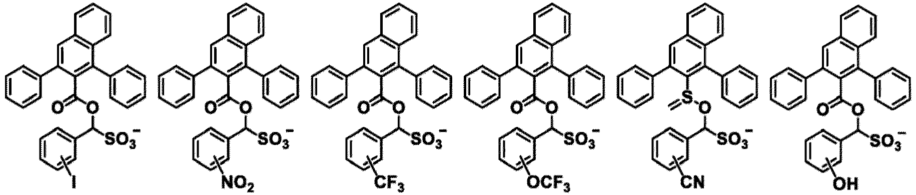

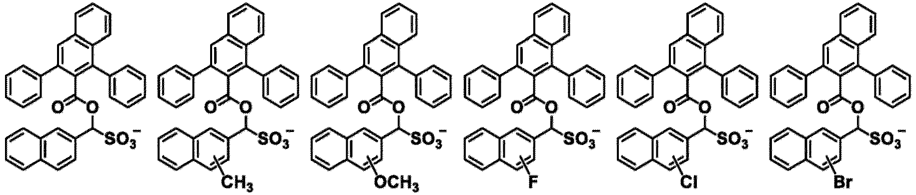

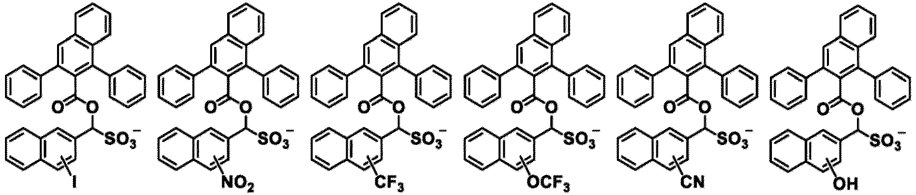

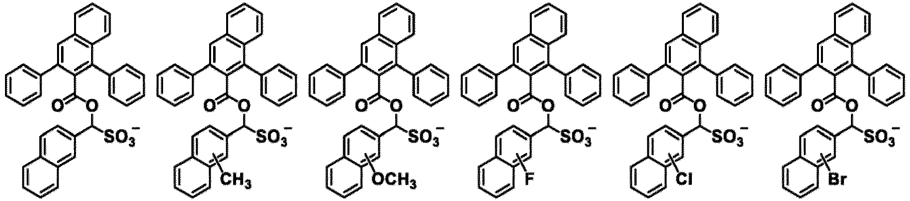

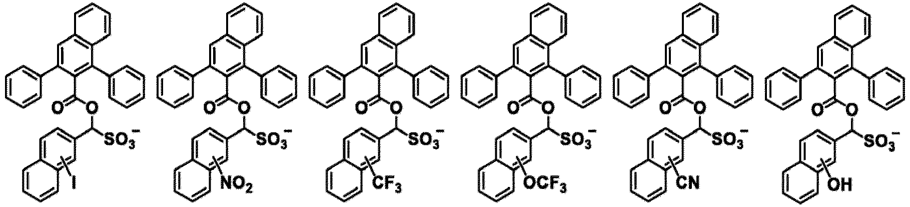

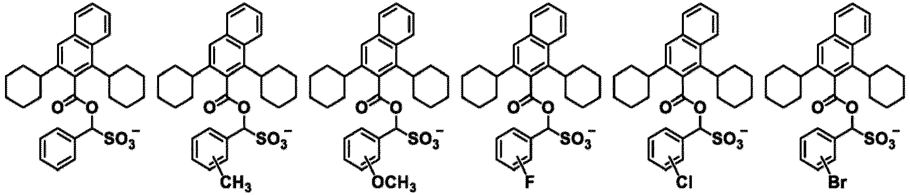

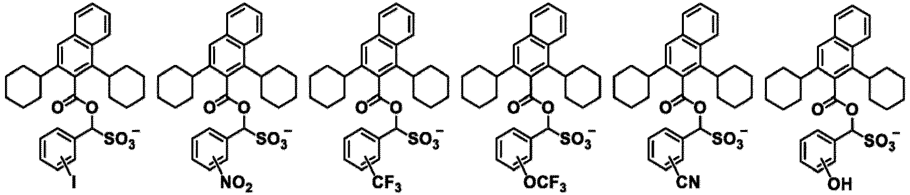

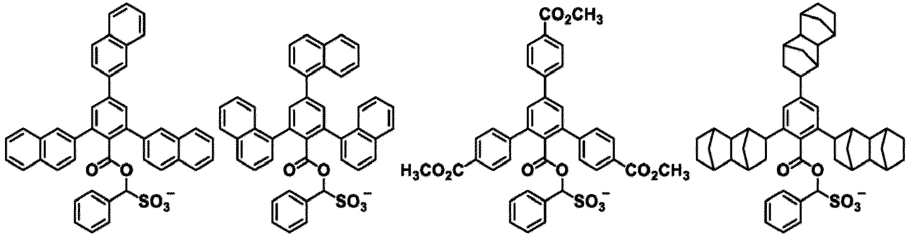

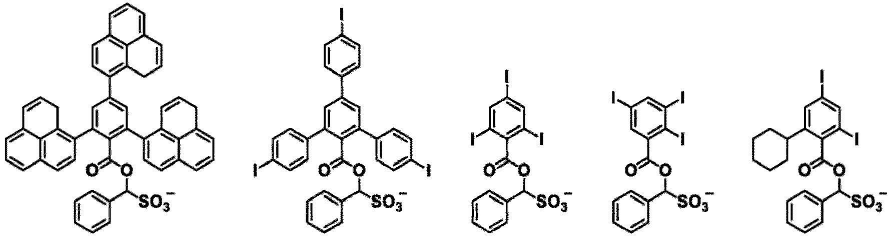

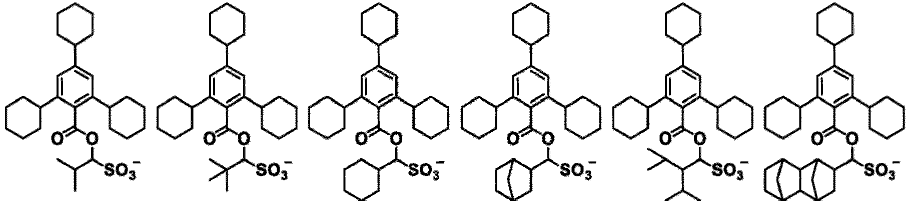

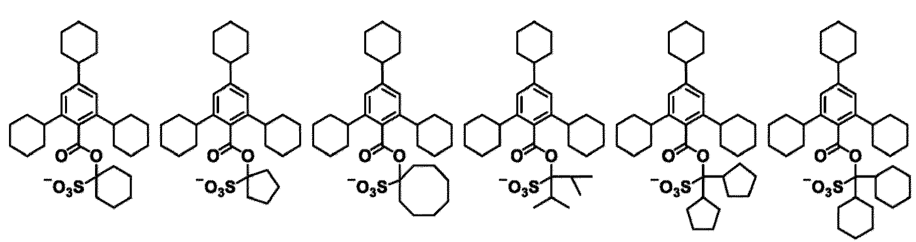

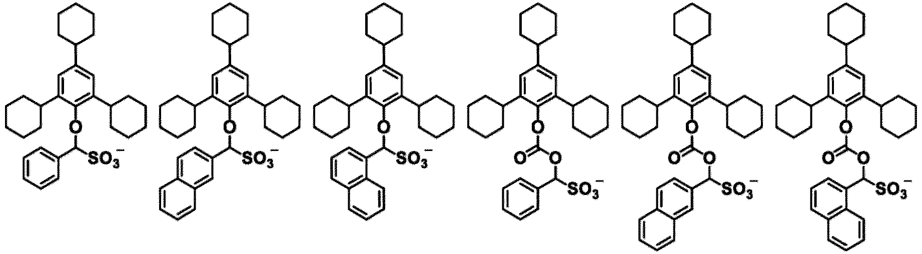

In formula (A), Z⁺ is an onium cation. The preferred onium cation is a sulfonium cation having the formula (cation-1) or iodonium cation having the formula (cation-2).

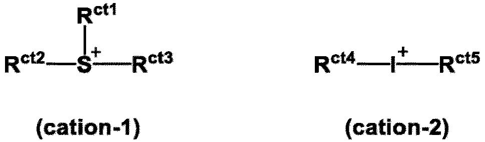

In formulae (cation-1) and (cation-2), R^{ct1} to R^{ct5} are each independently halogen or a C₁-C₃₀ hydrocarbyl group which may contain a heteroatom.

Suitable halogen atoms include fluorine, chlorine, bromine and iodine.

The hydrocarbyl group may be saturated or unsaturated and straight, branched or cyclic. Examples thereof include C₁-C₃₀ alkyl groups such as methyl, ethyl, n-propyl, isopropyl, n-butyl, isobutyl, sec-butyl, and tert-butyl; C₃-C₃₀ cyclic saturated hydrocarbyl groups such as cyclopropyl, cyclopentyl, cyclohexyl, cyclopropylmethyl, 4-methylcyclohexyl, cyclohexylmethyl, norbornyl, and adamantyl; C₂-C₃₀ alkenyl groups such as vinyl, allyl, propenyl, butenyl, and hexenyl; C₃-C₃₀ cyclic unsaturated hydrocarbyl groups such as cyclohexenyl; C₆-C₃₀ aryl groups such as phenyl, naphthyl and thienyl; C₇-C₃₀ aralkyl groups such as benzyl, 1-phenylethyl and 2-phenylethyl, and combinations thereof. Inter alia, aryl groups are preferred. In the hydrocarbyl group, some or all of the hydrogen atoms may be substituted by a moiety containing a heteroatom such as oxygen, sulfur, nitrogen or halogen and some constituent -CH₂- may be replaced by a moiety containing a heteroatom such as oxygen, sulfur or nitrogen, so that the group may contain a hydroxy, cyano, fluorine, chlorine, bromine, iodine, carbonyl, ether bond, ester bond, sulfonic ester bond, carbonate bond, lactone ring, sultone ring, carboxylic anhydride (-C(=O)-O-C(=O)-), or haloalkyl moiety.

Also, R^{ct1} and R^{ct2} may bond together to form a ring with the sulfur atom to which they are attached. Examples of the sulfonium cation having formula (cation-1) wherein R^{ct1} and R^{ct2} form a ring are shown below.

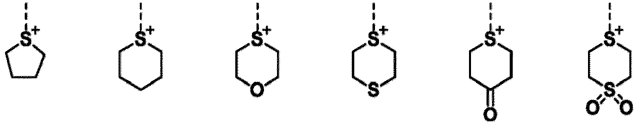

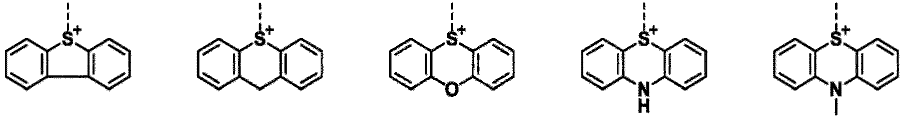

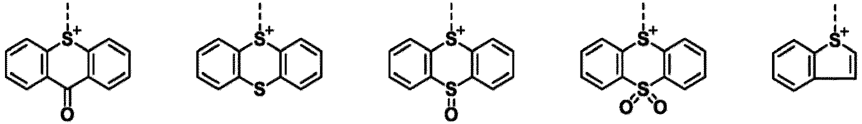

Herein the broken line designates a point of attachment to R^{ct3}.

Examples of the sulfonium cation having formula (cation-1) are shown below, but not limited thereto.

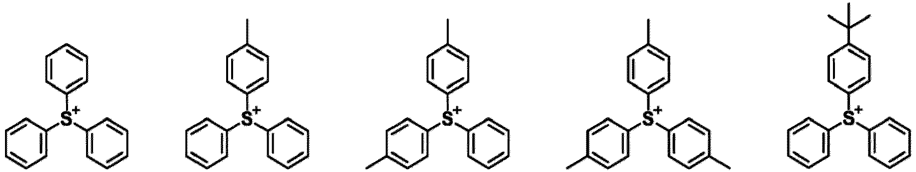

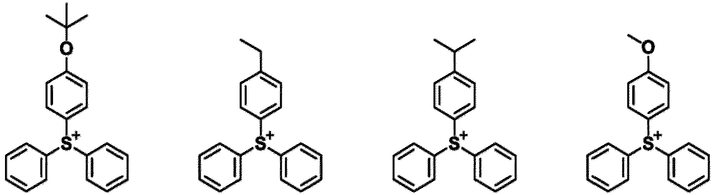

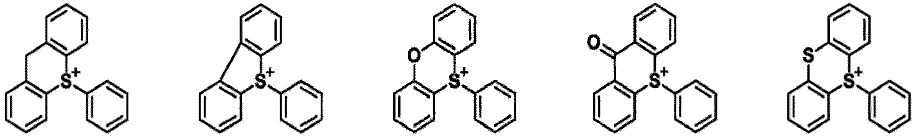

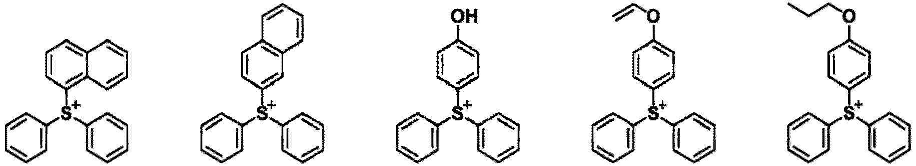

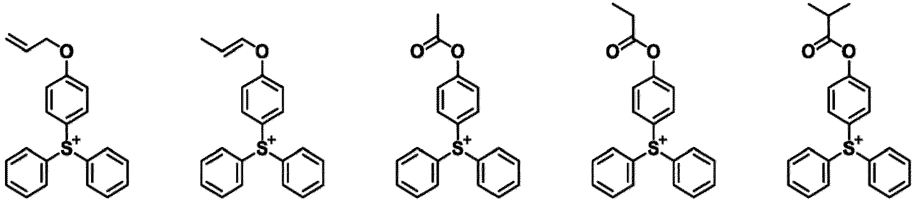

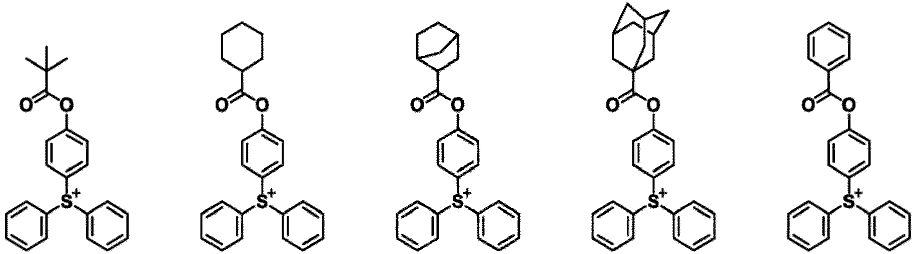

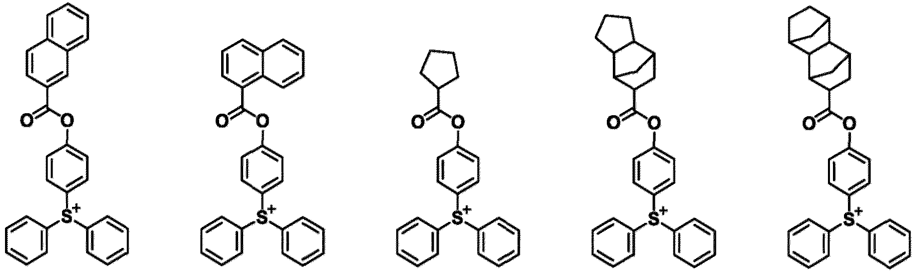

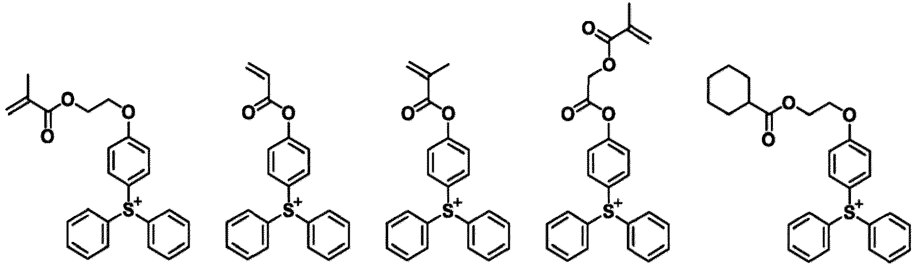

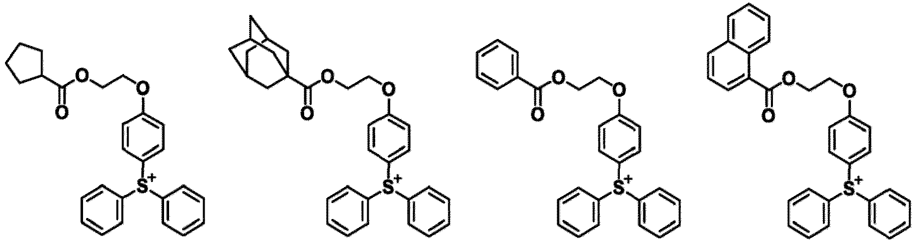

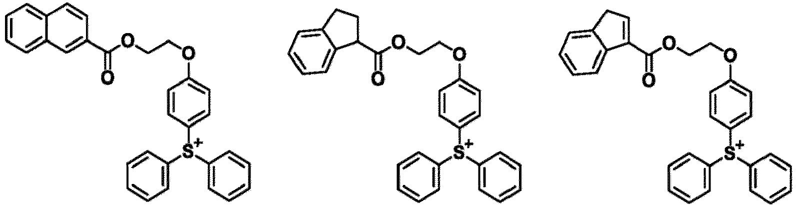

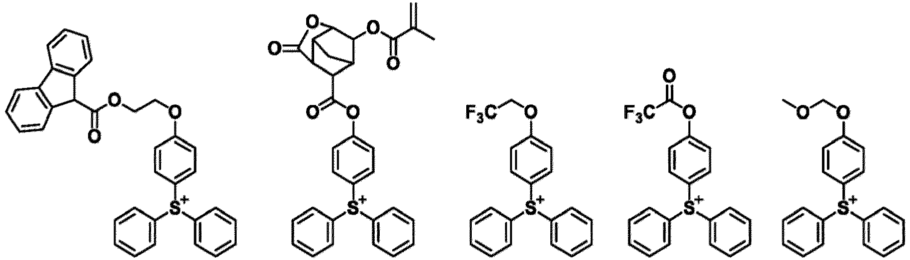

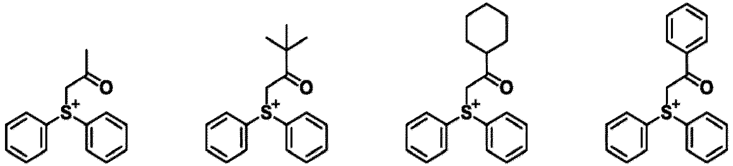

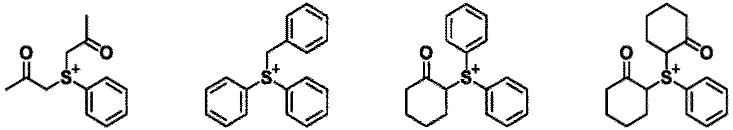

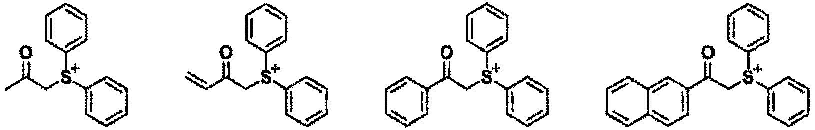

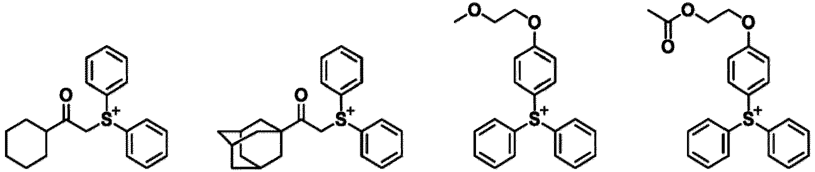

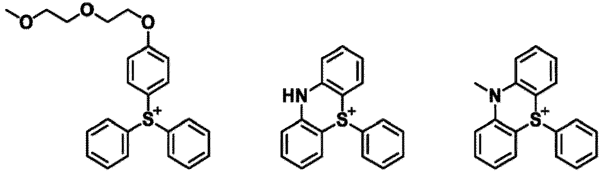

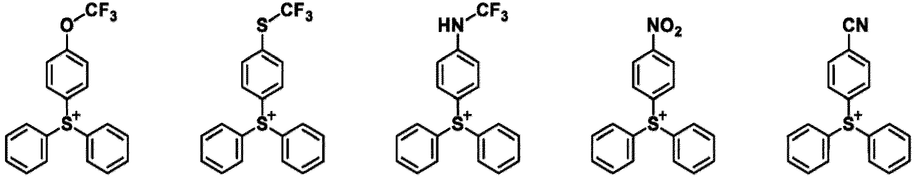

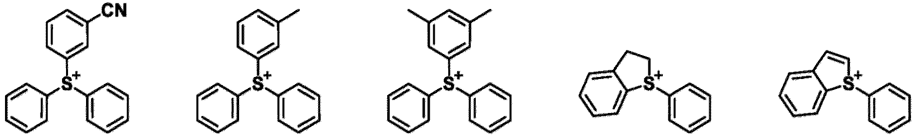

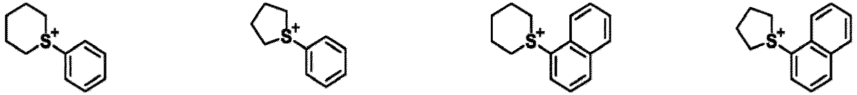

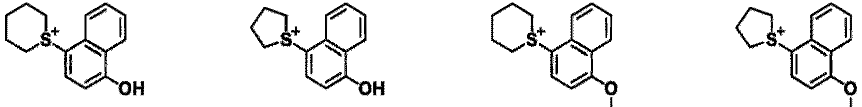

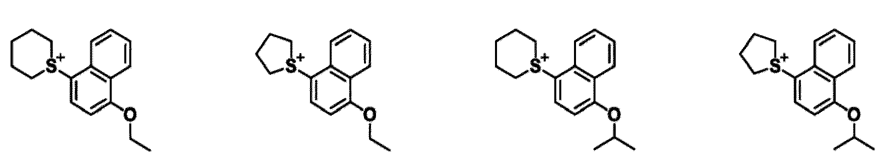

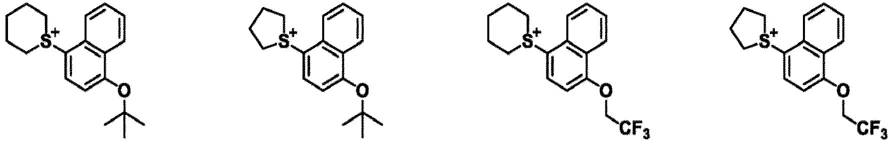

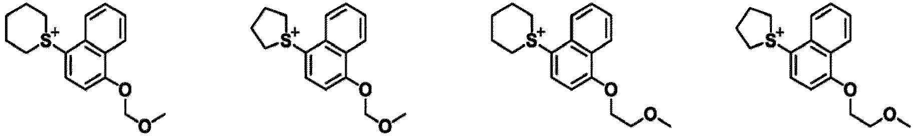

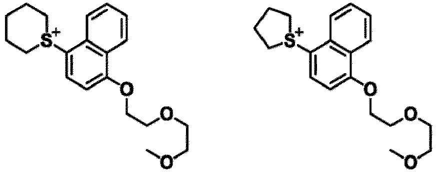

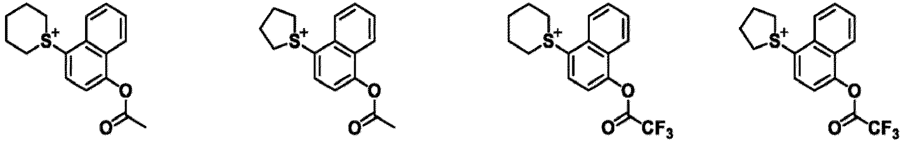

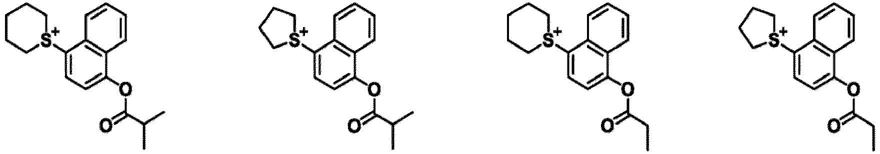

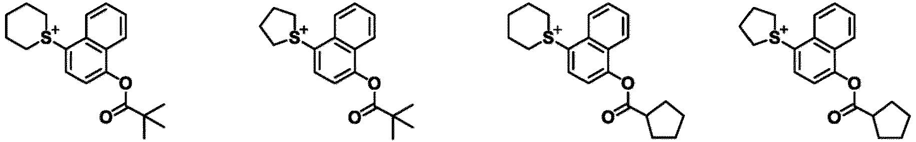

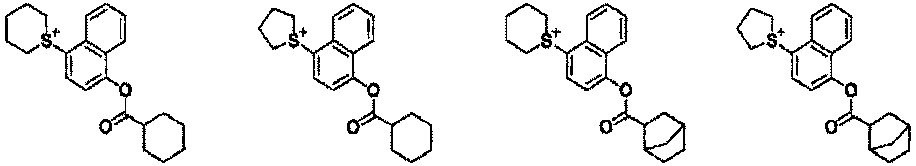

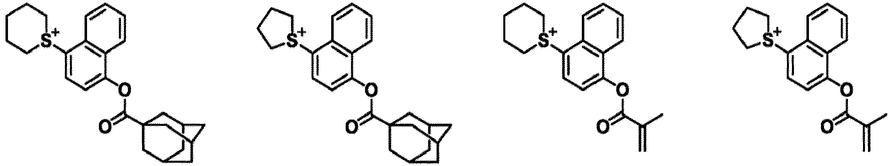

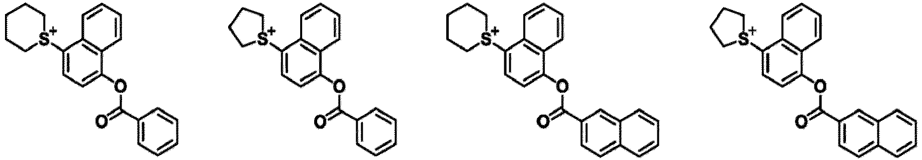

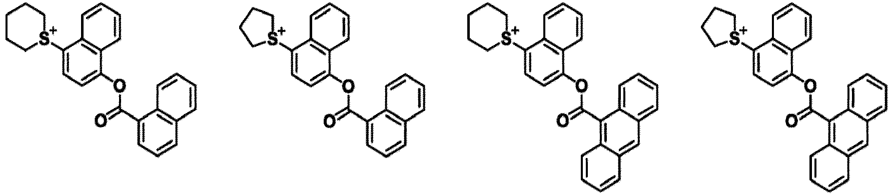

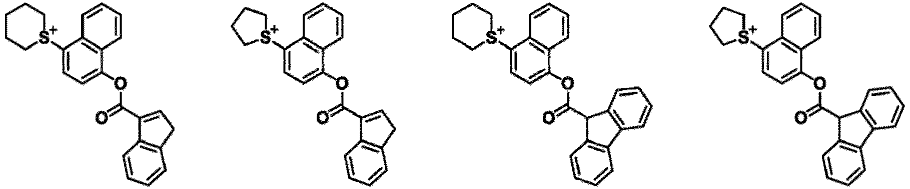

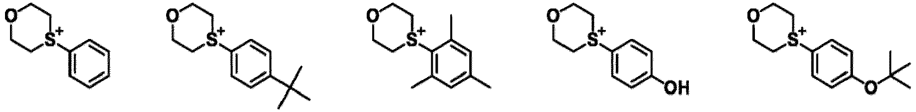

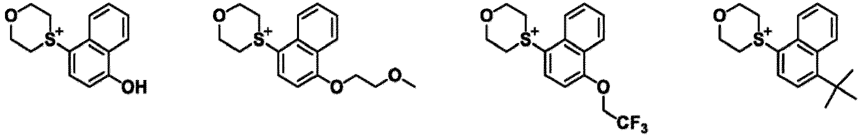

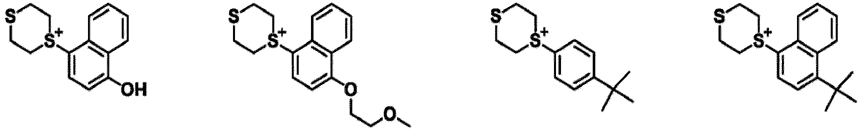

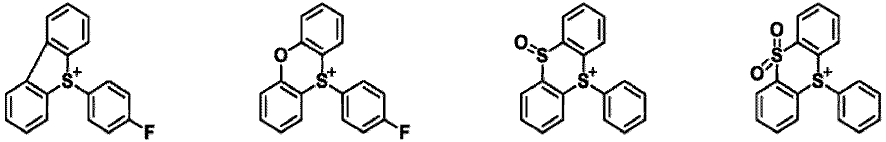

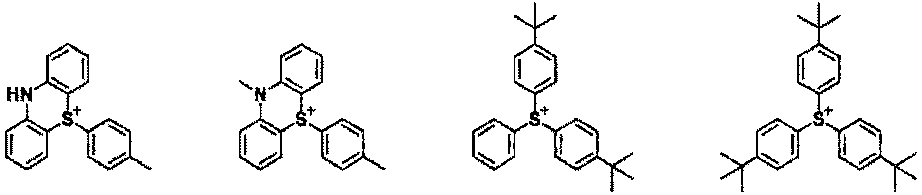

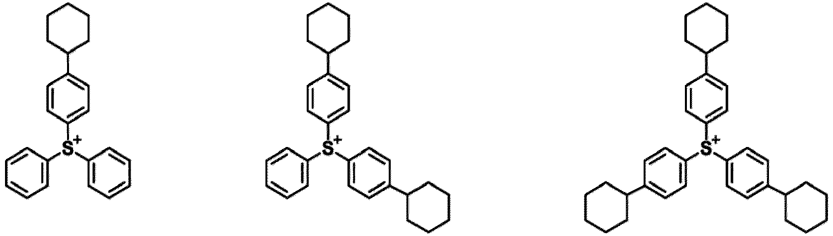

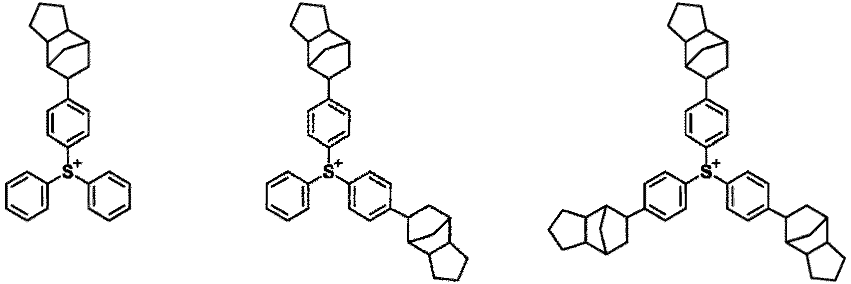

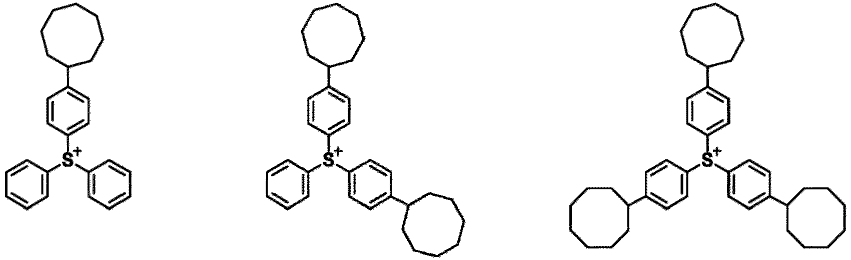

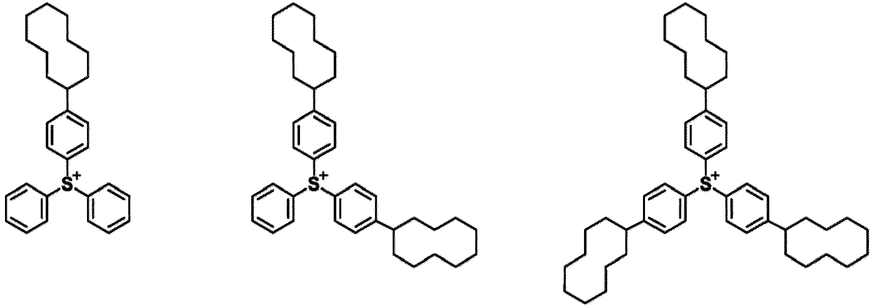

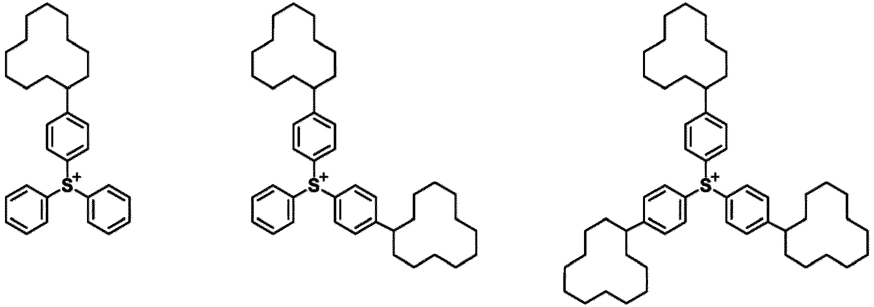

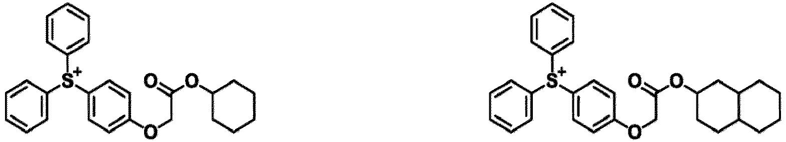

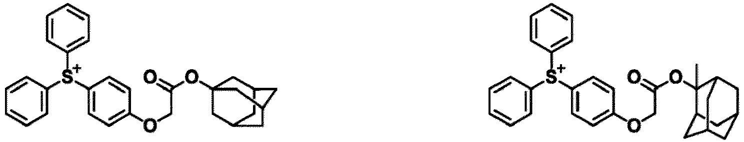

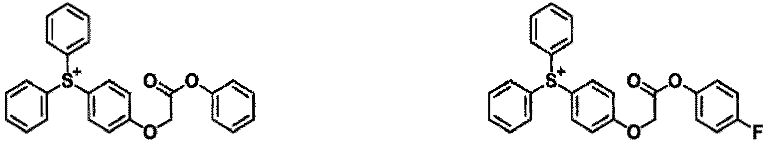

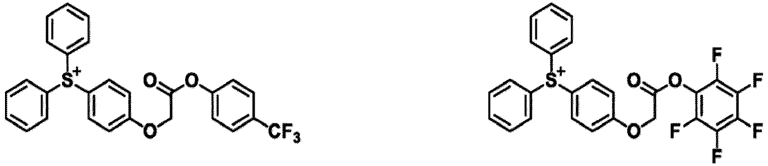

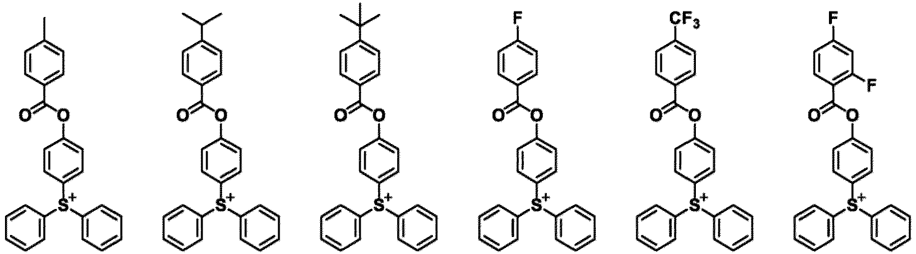

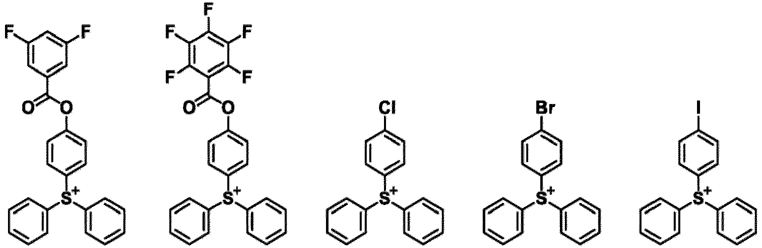

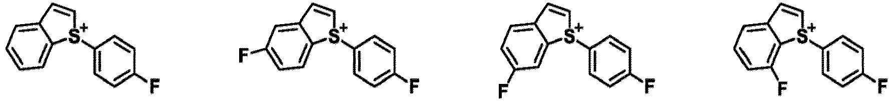

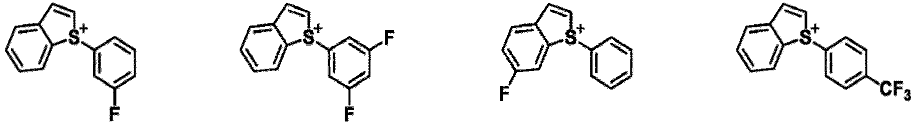

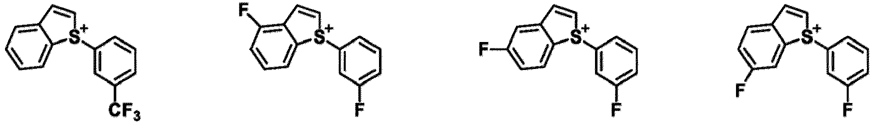

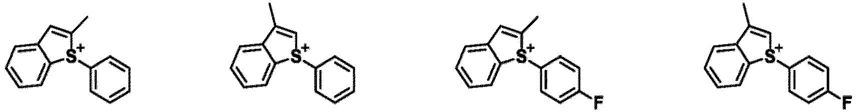

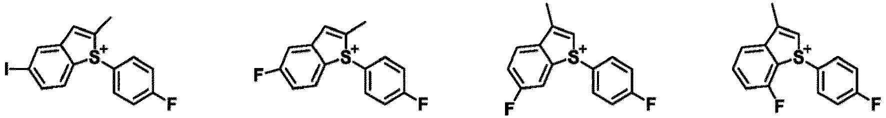

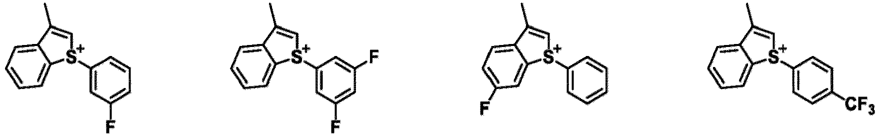

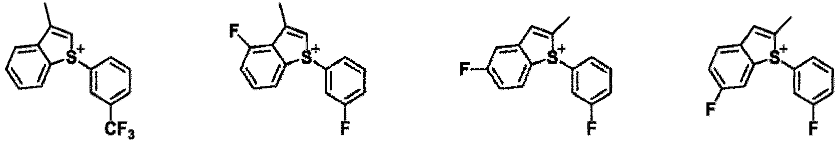

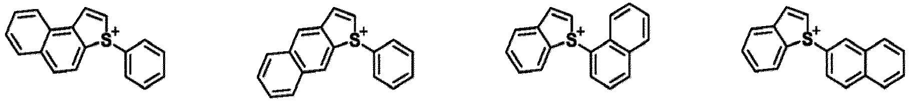

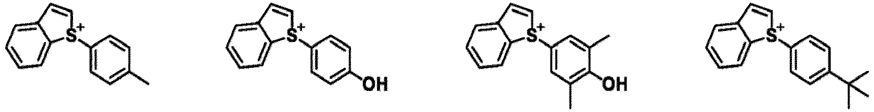

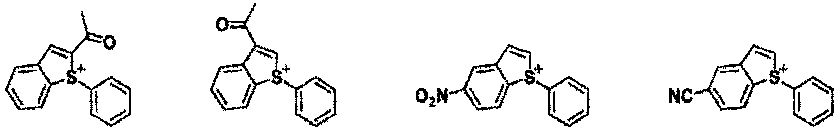

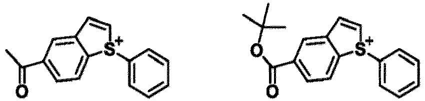

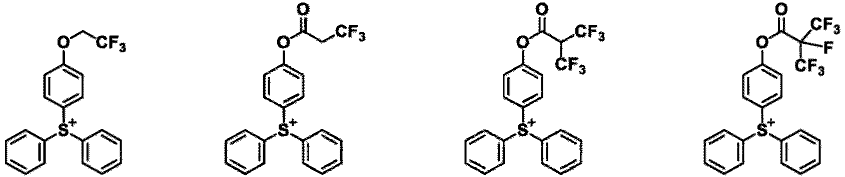

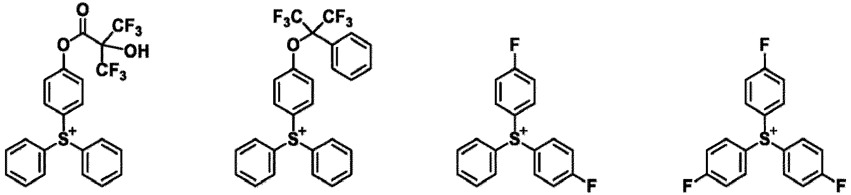

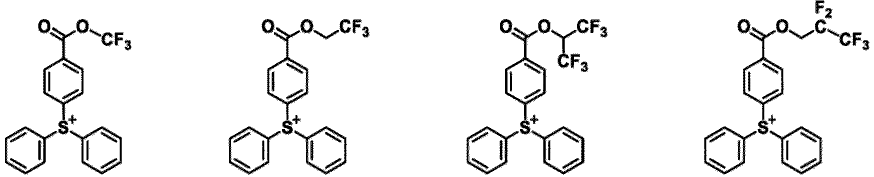

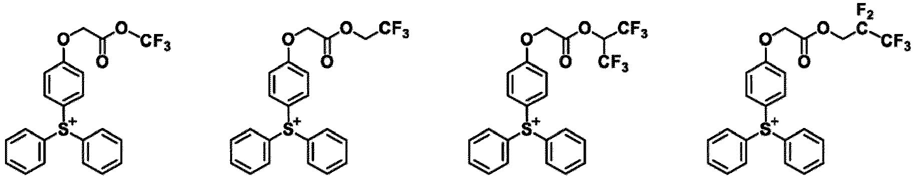

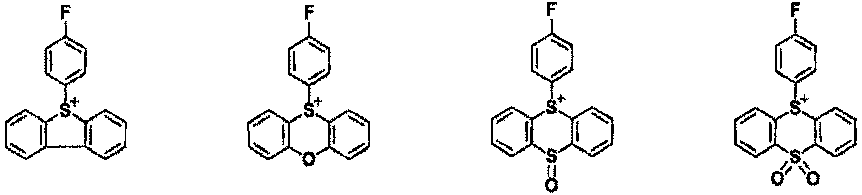

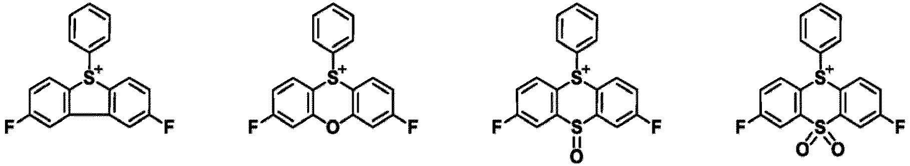

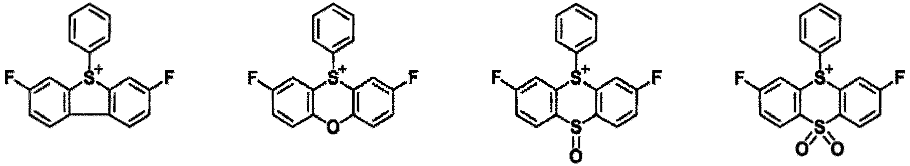

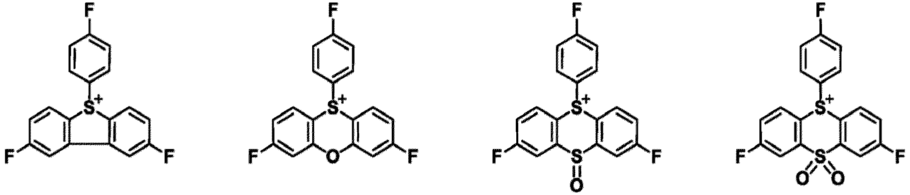

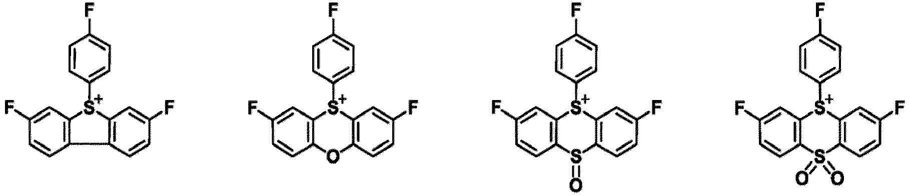

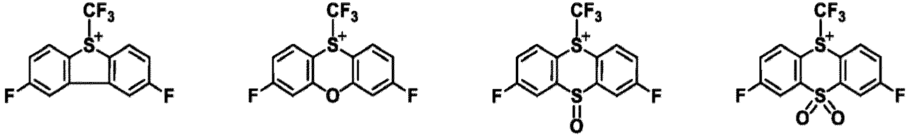

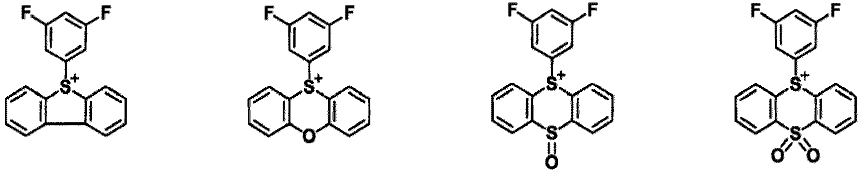

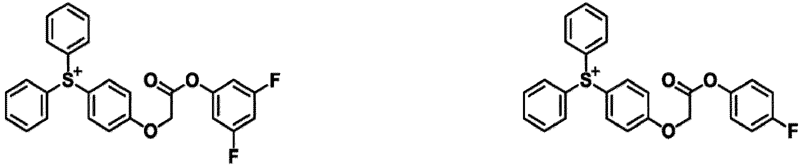

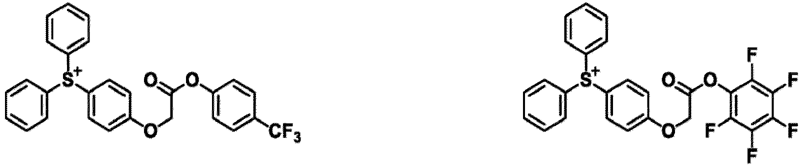

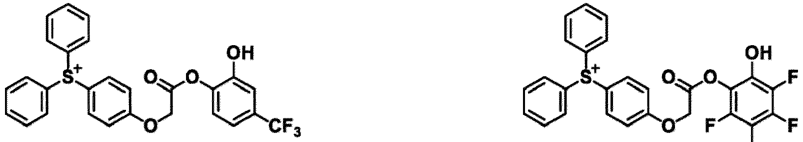

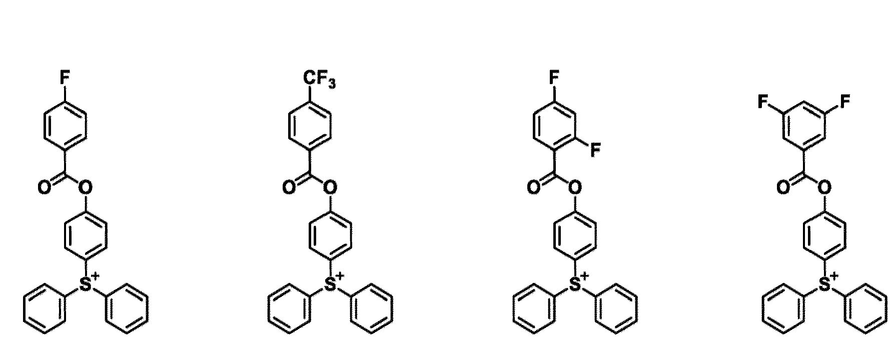

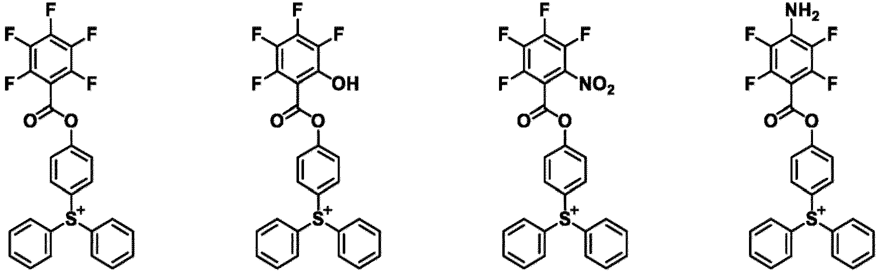

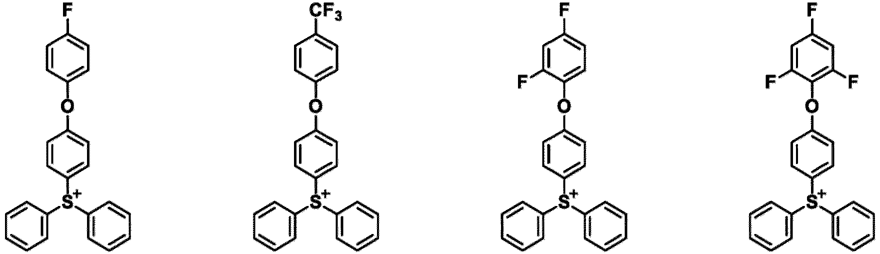

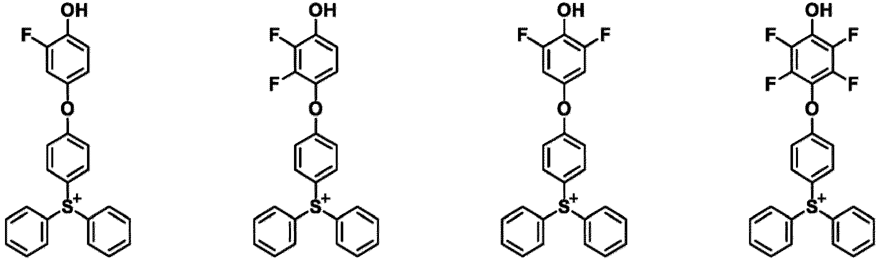

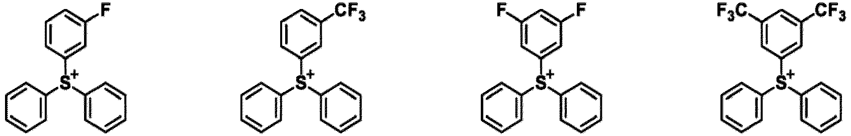

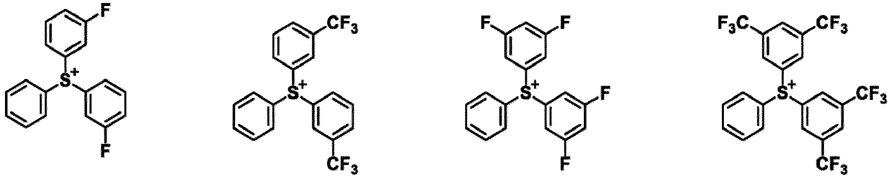

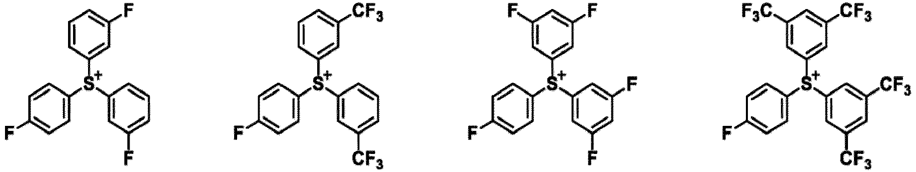

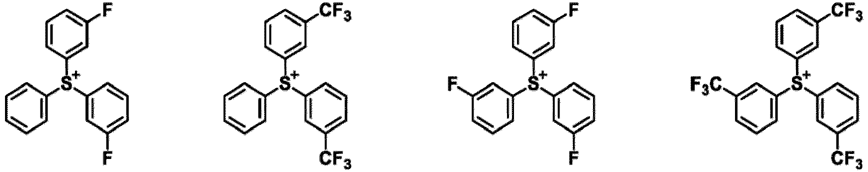

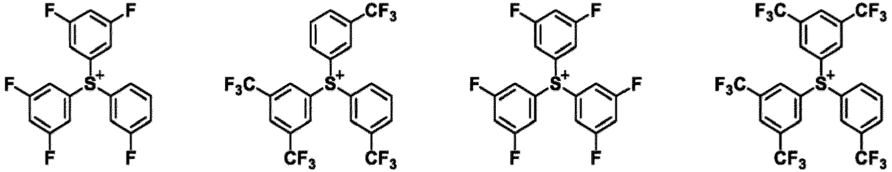

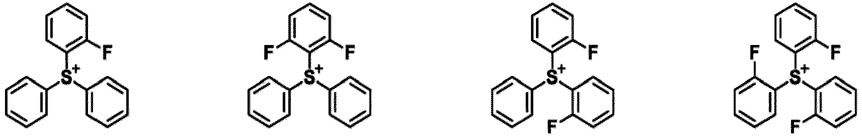

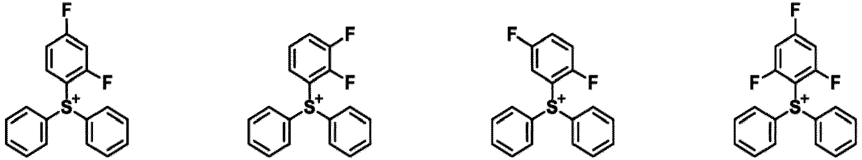

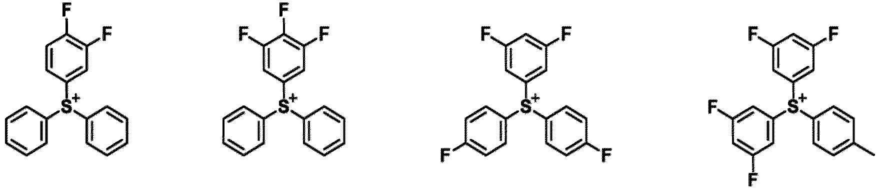

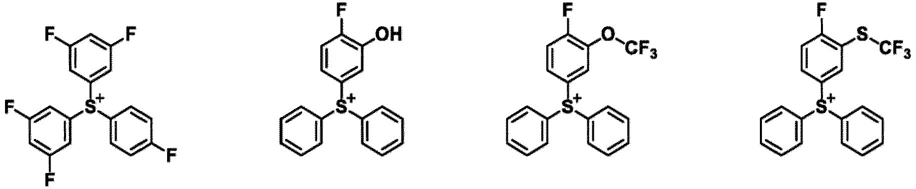

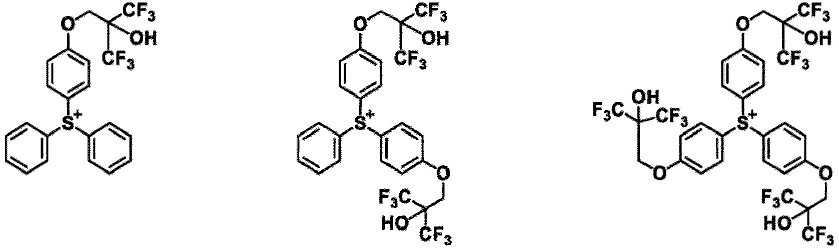

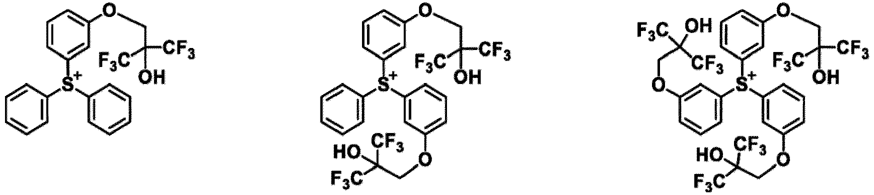

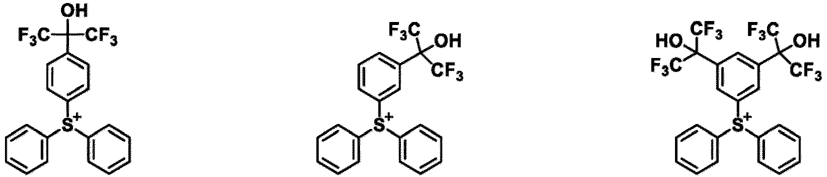

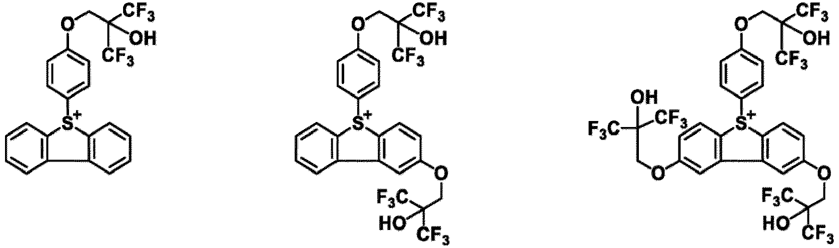

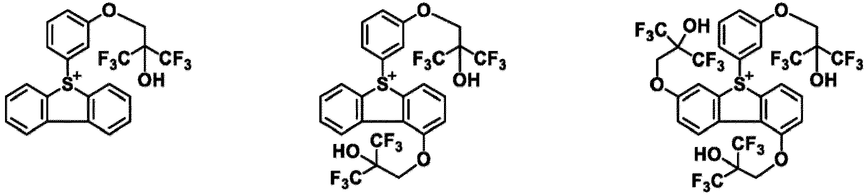

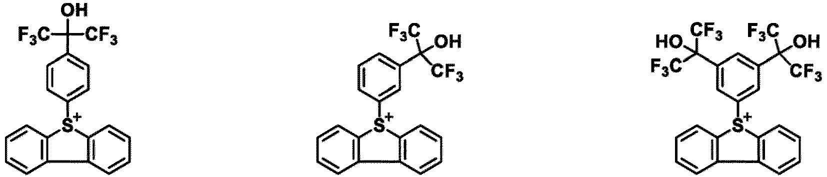

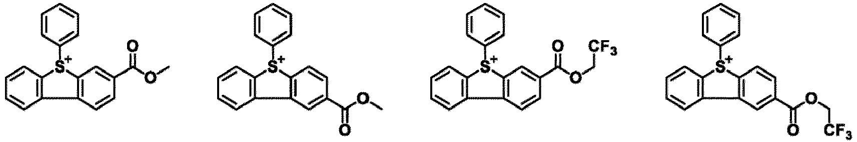

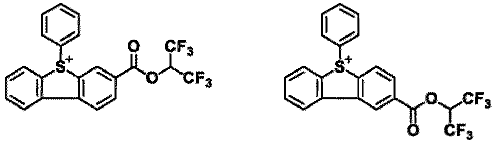

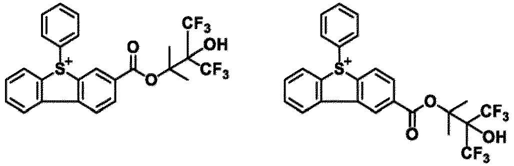

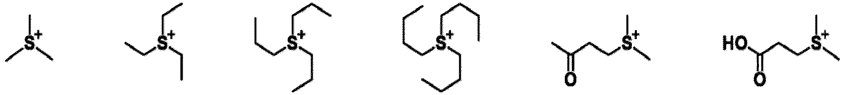

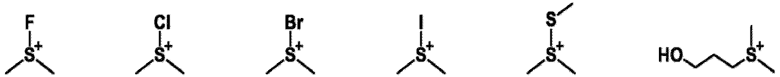

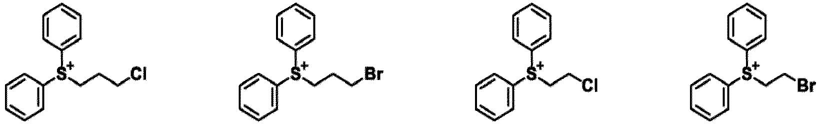

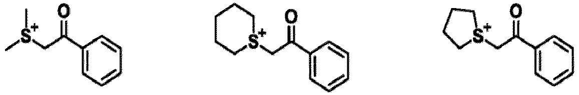

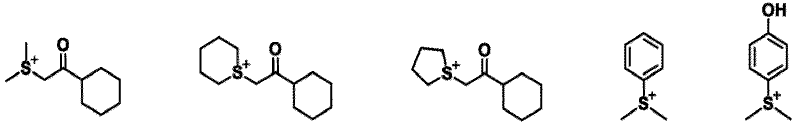

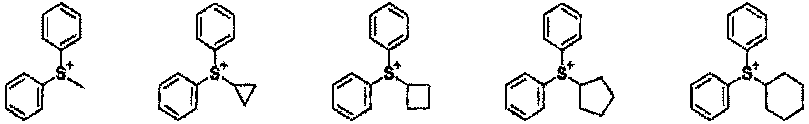

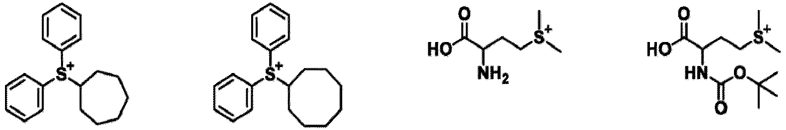

Examples of the iodonium cation having formula (cation-2) are shown below, but not limited thereto.

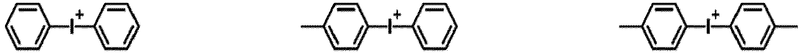

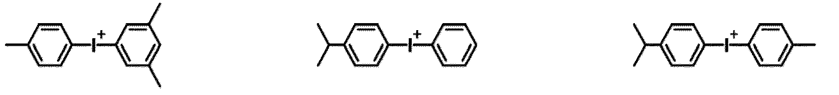

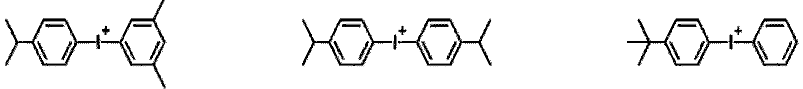

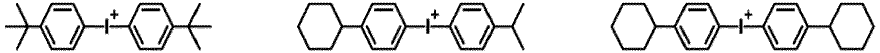

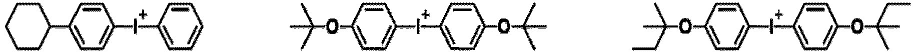

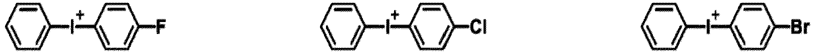

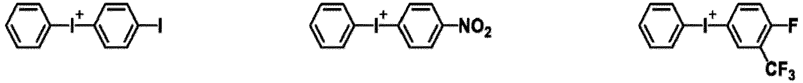

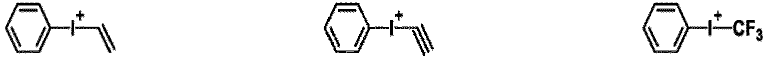

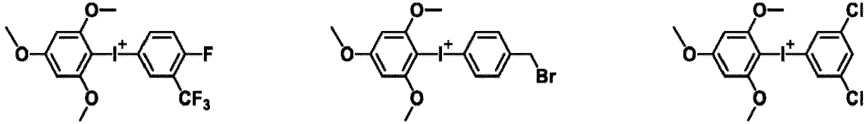

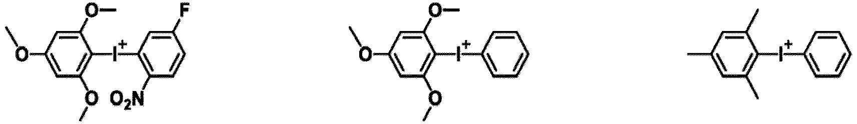

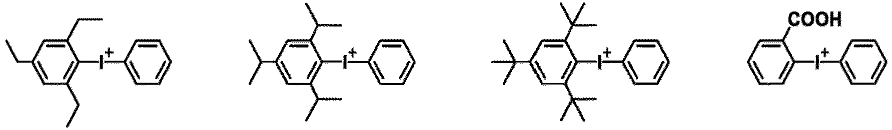

Specific structures of the onium salt include arbitrary combinations of the anion with the cation, both as exemplified above.

The onium salt is synthesized by any well-known methods. For example, it is described how to prepare an onium salt having formula (A) wherein L is a sulfonic ester bond, that is, the following formula (A'). 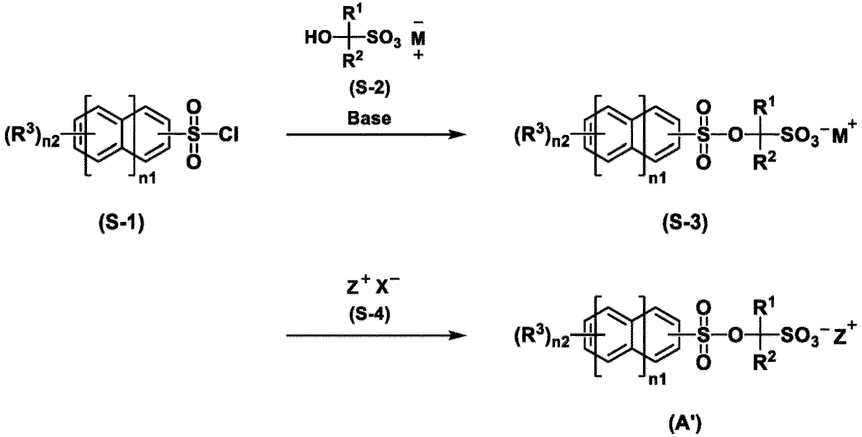 Herein n1, n2, R¹, R², R³, and Z⁺ are as defined above, M⁺ is a lithium, sodium or potassium ion, and X⁻ is a halide ion or methylsulfate ion.

The first step is to react sulfonic acid chloride (S-1) with hydroxysulfonic acid salt (S-2) to form sulfonic acid salt (S-3). The reaction may be conducted in a standard way, typically by sequentially or simultaneously adding sulfonic acid chloride (S-1), hydroxysulfonic acid salt (S-2), and a base to a solvent, and optionally cooling or heating.

Examples of the solvent which can be used in the first step include water; ethers such as tetrahydrofuran (THF), diethyl ether, diisopropyl ether, di-n-butyl ether and 1,4-dioxane; hydrocarbons such as n-hexane, n-heptane, benzene, toluene, and xylene; aprotic polar solvents such as acetonitrile, dimethyl sulfoxide (DMSO), and N,N-dimethylformamide (DMF); and chlorine organic solvents such as methylene chloride, chloroform and carbon tetrachloride. A suitable solvent may be chosen from these solvents in accordance with reaction conditions while the solvents may be used alone or in admixture.

Examples of the base which can be used in the first step include ammonia; amines such as triethylamine, pyridine, lutidine, collidine, and N,N-dimethylaniline; hydroxides such as sodium hydroxide, potassium hydroxide, and tetramethylammonium hydroxide; and carbonates such as potassium carbonate and sodium hydrogencarbonate. These bases may be used alone or in admixture.

The second step is ion exchange reaction between sulfonic acid salt (S-3) and onium salt (S-4) to form onium salt (A'). The sulfonic acid salt (S-3) may be one obtained through the first step, ordinary aqueous work-up, and isolation, whereas the reaction product obtained at the end of reaction may be used without post-treatment.

When sulfonic acid salt (S-3) as isolated is used, the desired onium salt (A') is obtained by dissolving sulfonic acid salt (S-3) in a solvent, mixing the solution with onium salt (S-4), and optionally cooling or heating the reaction mixture. Examples of the solvent include water; ethers such as THF, diethyl ether, diisopropyl ether, di-n-butyl ether and 1,4-dioxane; hydrocarbons such as n-hexane, n-heptane, benzene, toluene, and xylene; aprotic polar solvents such as acetonitrile, DMSO, and DMF; and chlorine organic solvents such as methylene chloride, chloroform and carbon tetrachloride. Finally, the onium salt (A') is recovered from the reaction mixture through ordinary aqueous workup. If necessary, the salt may be purified by a standard technique such as distillation, recrystallization or chromatography.

When the reaction product obtained at the end of reaction is used without post-treatment, the desired onium salt (A') is obtained by adding onium salt (S-4) to the reaction mixture in which the reaction to synthesize sulfonic acid salt (S-3) is terminated, and optionally cooling or heating the reaction mixture. In this step, there may be added a solvent, examples of which include water; ethers such as THF, diethyl ether, diisopropyl ether, di-n-butyl ether and 1,4-dioxane; hydrocarbons such as n-hexane, n-heptane, benzene, toluene, and xylene; aprotic polar solvents such as acetonitrile, DMSO, and DMF; and chlorine organic solvents such as methylene chloride, chloroform and carbon tetrachloride. Finally, the onium salt (A') is recovered from the reaction mixture through ordinary aqueous workup. If necessary, the salt may be purified by a standard technique such as recrystallization or chromatography.

Since the onium salt having formula (A) is an onium salt of a non-fluorinated sulfonic acid, it generates an acid having an adequate strength upon exposure to high-energy radiation. Since the onium salt has a bulky substituent at α-position of -SO₃⁻ group, the generated sulfonic acid is blocked in its surrounding. Since the onium salt possesses a bulky substituent at α-position and another bulky substituent on aromatic ring, the rotation of the bond axis of L linking them is restrained by the steric hindrance. Thus, excessive diffusion of the generated acid is restrained. By virtue of the synergy of these effects, the contrast between exposed and unexposed regions is improved, and a small-size pattern with reduced roughness can be formed. In addition, since the onium salt is fully lipophilic, its preparation and handling are easy, and an appropriate dissolution inhibition is displayed in the unexposed region.

The onium salt effectively functions as a photoacid generator.

### Resist composition

Another embodiment of the invention is a chemically amplified positive resist composition essentially comprising (A) a photoacid generator in the form of the onium salt having formula (A) defined above.

### (A) Photoacid generator

In the chemically amplified positive resist composition, the PAG (A) is preferably present in an amount of 0.1 to 40 parts by weight, more preferably 1 to 20 parts by weight per 80 parts by weight of a base polymer (B) to be described later. The range of the PAG ensures that it generates an acid in an amount necessary for the deprotection of acid labile groups and provides for storage stability. The PAG may be used alone or in admixture.

### (B) Base polymer

In a preferred embodiment, the resist composition further comprises (B) a base polymer containing a polymer which is decomposed under the action of acid to increase its solubility in alkaline developer.

The polymer preferably comprises repeat units having the following formula (B1). Notably, the unit having formula (B1) is also referred to as unit B1.

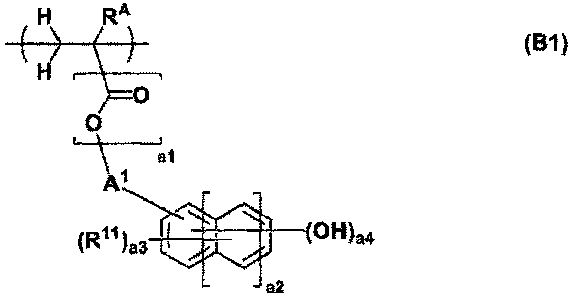

In formula (B1), a1 is 0 or 1. The subscript a2 is an integer of 0 to 2. The structure represents a benzene skeleton when a2=0, a naphthalene skeleton when a2=1, and an anthracene skeleton when a2=2. The subscript a3 is an integer satisfying 0 ≤ a3 ≤ 5+2(a2)-a4, and a4 is an integer of 1 to 3. In case of a2=0, preferably a3 is an integer of 0 to 3, and a4 is an integer of 1 to 3. In case of a2=1 or 2, preferably a3 is an integer of 0 to 4, and a4 is an integer of 1 to 3.

In formula (B1), R^{A} is hydrogen, fluorine, methyl or trifluoromethyl.

In formula (B1), R¹¹ is halogen, an optionally halogenated C₂-C₈ saturated hydrocarbylcarbonyloxy group, optionally halogenated C₁-C₆ saturated hydrocarbyl group, or optionally halogenated C₁-C₆ saturated hydrocarbyloxy group. The saturated hydrocarbyl group and saturated hydrocarbyl moiety in the saturated hydrocarbyloxy group and saturated hydrocarbylcarbonyloxy group may be straight, branched or cyclic, and examples thereof include alkyl groups such as methyl, ethyl, n-propyl, isopropyl, butyl, pentyl, hexyl, and structural isomers thereof, cycloalkyl groups such as cyclopropyl, cyclobutyl, cyclopentyl and cyclohexyl, and combinations thereof. A carbon count within the upper limit ensures good solubility in alkaline developer. A plurality of R¹¹ may be identical or different when a3 is 2 or more.

In formula (B1), A¹ is a single bond or C₁-C₁₀ saturated hydrocarbylene group in which some constituent -CH₂- may be replaced by -O-. The saturated hydrocarbylene group may be straight, branched or cyclic and examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of a1=1 in formula (B1), the ether bond may be incorporated at any position excluding the position between the α-carbon and β-carbon relative to the ester oxygen. In case of a1=0, the atom that bonds with the main chain becomes an ethereal oxygen, and a second ether bond may be incorporated at any position excluding the position between the α-carbon and β-carbon relative to that ethereal oxygen. Saturated hydrocarbylene groups having no more than 10 carbon atoms are desirable because of a sufficient solubility in alkaline developer.

Preferred examples of the repeat units B1 wherein a1=0 and A¹ is a single bond (meaning that the aromatic ring is directly bonded to the main chain of the polymer), that is, repeat units free of a linker: -C(=O)-O-A¹- include units derived from 3-hydroxystyrene, 4-hydroxystyrene, 5-hydroxy-2-vinylnaphthalene, and 6-hydroxy-2-vinylnaphthalene. Repeat units having the formula (B1-1) are especially preferred. 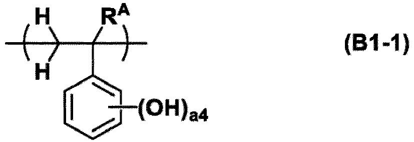 Herein R^{A} and a4 are as defined above.

Preferred examples of the repeat units B1 wherein a1=1, that is, having a linker: - C(=O)-O-A¹- are shown below, but not limited thereto. Herein R^{A} is as defined above.

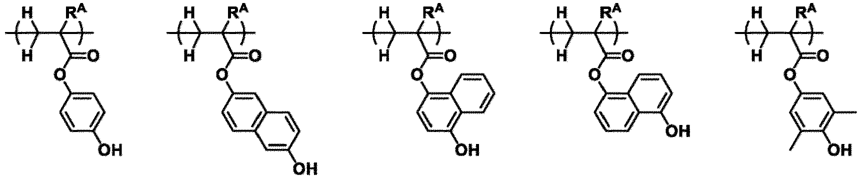

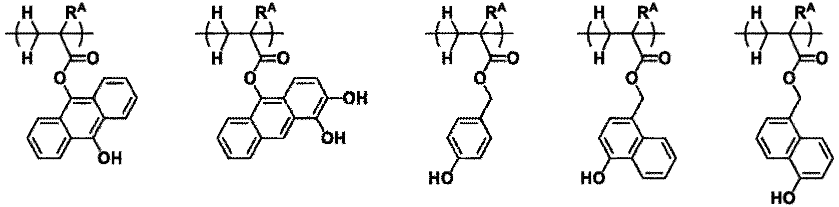

The content of repeat units B1 is preferably 30 to 90 mol%, more preferably 40 to 85 mol% of the overall repeat units of the polymer. When the polymer further contains repeat units having formula (B3) and/or repeat units having formula (B4), which provide the polymer with higher etch resistance, the repeat units having a phenolic hydroxy group as a substituent, the total content of repeat units B1 and repeat units B3 and/or B4 is preferably in the range. The repeat units B1 may be used alone or in admixture of two or more.

In a preferred embodiment, the polymer further contains repeat units B2 having an acidic functional group protected with an acid labile group (i.e., repeat units protected with an acid labile group and adapted to turn alkali soluble under the action of acid) in order that the positive resist composition in an exposed region turn soluble in alkaline developer.

Typical of the repeat unit B2 is a unit having the formula (B2-1), also referred to as repeat unit B2-1.

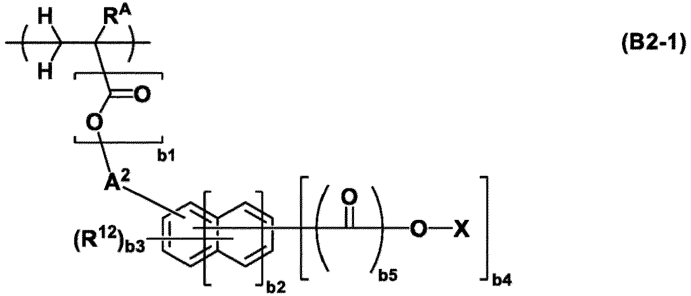

In formula (B2-1), b1 is 0 or 1. The subscript b2 is an integer of 0 to 2. The structure represents a benzene skeleton when b2=0, a naphthalene skeleton when b2=1, and an anthracene skeleton when b2=2. The subscript b3 is an integer meeting 0 <_ b3 ≤ 5+2(b2)-b4. The subscript b4 is an integer of 1 to 3, and b5 is 0 or 1. When b2=0, preferably b3 is an integer of 0 to 3 and b4 is an integer of 1 to 3. When b2=1 or 2, preferably b3 is an integer of 0 to 4 and b4 is an integer of 1 to 3.

In formula (B2-1), R^{A} is hydrogen, fluorine, methyl or trifluoromethyl.

In formula (B2-1), R¹² is halogen, an optionally halogenated C₂-C₈ saturated hydrocarbylcarbonyloxy group, optionally halogenated C₁-C₆ saturated hydrocarbyl group, or optionally halogenated C₁-C₆ saturated hydrocarbyloxy group. The saturated hydrocarbyl group and saturated hydrocarbyl moiety in the saturated hydrocarbyloxy group and saturated hydrocarbylcarbonyloxy group may be straight, branched or cyclic, and examples thereof include alkyl groups such as methyl, ethyl, n-propyl, isopropyl, butyl, pentyl, hexyl, and structural isomers thereof, cycloalkyl groups such as cyclopropyl, cyclobutyl, cyclopentyl and cyclohexyl, and combinations thereof. A carbon count within the upper limit ensures good solubility in alkaline developer. A plurality of R¹² may be identical or different when b3 is 2 or more.

In formula (B2-1), A² is a single bond or a C₁-C₁₀ saturated hydrocarbylene group in which some constituent -CH₂- may be replaced by -O-. The saturated hydrocarbylene group may be straight, branched or cyclic and examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of b1=1 in formula (B2-1), the ether bond may be incorporated at any position excluding the position between the α-carbon and β-carbon relative to the ester oxygen. In case of b1=0, the atom that bonds with the main chain becomes an ethereal oxygen, and a second ether bond may be incorporated at any position excluding the position between the α-carbon and β-carbon relative to that ethereal oxygen. Saturated hydrocarbylene groups having no more than 10 carbon atoms are desirable because of a sufficient solubility in alkaline developer.

In formula (B2-1), X is an acid labile group when b4=1, and hydrogen or an acid labile group, at least one X being an acid labile group, when b4=2 or 3. That is, repeat units B2-1 have phenolic hydroxy groups bonded to an aromatic ring, at least one of which is protected with an acid labile group, or repeat units B2-1 have a carboxy group bonded to an aromatic ring, which is protected with an acid labile group. The acid labile group used herein is not particularly limited as long as it is commonly used in a number of well-known chemically amplified resist compositions and eliminated under the action of acid to release an acidic group.

Typical of the acid labile group is a tertiary saturated hydrocarbyl group. The tertiary saturated hydrocarbyl group is preferably of 4 to 18 carbon atoms because a monomer for use in polymerization is recoverable by distillation.

The saturated hydrocarbyl group bonded to the tertiary carbon atom in the tertiary saturated hydrocarbyl group is preferably of 1 to 15 carbon atoms. The C₁-C₁₅ saturated hydrocarbyl group may be straight, branched or cyclic and contain an oxygen-containing functional group such as an ether bond or carbonyl group in its carbon-carbon bond. The saturated hydrocarbyl groups bonded to the tertiary carbon atom may bond together to form a ring with the tertiary carbon atom to which they are attached.

Examples of the alkyl substituent include methyl, ethyl, propyl, adamantyl, norbornyl, tetrahydrofuran-2-yl, 7-oxanorbornan-2-yl, cyclopentyl, 2-tetrahydrofuryl, tricyclo[5.2.1.0^{2,6}]decyl, 8-ethyl-8-tricyclo[5.2.1.0^{2,6}]decyl, 3-methyl-3-tetracyclo[4.4.0.1^{2,5}.1^{7,10}]dodecyl, tetracyclo[4.4.0.1^{2,5}.1^{7,10}]dodecyl, and 3-oxo-1-cyclohexyl.

Examples of the tertiary saturated hydrocarbyl group include, but are not limited to, tert-butyl, tert-pentyl, 1-ethyl-1-methylpropyl, 1,1-diethylpropyl, 1,1,2-trimethylpropyl, 1-adamantyl-1-methylethyl, 1-methyl-1-(2-norbornyl)ethyl, 1-methyl-1-(tetrahydrofuran-2-yl)ethyl, 1-methyl-1-(7 -oxanorbornan-2-yl)ethyl, 1-methylcyclopentyl, 1-ethylcyclopentyl, 1-propylcyclopentyl, 1-cyclopentylcyclopentyl, 1-cyclohexylcyclopentyl, 1-(2-tetrahydrofuryl)cyclopentyl, 1-(7-oxanorbornan-2-yl)cyclopentyl, 1-methylcyclohexyl, 1-ethylcyclohexyl, 1-cyclopentylcyclohexyl, 1-cyclohexylcyclohexyl, 2-methyl-2-norbornyl, 2-ethyl-2-norbornyl, 8-methyl-8-tricyclo[5.2.1.0^{2,6}]decyl, 8-ethyl-8-tricyclo[5.2.1.0^{2,6}]decyl, 3-methyl-3-tetracyclo[4.4.0.1^{2,5}.1^{7,10}]dodecyl, 3-ethyl-3-tetracyclo[4.4.0.1^{2,5}.1^{7,10}]dodecyl, 2-methyl-2-adamantyl, 2-ethyl-2-adamantyl, 1-methyl-3-oxo-1-cyclohexyl, 1-methyl-1-(tetrahydrofuran-2-yl)ethyl, 5-hydroxy-2-methyl-2-adamantyl, and 5-hydroxy-2-ethyl-2-adamantyl.

A group having the following formula (B2-1-1) is also suitable as the acid labile group. The group having formula (B2-1-1) is often used as the acid labile group. It is a good choice of the acid labile group that ensures to form a pattern having a relatively rectangular pattern-substrate interface in a consistent manner. An acetal structure is formed when X is a group having formula (B2-1-1).

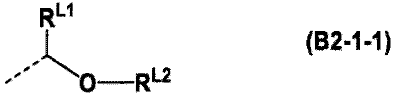

In formula (B2-1-1), R^{L1} is hydrogen or a C₁-C₁₀ saturated hydrocarbyl group. The saturated hydrocarbyl group may be straight, branched or cyclic.

A choice of R^{L1} may depend on the designed sensitivity of labile group to acid. For example, hydrogen is selected when the acid labile group is designed to ensure relatively high stability and to be decomposed with strong acid. A straight alkyl group is selected when the acid labile group is designed to have relatively high reactivity and high sensitivity to pH changes. Although the choice varies with a particular combination of acid generator and quencher in the resist composition, R^{L1} is preferably a group in which the carbon in bond with acetal carbon is secondary, when R^{L2} is a relatively large alkyl group substituted at the end and the acid labile group is designed to undergo a substantial change of solubility by decomposition. Examples of R^{L1} bonded to acetal carbon via secondary carbon include isopropyl, sec-butyl, cyclopentyl, and cyclohexyl, but are not limited thereto.

In formula (B2-1-1), R^{L2} is a C₁-C₃₀ hydrocarbyl group. The hydrocarbyl group may be saturated or unsaturated and straight, branched or cyclic. Illustrative are C₁-C₃₀ saturated hydrocarbyl groups and C₆-C₃₀ aryl groups. R^{L2} is preferably a C₁-C₆ hydrocarbyl group for acquiring a higher resolution in forming small-size patterns. When R^{L2} is a C₁-C₆ hydrocarbyl group, the alcohol created after a progress of acid-aided deprotection reaction is water soluble. Then, when a positive pattern is formed using an alkaline developer, the alcohol is dissolved in the developer so that defects remaining in the exposed region are minimized.

Preferred examples of the group having formula (B2-1-1) are given below, but not limited thereto. Herein R^{L1} is as defined above.

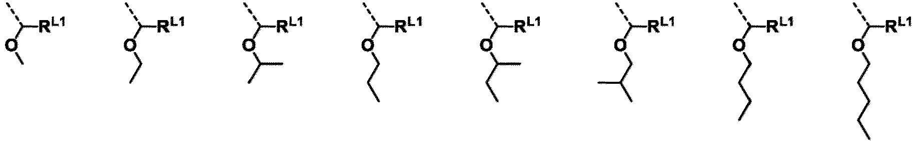

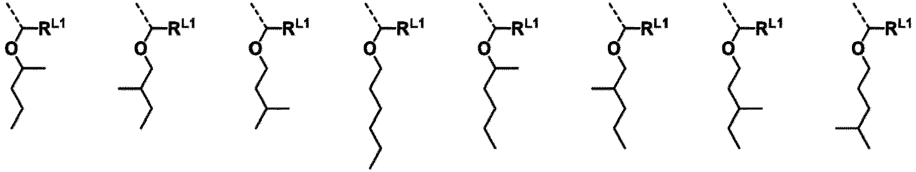

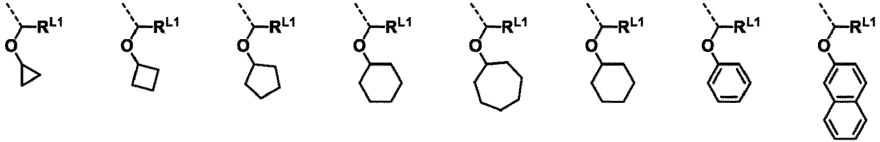

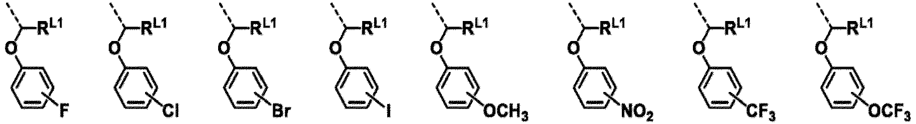

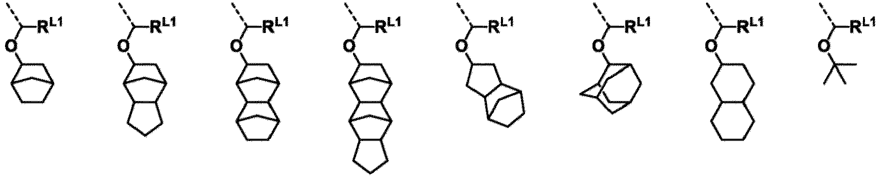

Another acid labile group which can be used herein is a phenolic hydroxy group whose hydrogen is substituted by -CH₂COO-(tertiary saturated hydrocarbyl group). The tertiary saturated hydrocarbyl group may be the same as the foregoing tertiary saturated hydrocarbyl group used for the protection of a phenolic hydroxy group.

Another example of repeat unit B2 is a repeat unit having the following formula (B2-2), referred to as repeat unit B2-2. The repeat unit B2-2 which enables to increase the dissolution rate in the exposed region is a useful choice of the acid labile group-containing unit which affords satisfactory performance against line width variations during develop loading.

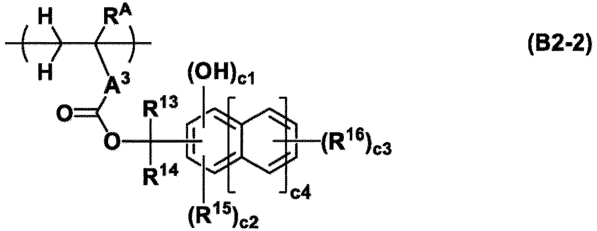

In formula (B2-2), c1 is an integer of 0 to 2, c2 is an integer of 0 to 2, c3 is an integer of 0 to 5, and c4 is an integer of 0 to 2.

In formula (B2-2), R^{A} is hydrogen, fluorine, methyl or trifluoromethyl.

In formula (B2-2), R¹³ and R¹⁴ are each independently a C₁-C₁₀ hydrocarbyl group which may contain a heteroatom. R¹³ and R¹⁴ may bond together to form a ring with the carbon atom to which they are attached.

In formula (B2-2), R¹⁵ is each independently fluorine, C₁-C₅ fluorinated alkyl group or C₁-C₅ fluorinated alkoxy group.

In formula (B2-2), R¹⁶ is each independently a C₁-C₁₀ hydrocarbyl group which may contain a heteroatom.

In formula (B2-2), A³ is a single bond, phenylene group, naphthylene group, or *-C(=O)-O-A³¹-. A³¹ is a C₁-C₂₀ aliphatic hydrocarbylene group which may contain hydroxy, ether bond, ester bond or lactone ring, or phenylene or naphthylene group, and * is a point of attachment to the carbon atom in the backbone.

Preferred examples of the repeat unit B2-2 are shown below, but not limited thereto. Herein R^{A} is as defined above.

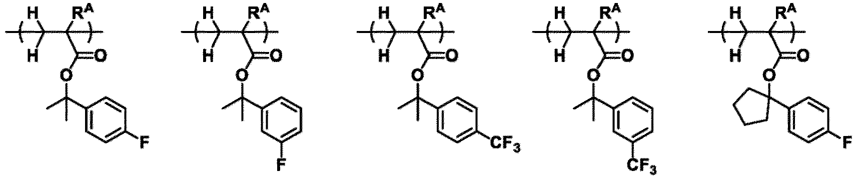

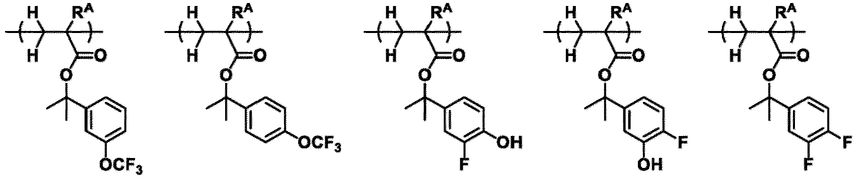

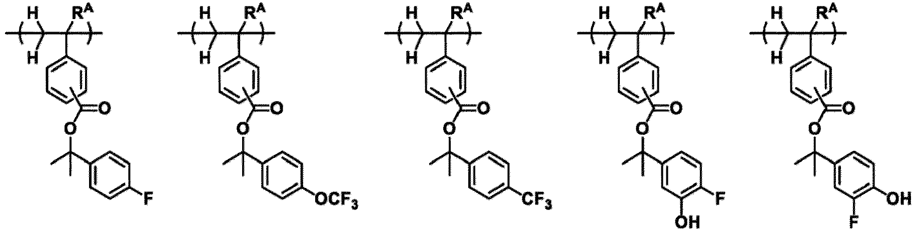

The content of repeat units B2 is preferably 5 to 95 mol%, more preferably 20 to 80 mol% based on the overall repeat units of the polymer. Each of repeat units B2 may be of one type or a mixture of two or more types.

In a preferred embodiment, the polymer further comprises repeat units of at least one type selected from units having the formulae (B3), (B4) and (B5). These repeat units are simply referred to as repeat units B3, B4 and B5, respectively.

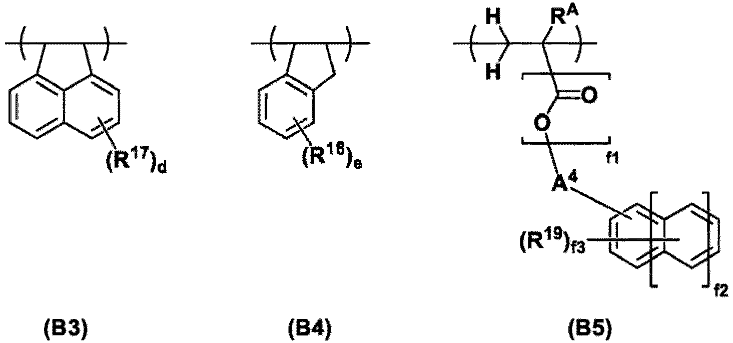

In formulae (B3) and (B4), d is an integer of 0 to 6 and e is an integer of 0 to 4.

In formulae (B3) and (B4), R¹⁷ and R¹⁸ are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group. The saturated hydrocarbyl group, saturated hydrocarbyloxy group and saturated hydrocarbylcarbonyloxy group may be straight, branched or cyclic. When d is 2 or more, a plurality of groups R¹⁷ may be identical or different. When e is 2 or more, a plurality of groups R¹⁸ may be identical or different.

In formula (B5), f1 is 0 or 1. The subscript f2 is an integer of 0 to 2, and the corresponding structure represents a benzene skeleton when f2=0, a naphthalene skeleton when f2=1, and an anthracene skeleton when f2=2. The subscript f3 is an integer of 0 to 5. In case of f2=0, preferably f3 is an integer of 0 to 3. In case of f2=1 or 2, preferably f3 is an integer of 0 to 4.

In formula (B5), R^{A} is hydrogen, fluorine, methyl or trifluoromethyl.

In formula (B5), R¹⁹ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen atom, nitro group, or cyano group. In case of f2=1 or 2, R¹⁹ may also be hydroxy. The saturated hydrocarbyl group, saturated hydrocarbyloxy group, saturated hydrocarbylcarbonyloxy group, saturated hydrocarbyloxyhydrocarbyl group, and saturated hydrocarbylthiohydrocarbyl group may be straight, branched or cyclic. When f3 is 2 or more, a plurality of groups R¹⁹ may be identical or different.

In formula (B5), A⁴ is a single bond or a C₁-C₁₀ saturated hydrocarbylene group in which some constituent -CH₂- may be replaced by -O-. The saturated hydrocarbylene group may be straight, branched or cyclic. Examples thereof are as exemplified above for A¹ in formula (B1).

When repeat units of at least one type selected from repeat units B3 to B5 are incorporated, better performance is obtained because not only the aromatic ring possesses etch resistance, but the cyclic structure incorporated into the main chain also exerts the effect of improving etch resistance and resistance to EB irradiation during pattern inspection step.

The content of repeat units B3 to B5 is preferably at least 5 mol% based on the overall repeat units of the polymer for obtaining the effect of improving etch resistance. Also, the content of repeat units B3 to B5 is preferably up to 25 mol%, more preferably up to 20 mol% based on the overall repeat units of the polymer. When the relevant units are free of functional groups or have a functional group other than hydroxy, their content of up to 25 mol% is preferred because the risk of forming development defects is eliminated. Each of the repeat units B3 to B5 may be of one type or a combination of plural types.

It is preferred that the polymer comprise repeat units B1, repeat units B2, and repeat units of at least one type selected from repeat units B3 to B5, because both etch resistance and high resolution are achievable. The total content of these repeat units is preferably at least 60 mol%, more preferably at least 70 mol%, even more preferably at least 80 mol%, most preferably at least 90 mol% based on the overall repeat units of the polymer.

In another preferred embodiment, the polymer further comprises repeat units of at least one type selected from repeat units having the formula (B6), repeat units having the formula (B7), repeat units having the formula (B8), repeat units having the formula (B9), repeat units having the formula (B10), repeat units having the formula (B11), repeat units having the formula (B12), and repeat units having the formula (B13), shown below. Notably these repeat units are also referred to as repeat units B6 to B13.

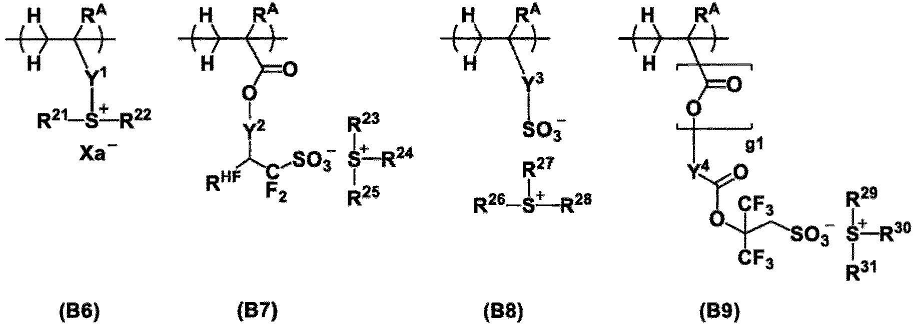

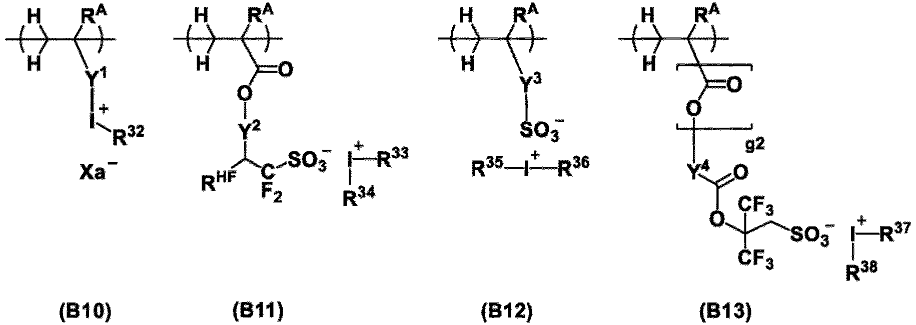

In formulae (B6) to (B13), R^{A} is each independently hydrogen, fluorine, methyl or trifluoromethyl. Y¹ is a single bond, a C₁-C₆ aliphatic hydrocarbylene group, phenylene group, naphthylene group or C₇-C₁₈ group obtained by combining the foregoing, *-O-Y¹¹-, *-C(=O)-O-Y¹¹-, or *-C(=O)-NH-Y¹¹-, wherein Y¹¹ is a C₁-C₆ aliphatic hydrocarbylene group, phenylene group, naphthylene group or C₇-C₁₈ group obtained by combining the foregoing, which may contain a carbonyl moiety, ester bond, ether bond or hydroxy moiety. Y² is a single bond or **-Y²¹-C(=O)-O-, wherein Y²¹ is a C₁-C₂₀ hydrocarbylene group which may contain a heteroatom. Y³ is a single bond, methylene, ethylene, phenylene, fluorinated phenylene, trifluoromethyl-substituted phenylene, *-O-Y³¹-, *-C(=O)-O-Y³¹-, or *-C(=O)-NH-Y³¹-, wherein Y³¹ is a C₁-C₆ aliphatic hydrocarbylene group, phenylene group, fluorinated phenylene group, trifluoromethyl-substituted phenylene group, or C₇-C₂₀ group obtained by combining the foregoing, which may contain a carbonyl moiety, ester bond, ether bond or hydroxy moiety. The asterisk (*) designates a point of attachment to the carbon atom in the backbone and ** designates a point of attachment to the oxygen atom in the formula. Y⁴ is a single bond or C₁-C₃₀ hydrocarbylene group which may contain a heteroatom, g1 and g2 are each independently 0 or 1, g1 and g2 are 0 when Y⁴ is a single bond.

In formulae (B6) and (B10), Xa⁻ is a non-nucleophilic counter ion. Examples of the non-nucleophilic counter ion Xa⁻ are as described in JP-A 2010-113209 and JP-A 2007-145797.

In formulae (B7) and (B11) wherein Y² is -Y²¹-C(=O)-O-, Y²¹ is a hydrocarbylene group which may contain a heteroatom. Illustrative, non-limiting examples of the hydrocarbylene group Y²¹ are given below.

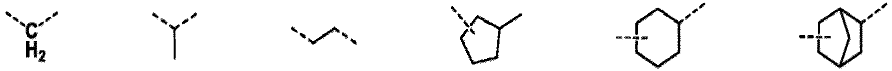

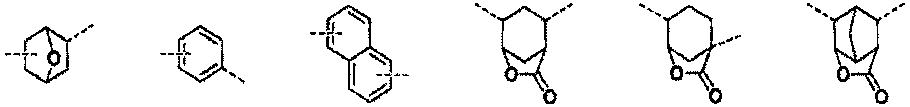

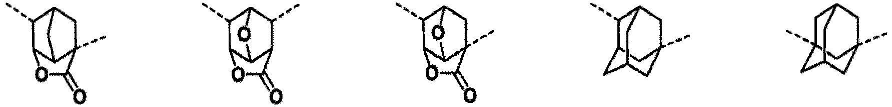

In formulae (B7) and (B11), R^{HF} is hydrogen or trifluoromethyl. Examples of the repeat units B7 and B11 wherein R^{HF} is hydrogen are as described in JP-A 2010-116550. Examples of the repeat units B7 and B11 wherein R^{HF} is trifluoromethyl are as described in JP-A 2010-077404. Examples of the repeat units B8 and B12 are as described in JP-A 2012-246265 and JP-A 2012-246426.

The C₁-C₃₀ hydrocarbylene group which may contain a heteroatom, represented by Y⁴, may be saturated or unsaturated and straight, branched or cyclic. Examples thereof include C₁-C₃₀ alkanediyl groups such as methylene, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl, decane-1,10-diyl, undecane-1,11-diyl, dodecane-1,12-diyl, tridecane-1,13-diyl, tetradecane-1,14-diyl, pentadecane-1,15-diyl, hexadecane-1,16-diyl, and heptadecane-1,17-diyl; C₃-C₃₀ cyclic saturated hydrocarbylene groups such as cyclopentanediyl, cyclohexanediyl, norbornanediyl and adamantanediyl; C₆-C₃₀ arylene groups such as phenylene, methylphenylene, ethylphenylene, n-propylphenylene, isopropylphenylene, n-butylphenylene, isobutylphenylene, sec-butylphenylene, tert-butylphenylene, naphthylene, methylnaphthylene, ethylnaphthylene, n-propylnaphthylene, isopropylnaphthylene, n-butylnaphthylene, isobutylnaphthylene, sec-butylnaphthylene, and tert-butylnaphthylene; and combinations thereof.

In these hydrocarbylene groups, some or all of the hydrogen atoms may be substituted by a moiety containing a heteroatom such as oxygen, sulfur, nitrogen or halogen, or some constituent -CH₂- may be replaced by a moiety containing a heteroatom such as oxygen, sulfur or nitrogen, so that the group may contain a hydroxy, cyano, fluorine, chlorine, bromine, iodine, carbonyl, ether bond, ester bond, sulfonic ester bond, carbonate bond, lactone ring, sultone ring, carboxylic anhydride (-C(=O)-O-C(=O)-) or haloalkyl moiety.

The repeat units B9 and B13 are capable of generating an acid having difluoromethyl at β-position of sulfonyl group upon exposure to high-energy radiation such as UV, deep UV, EB, EUV, X-ray, γ-ray, or synchrotron radiation. The generated acid has an acid strength suitable for inducing deprotection on a polymer comprising repeat units A2. When a polymer comprising repeat units B9 or B13 is used as the base polymer in the resist composition, the movement and diffusion of the generated acid can be adequately controlled.

A photoacid generator capable of generating an arenesulfonic acid upon exposure to high-energy radiation is commonly used for inducing deprotection to a polymer comprising repeat units protected with an acetal, tertiary alkyl or tert-butoxycarbonyl group. However, when arenesulfonic acid-generating units are incorporated into a polymer with the intention of exerting the desired effects, the polymer is sometimes not dissolvable in a solvent because of its low solvent solubility. Since a polymer having repeat units B9 or B13 is fully lipophilic, its preparation and handling are easy and a resist composition is readily prepared therefrom.

Preferred examples of the anion in the monomer from which repeat units B9 or B13 are derived are shown below, but not limited thereto.

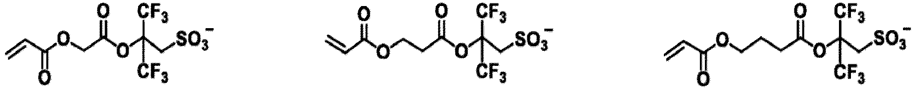

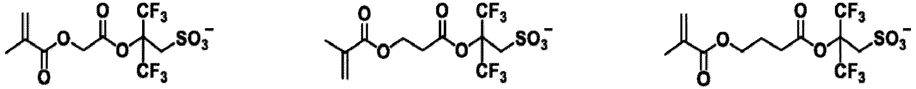

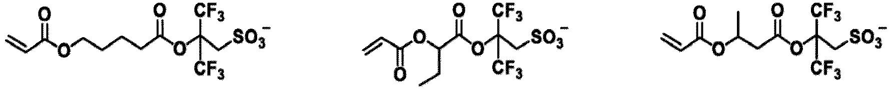

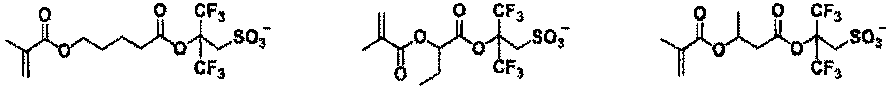

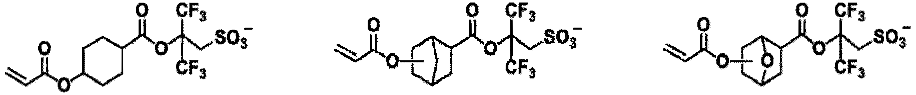

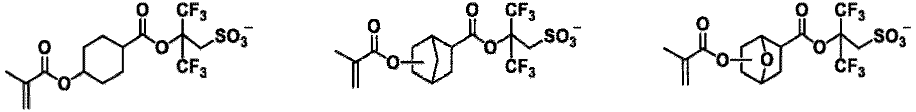

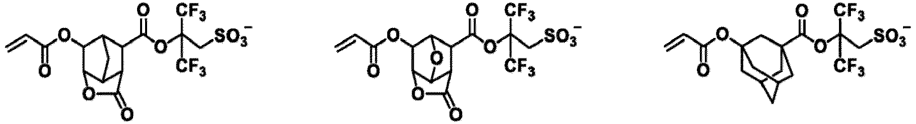

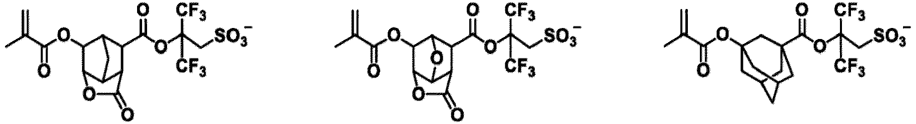

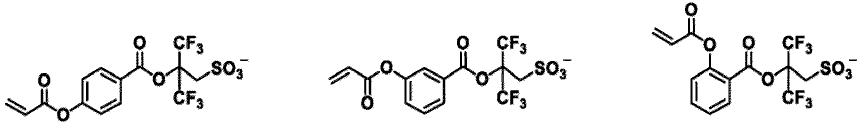

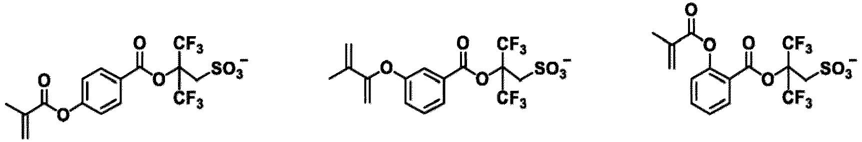

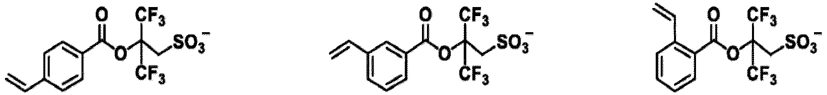

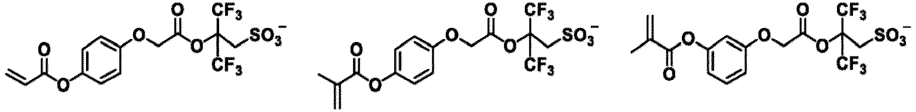

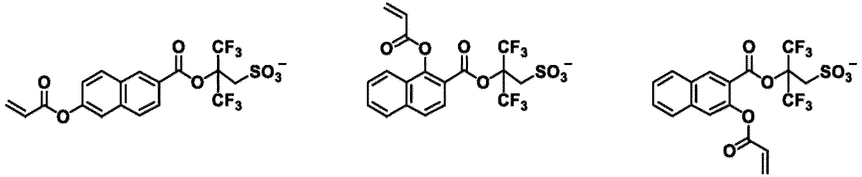

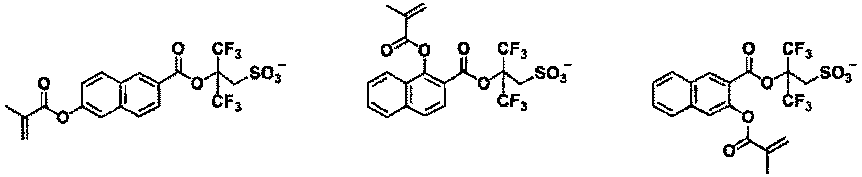

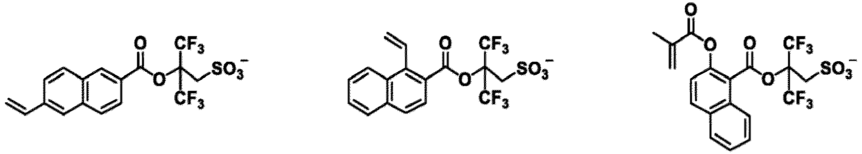

In formulae (B6) to (B13), R²¹ to R³⁸ are each independently halogen or a C₁-C₂₀ hydrocarbyl group which may contain a heteroatom.

Suitable halogen atoms include fluorine, chlorine, bromine and iodine.

The C₁-C₂₀ hydrocarbyl group may be saturated or unsaturated and straight, branched or cyclic. Examples thereof include C₁-C₂₀ alkyl groups such as methyl, ethyl, n-propyl, isopropyl, n-butyl, isobutyl, sec-butyl, tert-butyl, n-pentyl, tert-pentyl, n-hexyl, n-octyl, 2-ethylhexyl, n-nonyl, and n-decyl; C₃-C₂₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl and adamantylmethyl; and C₆-C₂₀ aryl groups such as phenyl, naphthyl, and anthracenyl. In the foregoing hydrocarbyl groups, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy, fluorine, chlorine, bromine, iodine, cyano, nitro, carbonyl, ether bond, ester bond, sulfonic ester bond, carbonate bond, lactone ring, sultone ring, carboxylic anhydride (-C(=O)-O-C(=O)-) or haloalkyl moiety.

Also, R²¹ and R²² may bond together to form a ring with the sulfur atom to which they are attached. R²³ and R²⁴, R²⁶ and R²⁷, or R²⁹ and R³⁰ may bond together to form a ring with the sulfur atom to which they are attached. Examples of the ring thus formed are as exemplified above for the ring that R^{ct1} and R^{ct2} in formula (cation-1), taken together, form with the sulfur atom to which they are attached.

Exemplary structures of the sulfonium cation in formulae (B7) to (B9) are as exemplified above for the sulfonium cation having formula (cation-1), but not limited thereto. Exemplary structures of the iodonium cation in formulae (B11) to (B13) are as exemplified above for the iodonium cation having formula (cation-2), but not limited thereto.

Of repeat units B6 to B13, repeat units B9 are preferred for the processing of photomask blanks because an optimum acid strength is available from the design of an acid labile group on the polymer.

The repeat units B6 to B13 are capable of generating an acid upon exposure to high-energy radiation. It is believed that binding of the relevant units to a polymer enables to appropriately control acid diffusion and to form a pattern with reduced LER. Since the acid-generating unit is bound to a polymer, the phenomenon that acid volatilizes from the exposed region and re-deposits on the unexposed region during bake in vacuum is suppressed. This is effective for reducing LER and for suppressing profile degradation due to unwanted film thickness loss in the unexposed region.

When repeat units B6 to B13 are included, their content is preferably 0.1 to 30 mol%, more preferably 0.5 to 20 mol% based on the overall repeat units of the polymer. Each of repeat units B6 to B13 may be of one type or a combination of plural types.

The content of repeat units having an aromatic ring structure is preferably at least 65 mol%, more preferably at least 75 mol%, even more preferably at least 85 mol% based on the overall repeat units of the polymer. When the polymer does not contain repeat units B6 to B13, it is preferred that all units have an aromatic ring structure.

The polymer may further comprise (meth)acrylate units protected with an acid labile group or (meth)acrylate units having an adhesive group such as lactone structure or hydroxy group other than phenolic hydroxy as commonly used in the art. These repeat units are effective for fine adjustment of properties of a resist film, but not essential.

Examples of the (meth)acrylate unit having an adhesive group include repeat units having the following formulae (B14) to (B16), which are also referred to as repeat units B14 to B16. While these units do not exhibit acidity, they may be used as auxiliary units for providing adhesion to substrates or adjusting solubility.

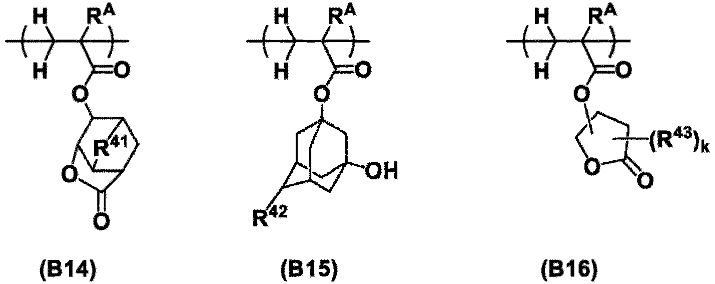

In formulae (B14) to (B16), R^{A} is each independently hydrogen, fluorine, methyl or trifluoromethyl. R⁴¹ is -O- or methylene. R⁴² is hydrogen or hydroxy. R⁴³ is a C₁-C₄ saturated hydrocarbyl group, and k is an integer of 0 to 3.

When repeat units B14 to B16 are included, their content is preferably 0 to 20 mol%, more preferably 0 to 10 mol% based on the overall repeat units of the polymer. Each of repeat units B14 to B16 may be of one type or a combination of plural types.

The polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630, for example.

The polymer should preferably have a Mw of 1,000 to 50,000, and more preferably 2,000 to 20,000. A Mw of at least 1,000 eliminates the risk that pattern features are rounded at their top, inviting degradations of resolution and LER. A Mw of up to 50,000 eliminates the risk that LER is degraded when a pattern with a line width of up to 100 nm is formed. As used herein, Mw is measured by GPC versus polystyrene standards using tetrahydrofuran (THF) or dimethylformamide (DMF) solvent.

The polymer preferably has a narrow molecular weight distribution or dispersity (Mw/Mn) of 1.0 to 2.0, more preferably 1.0 to 1.9. A polymer with such a narrow dispersity eliminates the risk that foreign particles are left on the pattern after development and the pattern profile is aggravated.

The base polymer is designed such that the dissolution rate in alkaline developer is preferably up to 10 nm/min, more preferably up to 7 nm/min, even more preferably up to 5 nm/min. In the advanced generation of lithography wherein the coating film on the substrate is in a thin film range of up to 100 nm, the influence of pattern film thickness loss during alkaline development becomes strong. When the polymer has an alkaline dissolution rate of greater than 10 nm/min, pattern collapse occurs, i.e., a small-size pattern cannot be formed. The problem becomes outstanding in the fabrication of photomasks requiring to be defectless and having a tendency of strong development process. It is noted that the dissolution rate of a base polymer in alkaline developer is computed by spin coating a 16.7 wt% solution of a polymer in propylene glycol monomethyl ether acetate (PGMEA) solvent onto a 8-inch silicon wafer, baking at 100°C for 90 seconds to form a film of 1,000 nm thick, developing the film in a 2.38 wt% aqueous solution of tetramethylammonium hydroxide (TMAH) at 23°C for 100 seconds, and measuring a loss of film thickness.

In addition to the polymer defined above, the base polymer (B) may contain another polymer. The other polymer may be any of prior art well-known base polymers used in resist compositions. The content of the other polymer is not particularly limited as long as the benefits of the invention are not impaired.

### (C) Organic solvent

The chemically amplified positive resist composition may comprise an organic solvent as component (C). The organic solvent used herein is not particularly limited as long as the components are soluble therein. Examples of the organic solvent are described in JP-A 2008-111103, paragraphs [0144] to [0145] (USP 7,537,880). Specifically, exemplary solvents include ketones such as cyclohexanone and methyl-2-n-pentyl ketone; alcohols such as 3-methoxybutanol, 3-methyl-3-methoxybutanol, 1-methoxy-2-propanol, 1-ethoxy-2-propanol, and diacetone alcohol; ethers such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate (PGMEA), propylene glycol monoethyl ether acetate, ethyl lactate (EL), ethyl pyruvate, butyl acetate, methyl 3-methoxypropionate, ethyl 3-ethoxypropionate, tert-butyl acetate, tert-butyl propionate, and propylene glycol mono-tert-butyl ether acetate; and lactones such as γ-butyrolactone (GBL), and mixtures thereof. Where an acid labile group of acetal form is used, a high boiling alcohol solvent such as diethylene glycol, propylene glycol, glycerol, 1,4-butanediol or 1,3-butanediol may be added to accelerate deprotection reaction of acetal.

Of the above organic solvents, it is recommended to use 1-ethoxy-2-propanol, PGMEA, PGME, cyclohexanone, EL, GBL, and mixtures thereof.

In the resist composition, the organic solvent (C) is preferably used in an amount of 200 to 10,000 parts, more preferably 400 to 5,000 parts by weight per 80 parts by weight of the base polymer (B). The organic solvent may be used alone or in admixture.

### (D) Fluorinated polymer

The chemically amplified positive resist composition may further comprise a fluorinated polymer for the purposes of enhancing contrast, preventing chemical flare of acid upon exposure to high-energy radiation, preventing mixing of acid from an anti-charging film in the step of coating an anti-charging film-forming material on a resist film, and suppressing unexpected unnecessary pattern degradation. The fluorinated polymer contains repeat units of at least one type selected from repeat units having the formula (D1), repeat units having the formula (D2), repeat units having the formula (D3), and repeat units having the formula (D4), and may contain repeat units of at least one type selected from repeat units having the formula (D5) and repeat units having the formula (D6). It is noted that repeat units having formulae (D1), (D2), (D3), (D4), (D5), and (D6) are also referred to as repeat units D1, D2, D3, D4, D5, and D6, respectively, hereinafter. Since the fluorinated polymer also has a surface active function, it can prevent insoluble residues from re-depositing onto the substrate during the development step and is thus effective for preventing development defects.

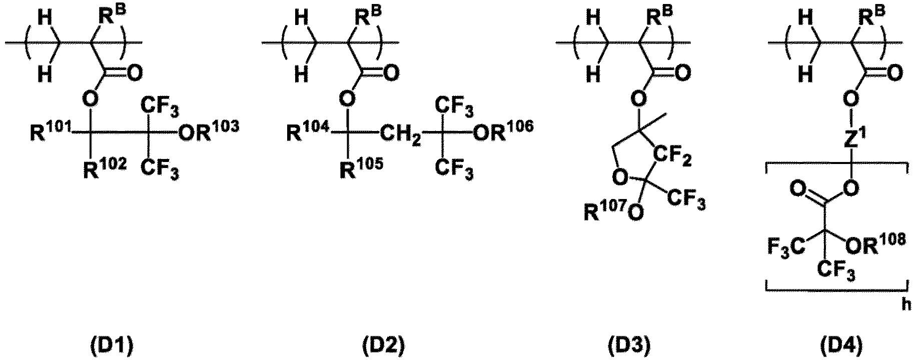

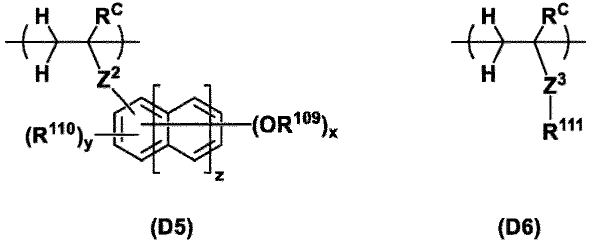

In formulae (D1) to (D6), x is an integer of 1 to 3, y is an integer satisfying: 0 ≤ y < 5+2z-x, z is 0 or 1, and h is an integer of 1 to 3. R^{B} is each independently hydrogen, fluorine, methyl or trifluoromethyl. R^{C} is each independently hydrogen or methyl. R¹⁰¹, R¹⁰², R¹⁰⁴ and R¹⁰⁵ are each independently hydrogen or a C₁-C₁₀ saturated hydrocarbyl group. R¹⁰³, R¹⁰⁶, R¹⁰⁷ and R¹⁰⁸ are each independently hydrogen, a C₁-C₁₅ hydrocarbyl group or fluorinated hydrocarbyl group, or an acid labile group, with the proviso that an ether bond or carbonyl moiety may intervene in a carbon-carbon bond in the hydrocarbyl groups or fluorinated hydrocarbyl groups represented by R¹⁰³, R¹⁰⁶, R¹⁰⁷ and R¹⁰⁸. R¹⁰⁹ is hydrogen or a C₁-C₅ straight or branched hydrocarbyl group in which a heteroatom-containing moiety may intervene in a carbon-carbon bond. R¹¹⁰ is a C₁-C₅ straight or branched hydrocarbyl group in which a heteroatom-containing moiety may intervene in a carbon-carbon bond. R¹¹¹ is a C₁-C₂₀ saturated hydrocarbyl group in which at least one hydrogen is substituted by fluorine and some constituent -CH₂- may be replaced by an ester bond or ether bond. Z¹ is a C₁-C₂₀ (h+1)-valent hydrocarbon group or C₁-C₂₀ (h+1)-valent fluorinated hydrocarbon group. Z² is a single bond, *-C(=O)-O- or *-C(=O)-NH- wherein the asterisk (*) designates a point of attachment to the carbon atom in the backbone. Z³ is a single bond, -O-, *-C(=O)-O-Z³¹-Z³²- or *-C(=O)-NH-Z³¹-Z³²-, wherein Z³¹ is a single bond or a C₁-C₁₀ saturated hydrocarbylene group, Z³² is a single bond, ester bond, ether bond or sulfonamide bond, and the asterisk (*) designates a point of attachment to the carbon atom in the backbone.

In formulae (D1) and (D2), the C₁-C₁₀ saturated hydrocarbyl group represented by R¹⁰¹, R¹⁰² , R¹⁰⁴ and R¹⁰⁵ may be straight, branched or cyclic and examples thereof include C₁-C₁₀ alkyl groups such as methyl, ethyl, n-propyl, isopropyl, n-butyl, isobutyl, sec-butyl, tert-butyl, n-pentyl, n-hexyl, n-heptyl, n-octyl, n-nonyl, and n-decyl, and C₃-C₁₀ cyclic saturated hydrocarbyl groups such as cyclopropyl, cyclobutyl, cyclopentyl, cyclohexyl, adamantyl, and norbornyl. Inter alia, C₁-C₆ saturated hydrocarbyl groups are preferred.

In formulae (D1) to (D4), the C₁-C₁₅ hydrocarbyl group represented by R¹⁰³, R¹⁰⁶, R¹⁰⁷ and R¹⁰⁸ may be straight, branched or cyclic and examples thereof include C₁-C₁₅ alkyl, C₂-C₁₅ alkenyl and C₂-C₁₅ alkynyl groups, with the alkyl groups being preferred. Suitable alkyl groups include methyl, ethyl, n-propyl, isopropyl, n-butyl, isobutyl, sec-butyl, n-pentyl, n-hexyl, n-heptyl, n-octyl, n-nonyl, n-decyl, n-undecyl, n-dodecyl, n-tridecyl, n-tetradecyl and n-pentadecyl. The fluorinated hydrocarbyl groups correspond to the foregoing hydrocarbyl groups in which some or all carbon-bonded hydrogen atoms are substituted by fluorine atoms.

In formula (D4), examples of the C₁-C₂₀ (h+1)-valent hydrocarbon group Z¹ include the foregoing C₁-C₂₀ alkyl groups and C₃-C₂₀ cyclic saturated hydrocarbyl groups, with h number of hydrogen atoms being eliminated. Examples of the C₁-C₂₀ (h+1)-valent fluorinated hydrocarbon group Z¹ include the foregoing (h+1)-valent hydrocarbon groups in which at least one hydrogen atom is substituted by fluorine.

Examples of the repeat units D1 to D4 are given below, but not limited thereto. Herein R^{B} is as defined above.

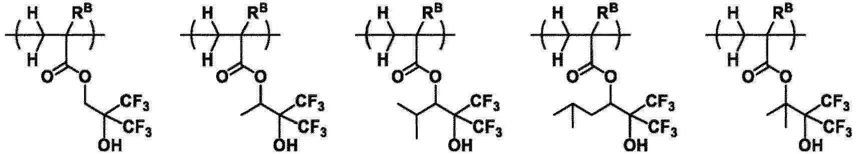

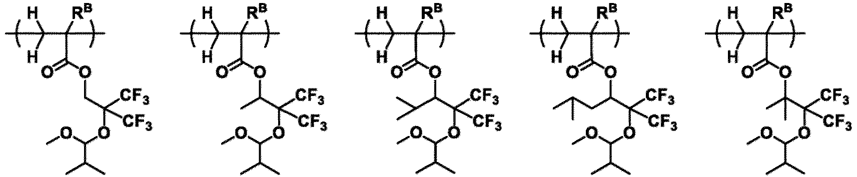

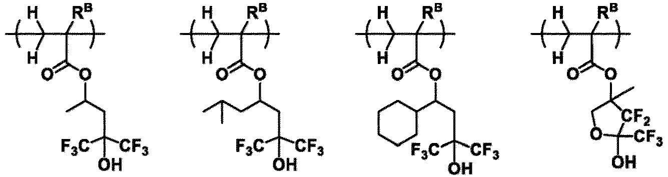

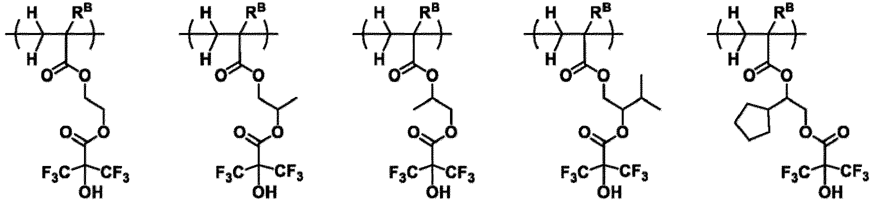

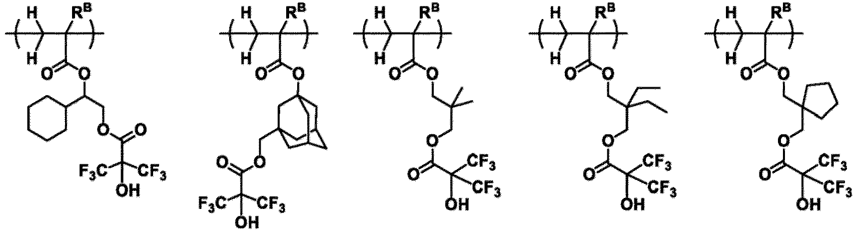

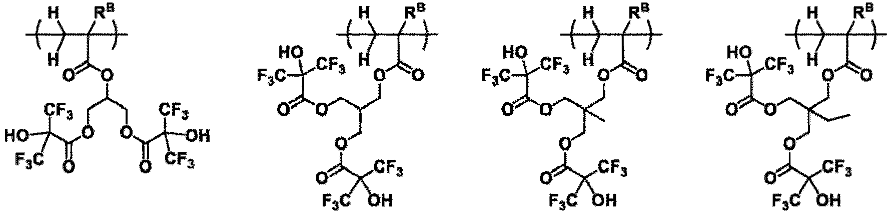

In formula (D5), examples of the C₁-C₅ hydrocarbyl groups R¹⁰⁹ and R¹¹⁰ include alkyl, alkenyl and alkynyl groups, with the alkyl groups being preferred. Suitable alkyl groups include methyl, ethyl, n-propyl, isopropyl, n-butyl, isobutyl, sec-butyl, and n-pentyl. In these groups, a moiety containing a heteroatom such as oxygen, sulfur or nitrogen may intervene in a carbon-carbon bond.

In formula (D5), -OR¹⁰⁹ is preferably a hydrophilic group. In this case, R¹⁰⁹ is preferably hydrogen or a C₁-C₅ alkyl group in which oxygen intervenes in a carbon-carbon bond.

In formula (D5), Z² is preferably *-C(=O)-O- or *-C(=O)-NH-. Also preferably R^{C} is methyl. The inclusion of carbonyl in Z² enhances the ability to trap the acid originating from the anti-charging film. A polymer wherein R^{C} is methyl is a robust polymer having a high glass transition temperature (Tg) which is effective for suppressing acid diffusion. As a result, the resist film is improved in stability with time, and neither resolution nor pattern profile is degraded.

Examples of the repeat unit D5 are given below, but not limited thereto. Herein R^{C} is as defined above.

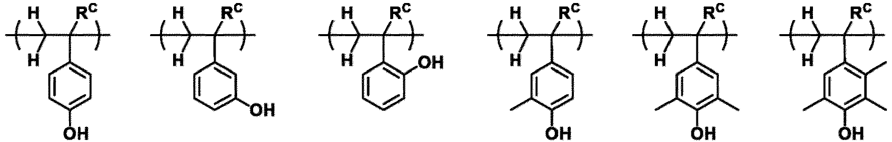

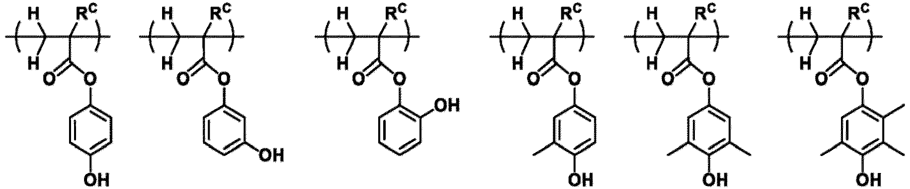

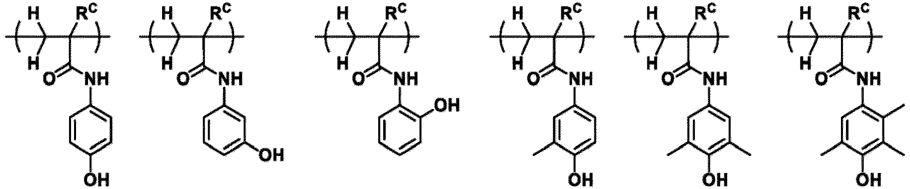

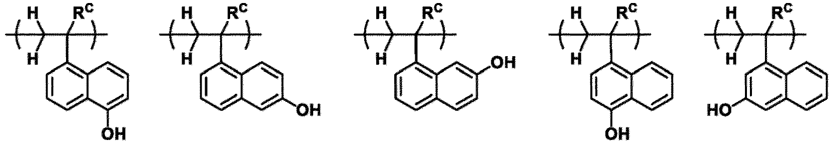

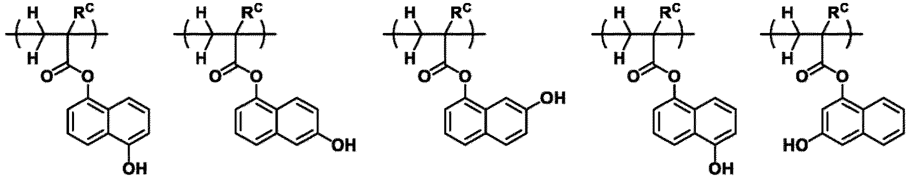

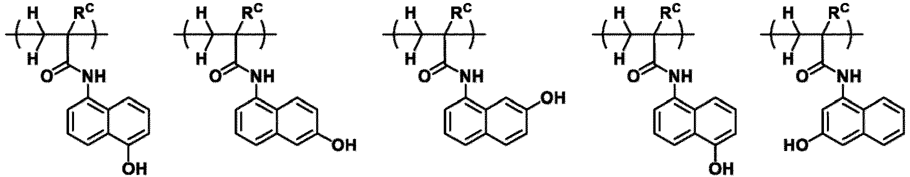

In formula (D6), the C₁-C₁₀ saturated hydrocarbylene group Z³ may be straight, branched or cyclic and examples thereof include methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, and 1,1-dimethylethane-1,2-diyl.

The C₁-C₂₀ saturated hydrocarbyl group having at least one hydrogen substituted by fluorine, represented by R¹¹¹, may be straight, branched or cyclic and examples thereof include C₁-C₂₀ alkyl groups and C₃-C₂₀ cyclic saturated hydrocarbyl groups in which at least one hydrogen is substituted by fluorine.

Examples of the repeat unit D6 are given below, but not limited thereto. Herein R^{C} is as defined above.

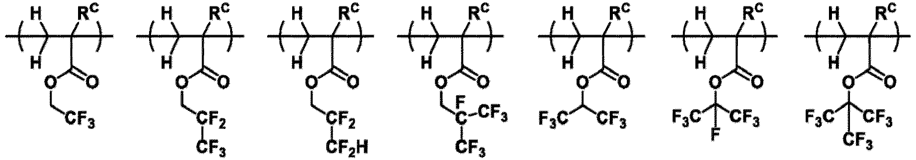

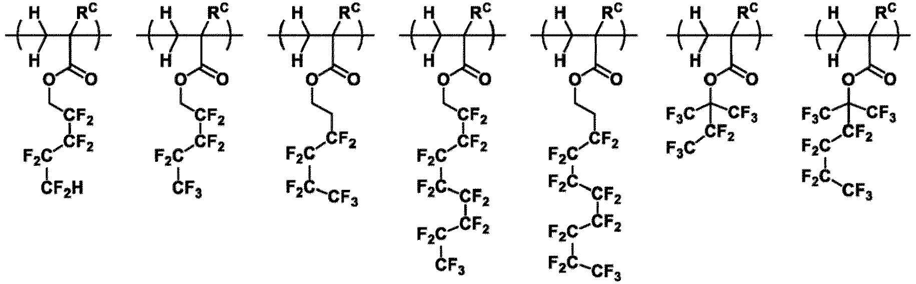

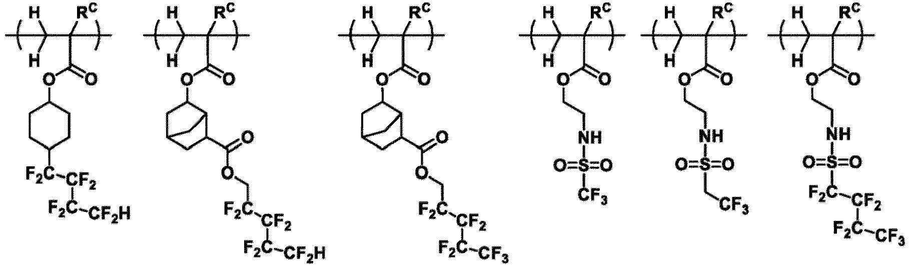

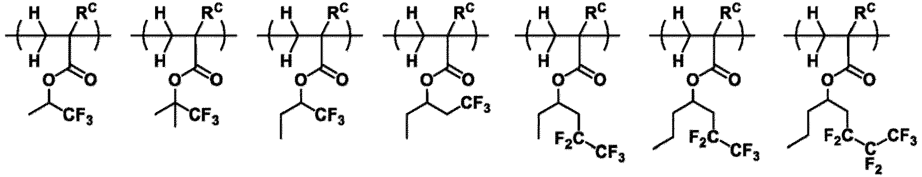

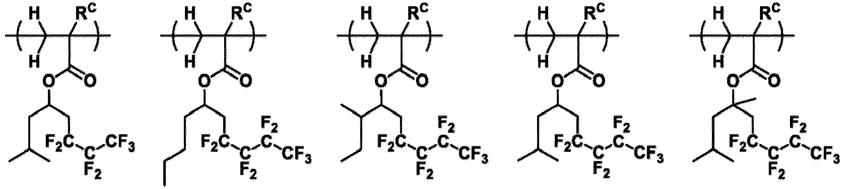

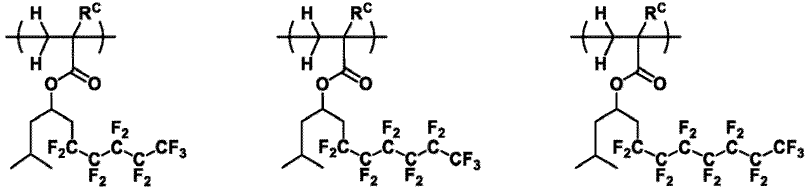

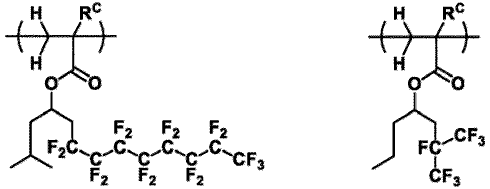

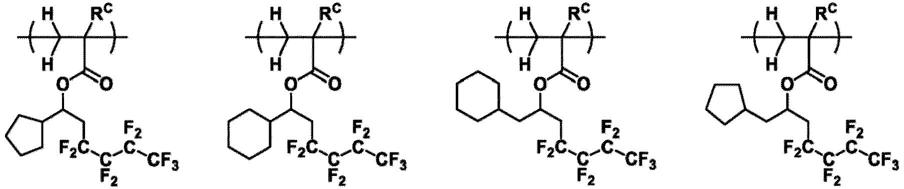

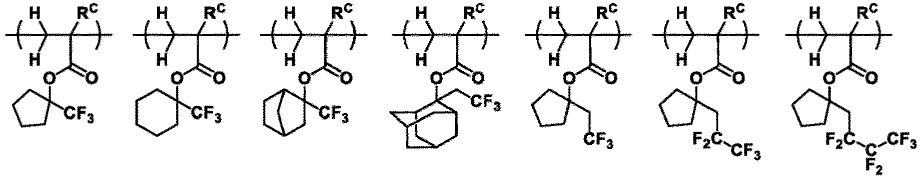

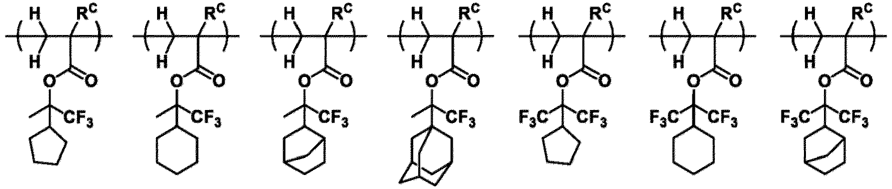

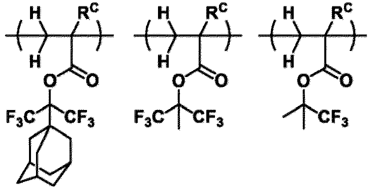

The content of repeat units D1 to D4 is preferably 15 to 95 mol%, more preferably 20 to 85 mol% based on the overall repeat units of the fluorinated polymer. The content of repeat unit D5 and/or D6 is preferably 5 to 85 mol%, more preferably 15 to 80 mol% based on the overall repeat units of the fluorinated polymer. Each of repeat units D1 to D6 may be used alone or in admixture.

The fluorinated polymer may comprise additional repeat units as well as the repeat units D1 to D6. Suitable additional repeat units include those described in USP 9,091,918 (JP-A 2014-177407, paragraphs [0046]-[0078]). When the fluorinated polymer comprises additional repeat units, their content is preferably up to 50 mol% based on the overall repeat units.

The fluorinated polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630.

The fluorinated polymer should preferably have a Mw of 2,000 to 50,000, and more preferably 3,000 to 20,000. A fluorinated polymer with a Mw of less than 2,000 helps acid diffusion, degrading resolution and detracting from age stability. A polymer with too high Mw has a reduced solubility in solvent, with a risk of leaving coating defects. The fluorinated polymer preferably has a dispersity (Mw/Mn) of 1.0 to 2.2, more preferably 1.0 to 1.7.

In the resist composition, the fluorinated polymer (D) is preferably used in an amount of 0.01 to 30 parts by weight, more preferably 0.1 to 20 parts by weight per 80 parts by weight of the base polymer (B). The fluorinated polymer may be used alone or in admixture.

### (E) Quencher

The resist composition may further contain a quencher as component (E). The quencher is a compound having a function of trapping the acid generated by the acid generator. The quencher is effective for holding down the rate of diffusion of the acid (generated by the acid generator) in the resist film. Even when a substrate whose outermost surface is made of a chromium-containing material is used, the quencher is effective for suppressing the influence of the acid (generated in the resist film) on the chromium-containing material.

One preferred example of the quencher is an onium salt of carboxylic acid having the formula (E1).

R²⁰¹-CO₂⁻ Mq⁺ (E1)

In formula (E1), R²⁰¹ is a C₁-C₄₀ hydrocarbyl group which may contain a heteroatom. The hydrocarbyl group may be saturated or unsaturated and straight, branched or cyclic. Examples include C₁-C₄₀ alkyl groups, C₃-C₄₀ cyclic saturated hydrocarbyl groups, C₂-C₄₀ alkenyl groups, C₂-C₄₀ alkynyl groups, C₃-C₄₀ cyclic unsaturated aliphatic hydrocarbyl groups, C₆-C₄₀ aryl groups, C₇-C₄₀ aralkyl groups, and combinations thereof. In the hydrocarbyl group, some or all hydrogen may be substituted by a hydroxy, carboxy, halogen, cyano, amide, nitro, mercapto, sultone, sulfone or sulfonium salt-containing moiety, and some constituent -CH₂- may be replaced by an ether bond, ester bond, carbonyl moiety, carbonate moiety or sulfonic ester bond.

In formula (E1), Mq⁺ is an onium cation. Suitable onium cations include sulfonium, iodonium and ammonium cations, with the sulfonium cations being preferred. Preferred sulfonium cations are as exemplified above for the sulfonium cation having formula (cation-1).

When the resist composition contains the carboxylic onium salt having formula (E1) as the quencher, its content is preferably 0.1 to 40 parts by weight, more preferably 0.1 to 20 parts by weight per 80 parts by weight of the base polymer (B).

Another preferred example of the quencher is an onium salt of carboxylic acid having the formula (E2) or (E3).

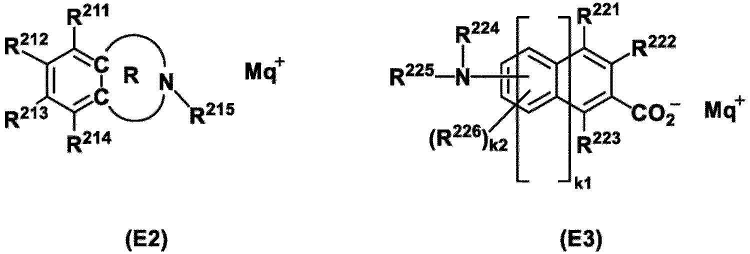

Herein, Mq⁺ is as defined above.

In formula (E2), R²¹¹ to R²¹⁴ are each independently hydrogen, -L^{A}-CO₂⁻, or a C₁-C₂₀ hydrocarbyl group which may contain a heteroatom. A pair of R²¹¹ and R²¹², R²¹² and R²¹³, or R²¹³ and R²¹⁴ may bond together to form a ring with the carbon atoms to which they are attached. L^{A} is a single bond or a C₁-C₂₀ hydrocarbylene group which may contain a heteroatom. R¹¹⁵ is hydrogen or a C₁-C₂₀ hydrocarbyl group which may contain a heteroatom.

In formula (E2), the ring R is a ring of 2 to 6 carbon atoms including the carbon and nitrogen atoms depicted in the formula, in which some or all of the carbon-bonded hydrogen atoms may be substituted by a C₁-C₂₀ hydrocarbyl moiety or -L^{A}-CO₂⁻, and some constituent - CH₂- may be replaced by a divalent moiety containing sulfur, oxygen or nitrogen. The ring may be alicyclic or aromatic and is preferably 5- or 6-membered. Examples include pyridine, pyrrole, pyrrolidine, piperidine, pyrazole, imidazoline, pyridazine, pyrimidine, pyrazine, imidazoline, oxazole, thiazole, morpholine, thiazine, and triazole rings.

The carboxylic onium salt having formula (E2) has at least one -L^{A}-CO₂⁻ group. That is, at least one of R²¹¹ to R²¹⁴ is -L^{A}-CO₂⁻ and/or at least one of carbon-bonded hydrogen atoms in the ring R is substituted by -L^{A}-CO₂⁻.

In formula (E3), R²²¹ to R²²⁶ are each independently hydrogen or a C₁-C₂₀ hydrocarbyl group which may contain a heteroatom. A pair of R²²¹ and R²²², or R²²³ and R²²⁶ may bond together to form a ring with the carbon atoms to which they are attached, and a pair of R²²⁴ and R²²⁵ may bond together to form a ring with the nitrogen atom to which they are attached. The subscript k1 is 0 or 1, and k2 is 0 or 1 in case of k1=0 and k2 is an integer of 0 to 3 in case of k1=1.

When the resist composition contains the carboxylic onium salt having formula (E2) or (E3) as the quencher, its content is preferably 0.1 to 50 parts by weight, more preferably 0.5 to 30 parts by weight per 80 parts by weight of the base polymer (B).

A further preferred example of the quencher is a sulfonium compound having the formula (E4).

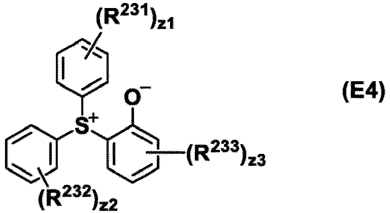

In formula (E4), R²³¹, R²³² and R²³³ are each independently a C₁-C₂₀ hydrocarbyl group which may contain a heteroatom. In the hydrocarbyl group, some hydrogen may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, cyano moiety, carbonyl moiety, ether bond, thioether bond, ester bond, sulfonic ester bond, carbonate bond, carbamate bond, lactone ring, sultone ring, carboxylic anhydride (-C(=O)-O-C(=O)-) or haloalkyl moiety. The constituent -CH₂- in the hydrocarbyl group may be a carbon atom bonded to the benzene ring.

In formula (E4), z1 and z2 are each independently an integer of 0 to 5, and z3 is an integer of 0 to 4. From the standpoints of ease of synthesis and availability of reactants, z1, z2 and z3 each are preferably 0, 1 or 2.

When z1 is 2 to 5, two adjoining R²³¹ may bond together to form a ring with the carbon atoms to which they are attached. When z2 is 2 to 5, two adjoining R²³² may bond together to form a ring with the carbon atoms to which they are attached. When z3 is 2 to 4, two adjoining R²³³ may bond together to form a ring with the carbon atoms to which they are attached.

When the resist composition contains the sulfonium compound having formula (E4) as the quencher, its content is preferably 0.1 to 40 parts by weight, more preferably 1 to 20 parts by weight per 80 parts by weight of the base polymer (B).

In combination with the above-mentioned onium salt compound, a photo-decomposable onium salt having a nitrogen-containing substituent group may be used as the quencher, if desired. This compound functions as a quencher in the unexposed region, but as a so-called photo-degradable base in the exposed region because it loses the quencher function in the exposed region due to neutralization thereof with the acid generated by itself. Using a photo-degradable base, the contrast between exposed and unexposed regions can be further enhanced. With respect to the photo-degradable base, reference may be made to JP-A 2009-109595, 2012-046501 and JP-A 2013-209360, for example. When the resist composition contains the photo-degradable base as the quencher, its content is preferably 0.1 to 40 parts by weight, more preferably 0.1 to 20 parts by weight per 80 parts by weight of the base polymer (B).

An amine compound may also be used as the quencher. Suitable amine compounds include primary, secondary and tertiary amine compounds as described in JP-A 2008-111103, paragraphs [0146]-[0164] (USP 7,537,880), especially amine compounds having a hydroxy group, ether bond, ester bond, lactone ring, cyano group or sulfonic ester bond. Also useful are compounds having primary or secondary amine protected with a carbamate group, as described in JP 3790649. When the resist composition contains the amine compound as the quencher, its content is preferably 0.001 to 12 parts by weight, more preferably 0.01 to 8 parts by weight per 80 parts by weight of the base polymer (B).

### (F) Other photoacid generator

In addition to the PAG in the form of the onium salt having formula (A), the chemically amplified positive resist composition may further comprise another photoacid generator (PAG). The other PAG used herein may be any compound capable of generating an acid upon exposure to high-energy radiation. Suitable PAGs include sulfonium salts, iodonium salts, sulfonyldiazomethane, N-sulfonyloxyimide, and oxime-O-sulfonate acid generators.

Suitable other PAGs include nonafluorobutane sulfonate, partially fluorinated sulfonates described in JP-A 2012-189977, paragraphs [0247]-[0251], partially fluorinated sulfonates described in JP-A 2013-101271, paragraphs [0261]-[0265], and those described in JP-A 2008-111103, paragraphs [0122]-[0142] and JP-A 2010-215608, paragraphs [0080]-[0081]. Among others, arylsulfonate and alkanesulfonate type PAGs are preferred because they generate acids having an appropriate strength to deprotect the acid labile group in repeat unit B2.

The preferred PAGs are salt compounds having a sulfonium anion of the structure shown below.

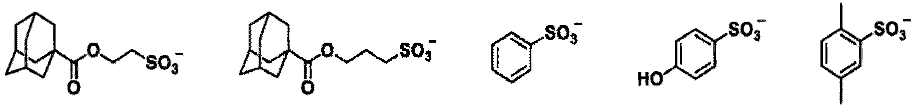

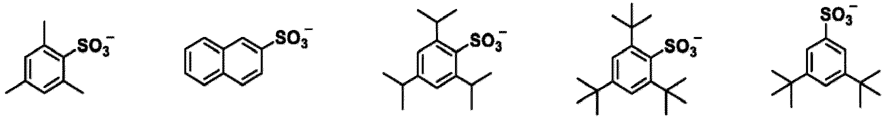

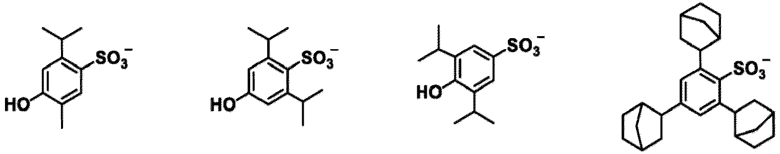

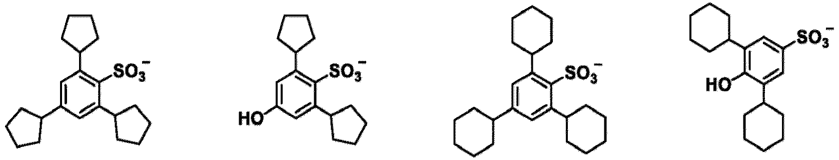

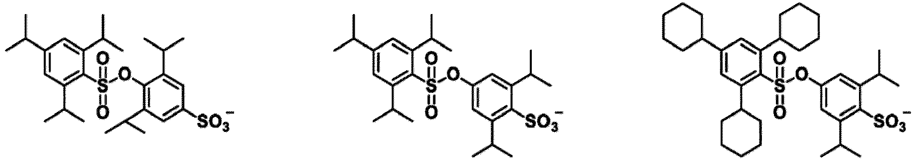

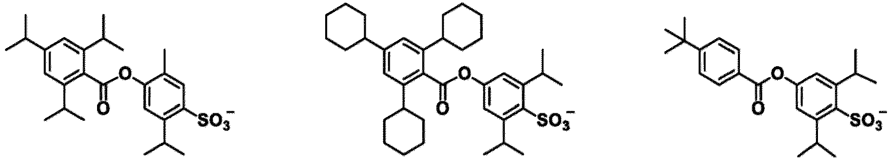

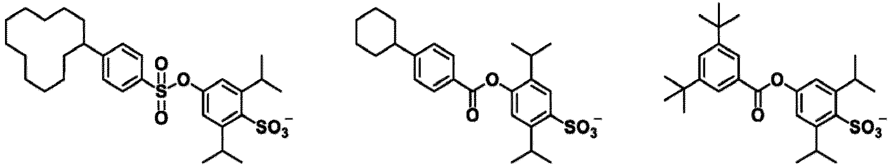

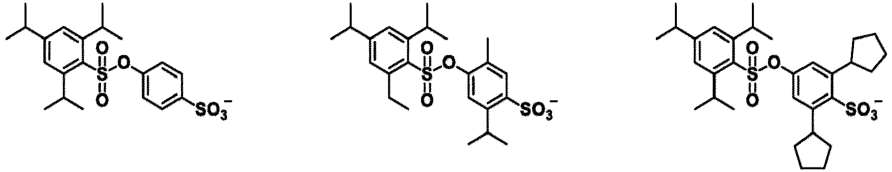

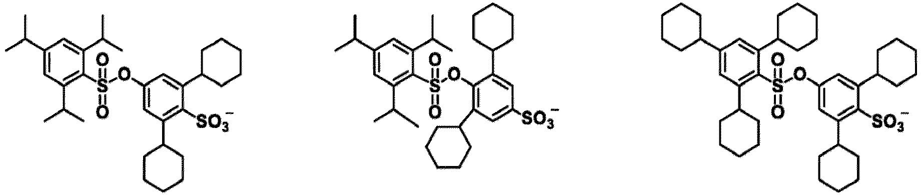

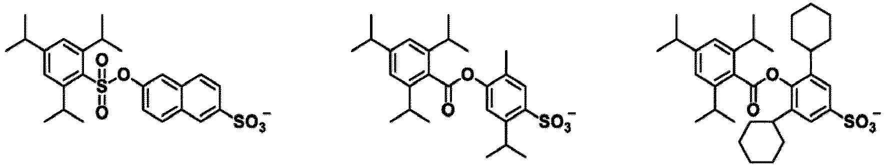

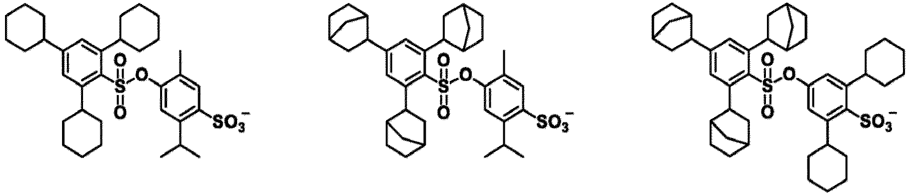

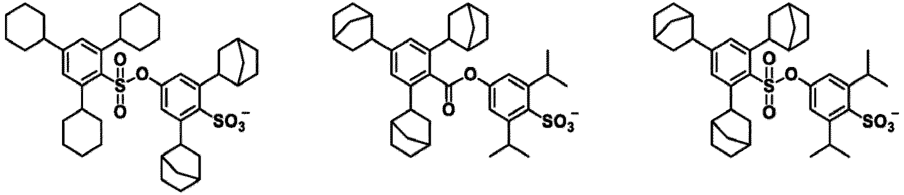

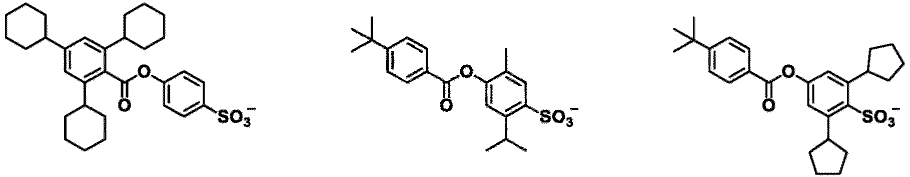

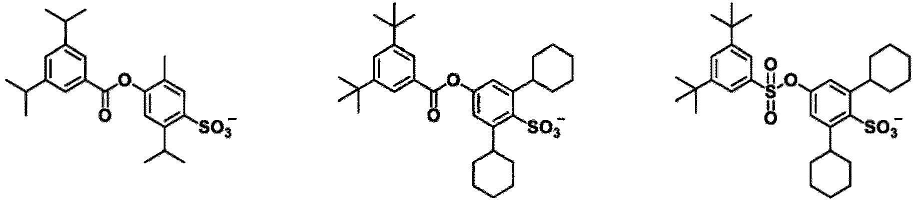

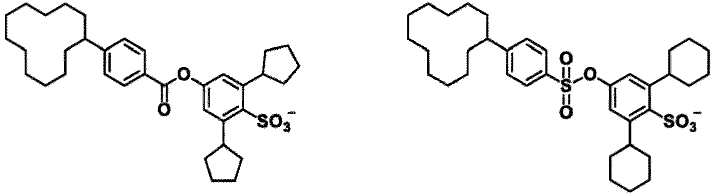

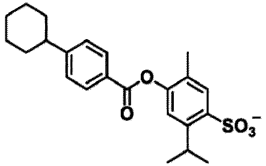

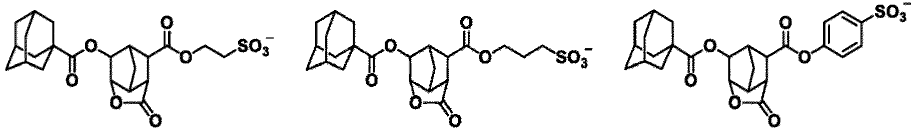

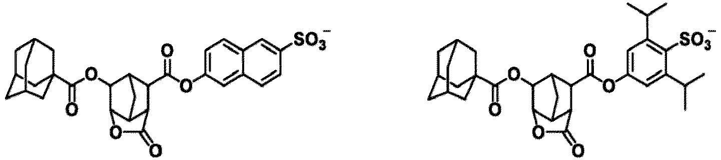

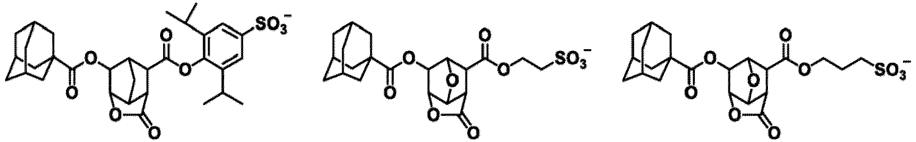

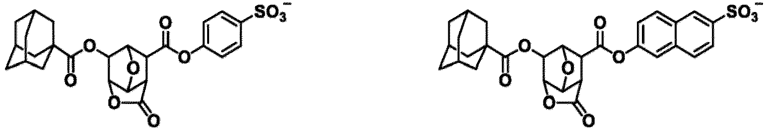

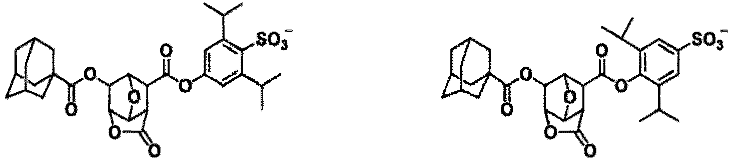

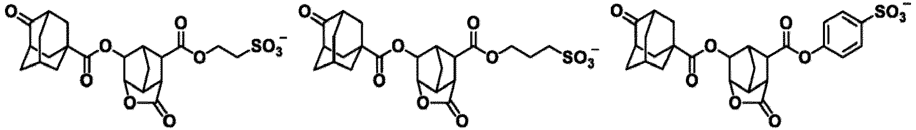

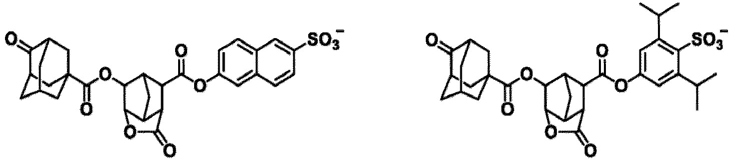

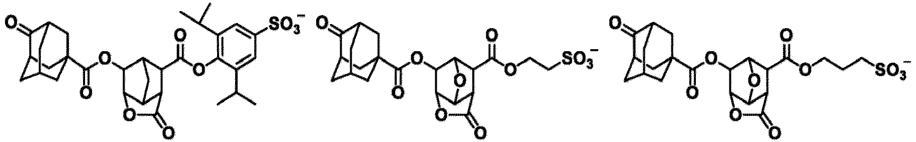

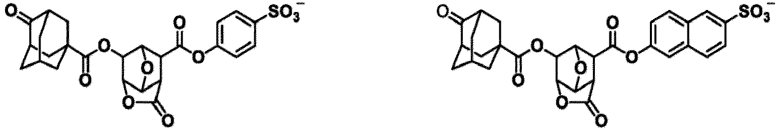

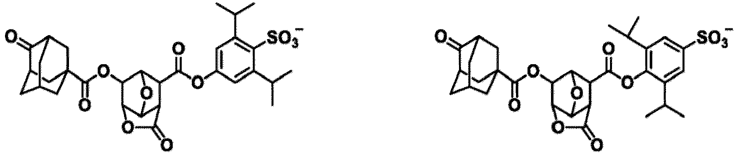

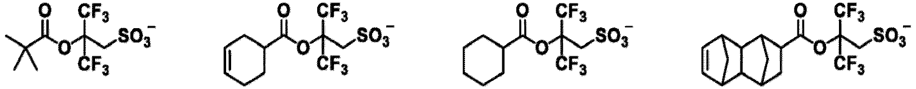

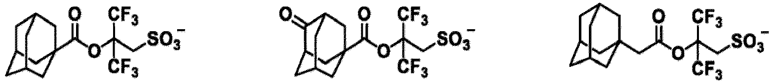

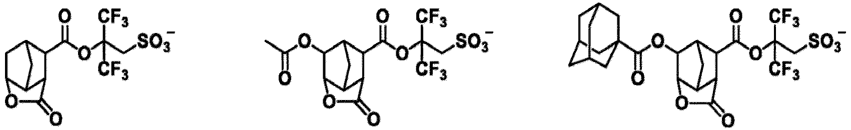

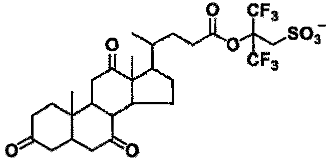

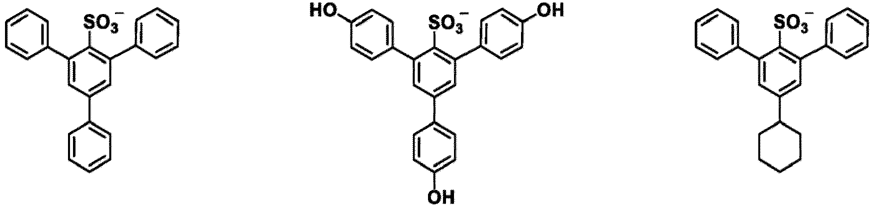

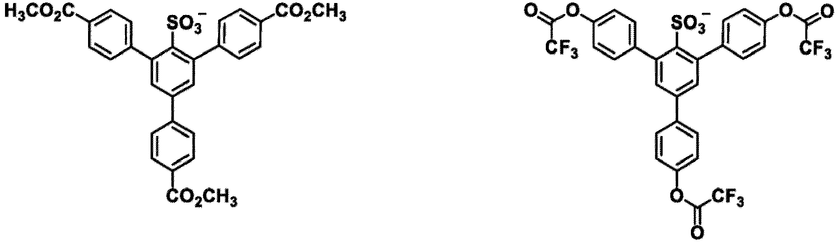

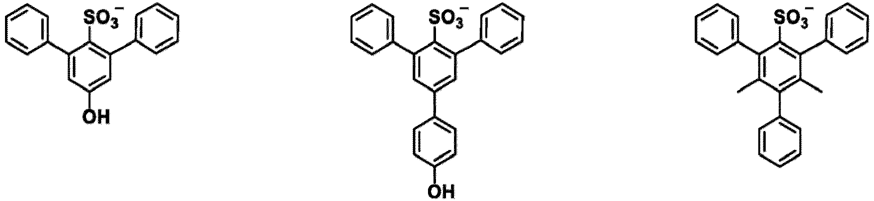

Also preferred as the PAG is a salt compound containing an anion having the formula (F1).

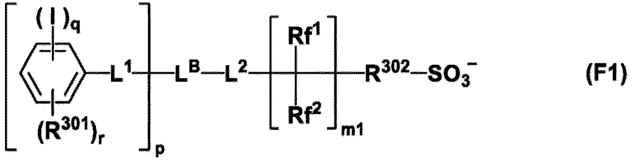

In formula (F1), m1 is 0 or 1, p is an integer of 1 to 3, q is an integer of 1 to 5, and r is an integer of 0 to 3.

In formula (F1), L¹ is a single bond, ether bond, ester bond, sulfonic ester bond, carbonate bond or carbamate bond.

In formula (F1), L² is an ether bond, ester bond, sulfonic ester bond, carbonate bond or carbamate bond.

In formula (F1), L^{B} is a single bond or a C₁-C₂₀ hydrocarbylene group when p is 1. L^{B} is a C₁-C₂₀ (p+1)-valent hydrocarbon group when p is 2 or 3. The hydrocarbylene group and (p+1)-valent hydrocarbon group may contain at least one moiety selected from ether bond, carbonyl moiety, ester bond, amide bond, sultone ring, lactam ring, carbonate bond, halogen, hydroxy moiety and carboxy moiety.

The C₁-C₂₀ hydrocarbylene group L^{B} may be saturated or unsaturated and straight, branched or cyclic. Examples thereof include C₁-C₂₀ alkanediyl groups such as methylene, ethane-1,1-diyl, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl, decane-1,10-diyl, undecane-1,11-diyl, dodecane-1,12-diyl; C₃-C₂₀ cyclic saturated hydrocarbylene groups such as cyclopentanediyl, cyclohexanediyl, norbornanediyl and adamantanediyl; C₂-C₂₀ unsaturated aliphatic hydrocarbylene groups such as vinylene and propene-1,3-diyl; C₆-C₂₀ arylene groups such as phenylene and naphthylene; and combinations thereof. The C₁-C₂₀ (p+1)-valent hydrocarbon group L^{B} may be saturated or unsaturated and straight, branched or cyclic. Examples thereof include those exemplified above for the C₁-C₂₀ hydrocarbylene group, with one or two hydrogen atoms being eliminated.

In formula (F1), Rf¹ and Rf² are each independently hydrogen, fluorine or trifluoromethyl, at least one being fluorine or trifluoromethyl.

In formula (F1), R³⁰¹ is hydroxy, carboxy, a C₁-C₆ saturated hydrocarbyl group, C₁-C₆ saturated hydrocarbyloxy group, C₂-C₆ saturated hydrocarbylcarbonyloxy group, fluorine, chlorine, bromine, amino, -N(R^{301A})-C(=O)-R^{301B} or -N(R^{301A})-C(=O)-O-R^{301B}. R^{301A} is hydrogen or a C₁-C₆ saturated hydrocarbyl group. R^{301B} is a C₁-C₆ saturated hydrocarbyl group or C₂-C₈ unsaturated aliphatic hydrocarbyl group.

The C₁-C₆ saturated hydrocarbyl group represented by R³⁰¹, R^{301A} and R^{301B} may be straight, branched or cyclic. Examples thereof include C₁-C₆ alkyl groups such as methyl, ethyl, n-propyl, isopropyl, n-butyl, isobutyl, sec-butyl, tert-butyl, n-pentyl, and n-hexyl; and C₃-C₆ cyclic saturated hydrocarbyl groups such as cyclopropyl, cyclobutyl, cyclopentyl, and cyclohexyl. Examples of the saturated hydrocarbyl moiety in the C₁-C₆ saturated hydrocarbyloxy group represented by R³⁰¹ are as exemplified above for the saturated hydrocarbyl group. Examples of the saturated hydrocarbyl moiety in the C₂-C₆ saturated hydrocarbylcarbonyloxy group represented by R³⁰¹ are as exemplified above for the C₁-C₆ saturated hydrocarbyl group, but of 1 to 5 carbon atoms.

The C₂-C₈ unsaturated aliphatic hydrocarbyl group represented by R^{301B} may be straight, branched or cyclic and examples thereof include C₂-C₈ alkenyl groups such as vinyl, propenyl, butenyl, and hexenyl; C₂-C₈ alkynyl groups such as ethynyl, propynyl, and butynyl; and C₃-C₈ cyclic unsaturated aliphatic hydrocarbyl groups such as cyclohexenyl and norbornenyl.

In formula (F1), R³⁰² is a C₁-C₂₀ saturated hydrocarbylene group or C₆-C₁₄ arylene group. Some or all of the hydrogen atoms in the saturated hydrocarbylene group may be substituted by halogen other than fluorine. Some or all of the hydrogen atoms in the arylene group may be substituted by a substituent selected from C₁-C₂₀ saturated hydrocarbyl groups, C₁-C₂₀ saturated hydrocarbyloxy groups, C₆-C₁₄ aryl groups, halogen, and hydroxy.

The C₁-C₂₀ saturated hydrocarbylene group represented by R³⁰² may be straight, branched or cyclic. Examples thereof include C₁-C₂₀ alkanediyl groups such as methylene, ethane-1,1-diyl, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl, decane-1,10-diyl, undecane-1,11-diyl, dodecane-1,12-diyl; and C₃-C₂₀ cyclic saturated hydrocarbylene groups such as cyclopentanediyl, cyclohexanediyl, norbornanediyl and adamantanediyl.

Examples of the C₆-C₁₄ arylene group represented by R³⁰² include phenylene, naphthylene, phenanthrenediyl, and anthracenediyl. The C₁-C₂₀ saturated hydrocarbyl moiety and hydrocarbyl moiety in the C₁-C₂₀ hydrocarbyloxy moiety, which are substituents on the arylene group, may be straight, branched or cyclic and examples thereof include C₁-C₂₀ alkyl groups such as methyl, ethyl, n-propyl, isopropyl, n-butyl, isobutyl, sec-butyl, tert-butyl, n-pentyl, n-hexyl, n-octyl, n-nonyl, n-decyl, undecyl, dodecyl, tridecyl, tetradecyl, pentadecyl, heptadecyl, octadecyl, nonadecyl, and icosyl; and C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl and adamantyl. Examples of the C₆-C₁₄ arylene moiety which is a substituent on the arylene group include phenylene, naphthylene, phenanthrenediyl and anthracenediyl.

More preferably, the anion has the formula (F2).

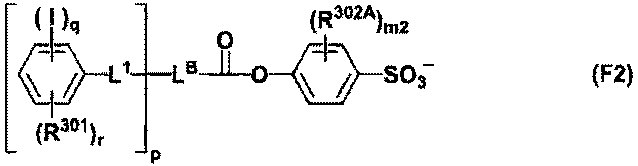

In formula (F2), p, q, r, L¹, E^{B}, and R³⁰¹ are as defined above. The subscript m2 is an integer of 1 to 4. R^{302A} is a C₁-C₂₀ saturated hydrocarbyl group, C₁-C₂₀ saturated hydrocarbyloxy group, C₆-C₁₄ aryl group, halogen or hydroxy group. When m2 is 2, 3 or 4, a plurality of R^{302A} may be identical or different.

Examples of the anion having formula (F1) are shown below, but not limited thereto.

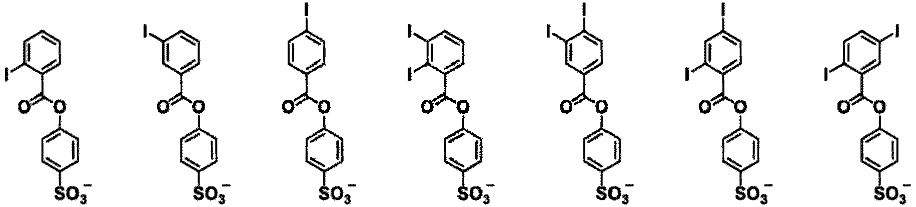

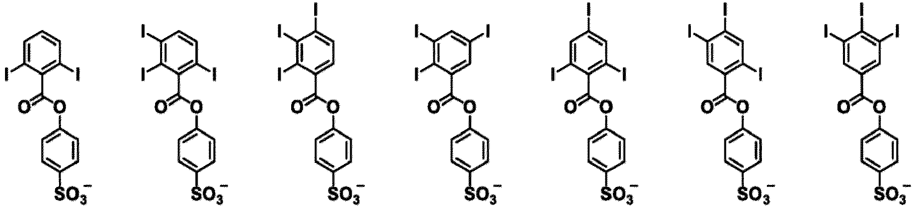

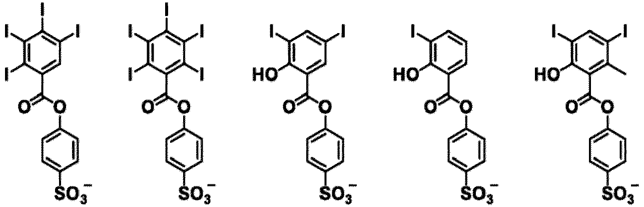

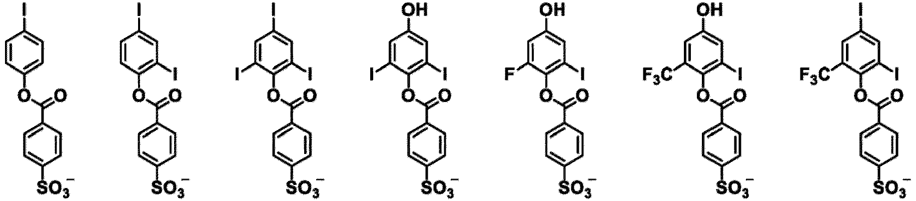

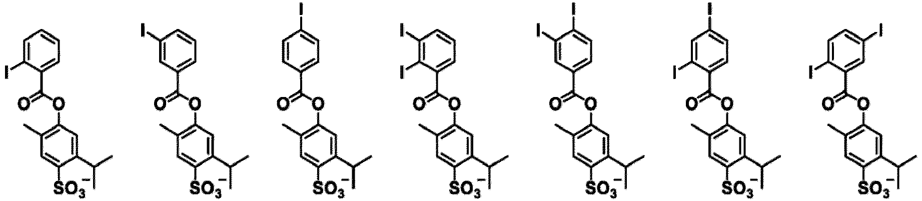

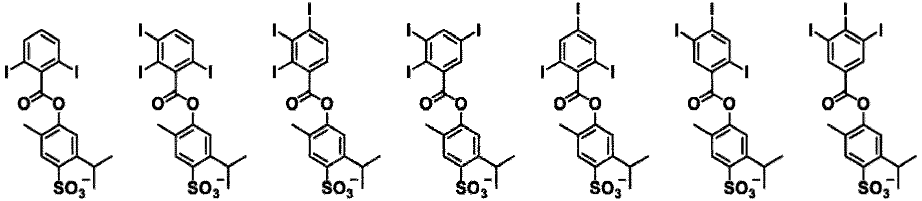

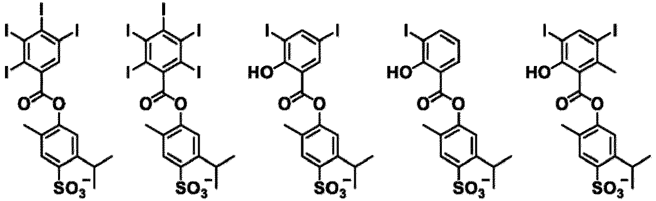

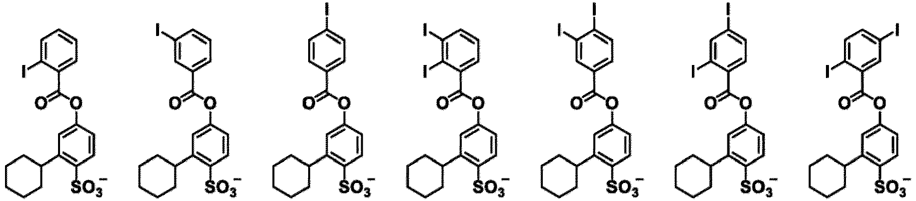

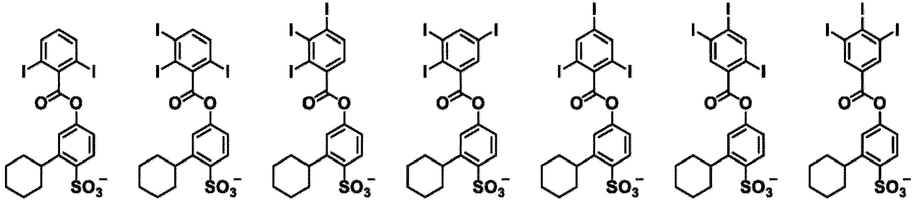

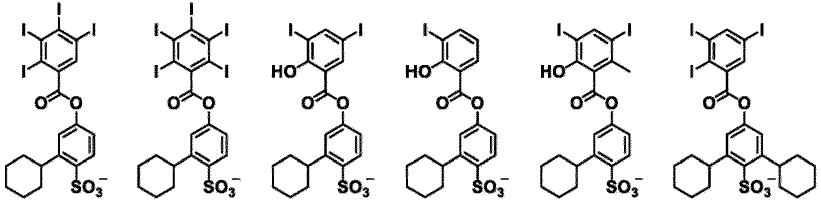

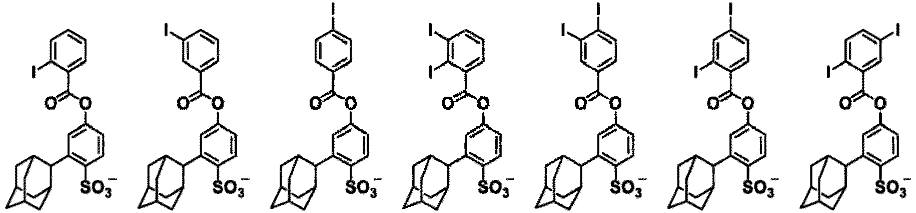

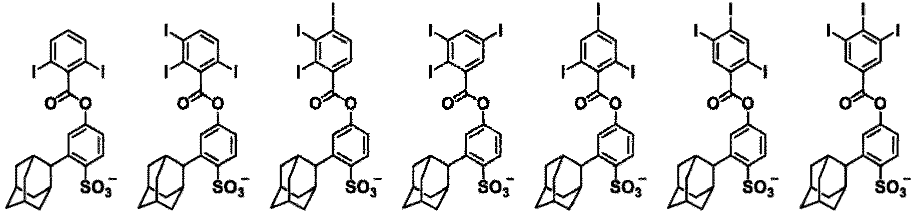

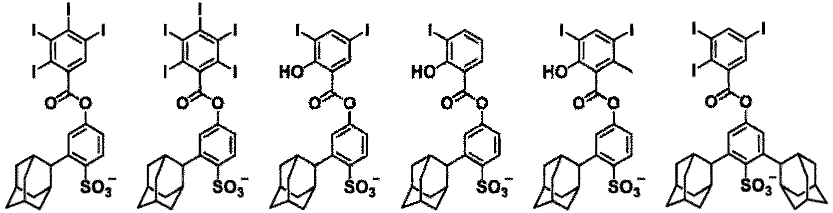

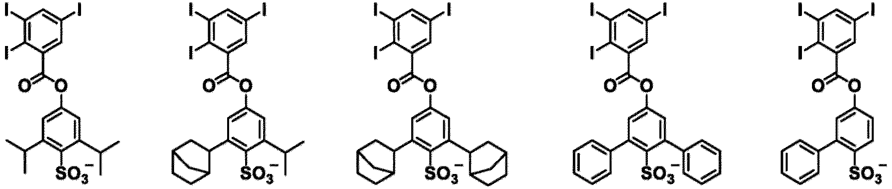

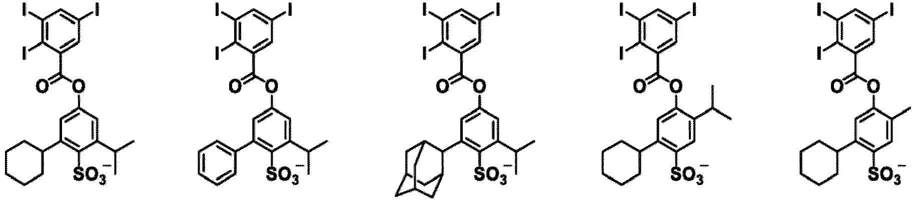

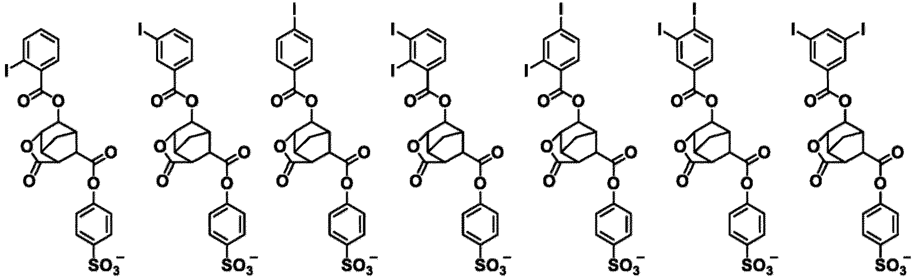

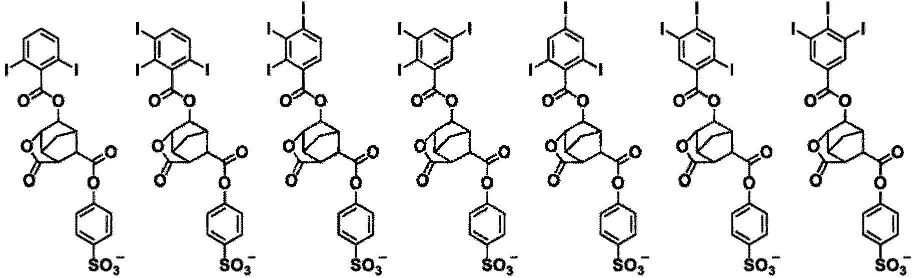

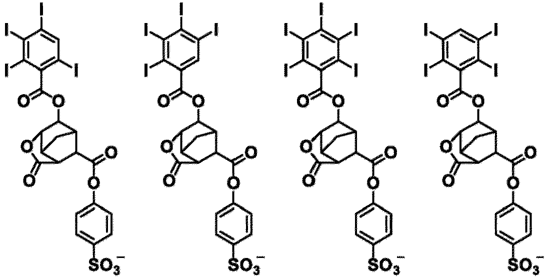

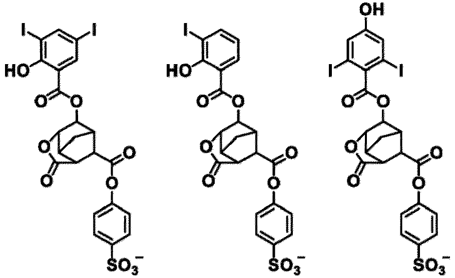

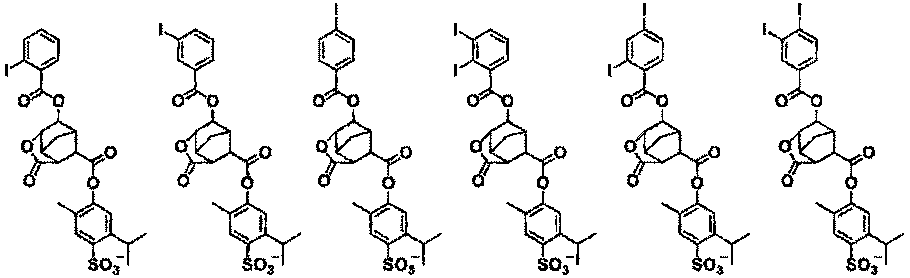

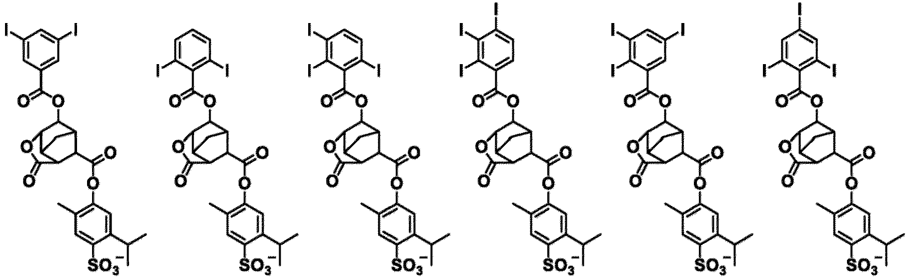

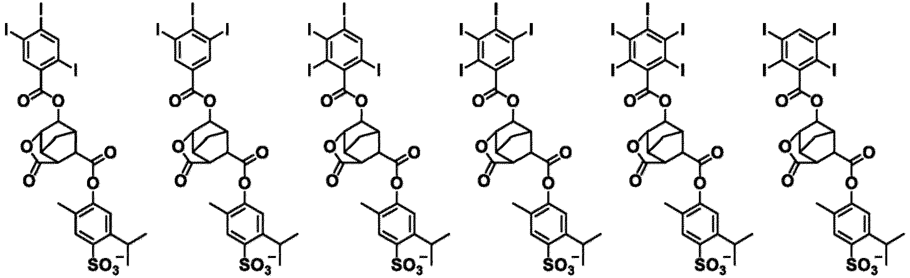

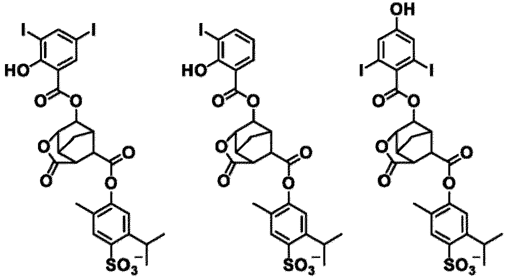

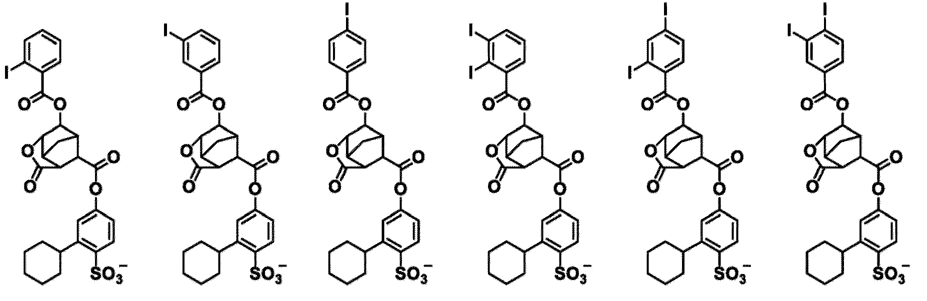

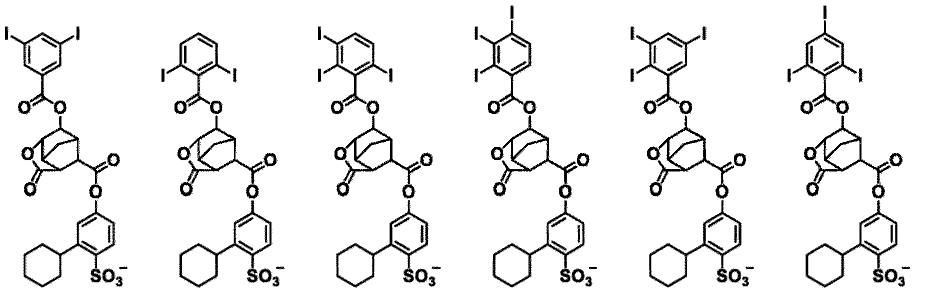

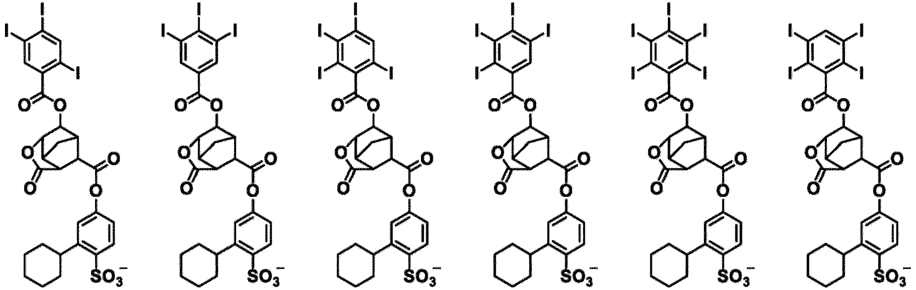

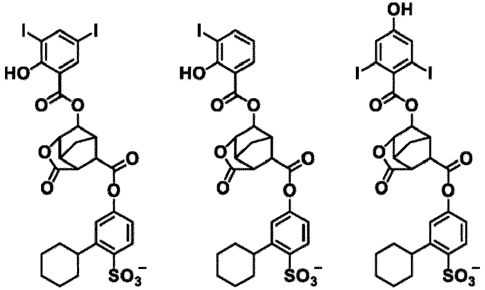

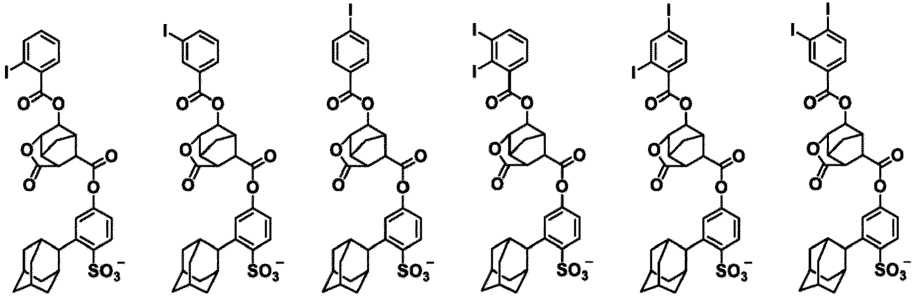

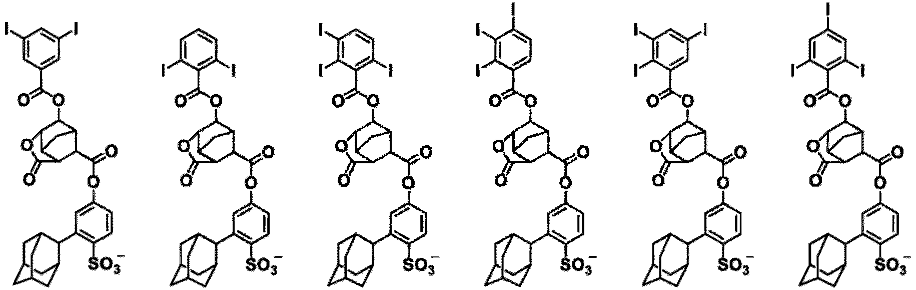

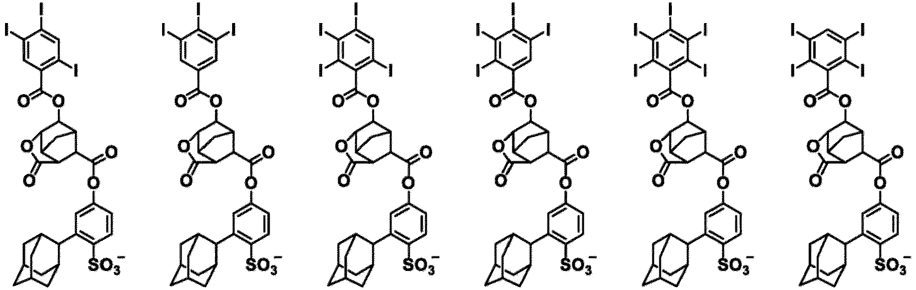

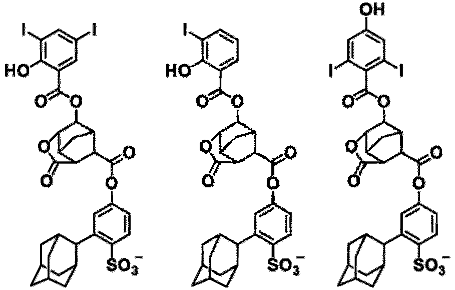

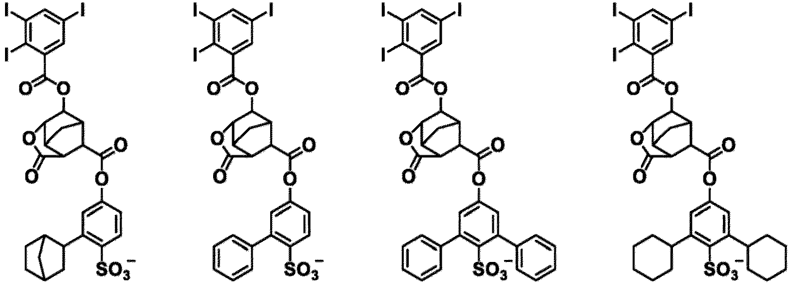

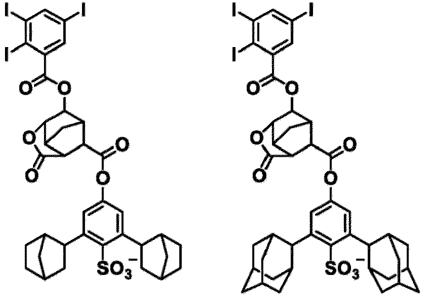

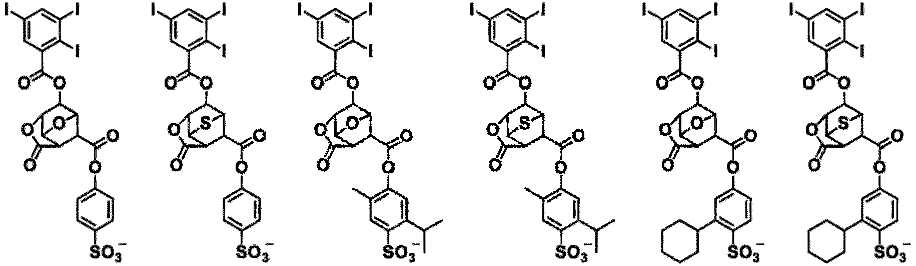

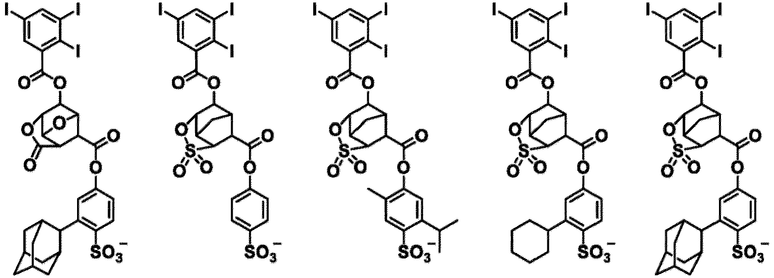

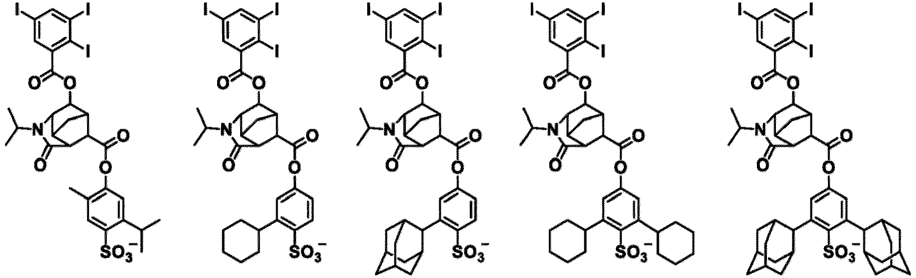

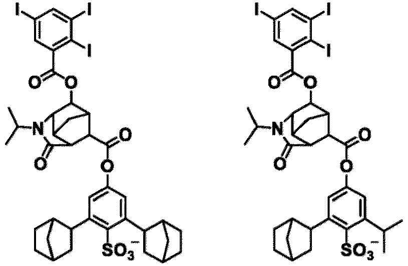

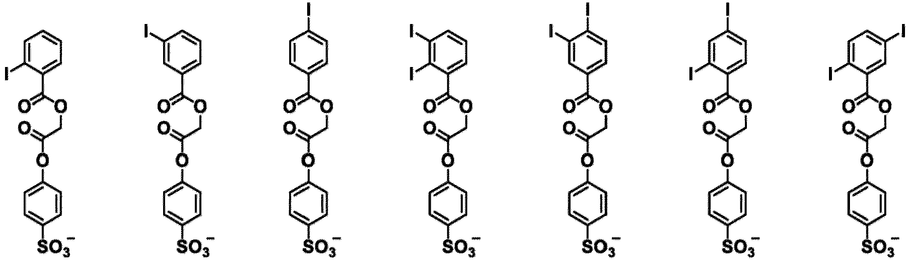

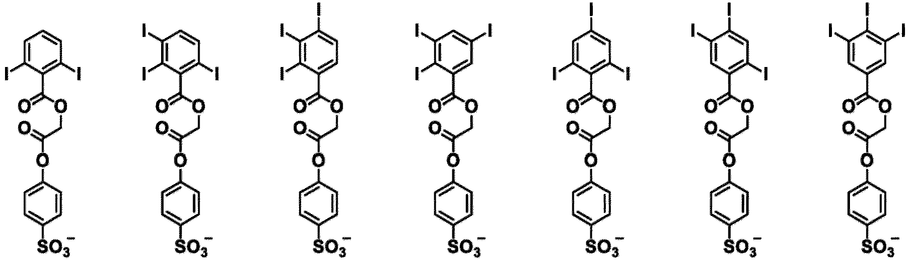

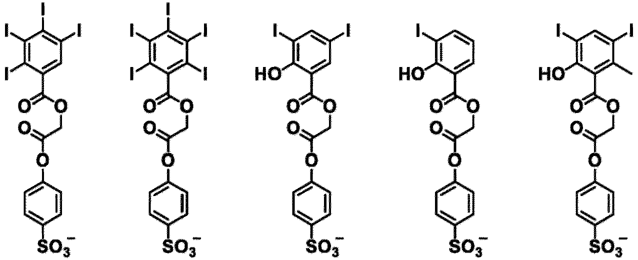

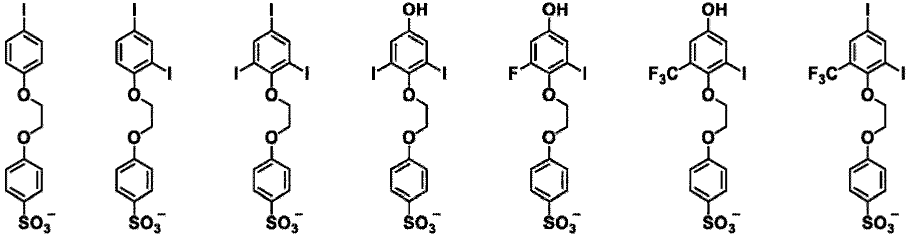

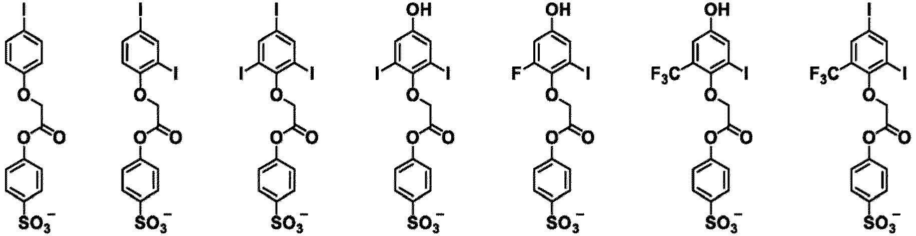

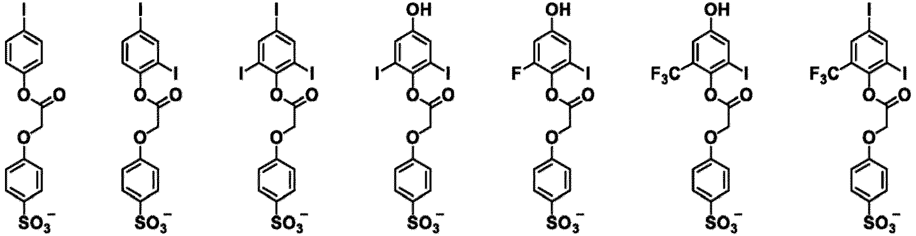

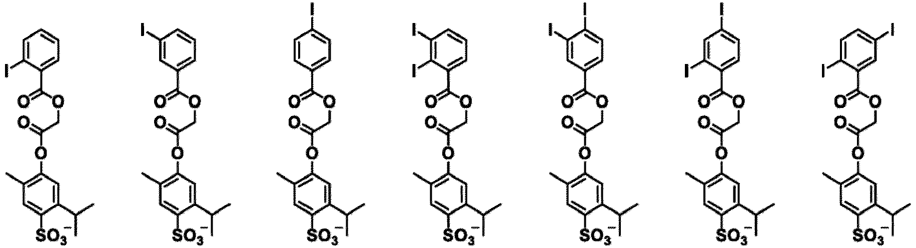

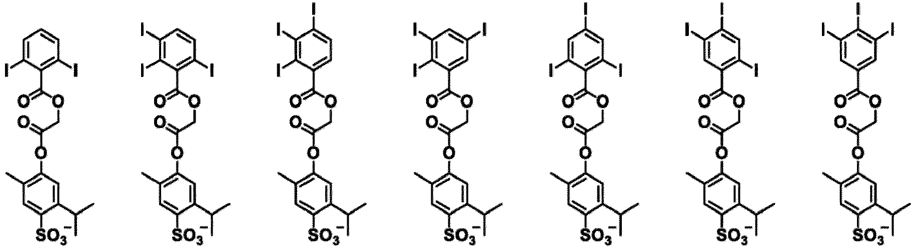

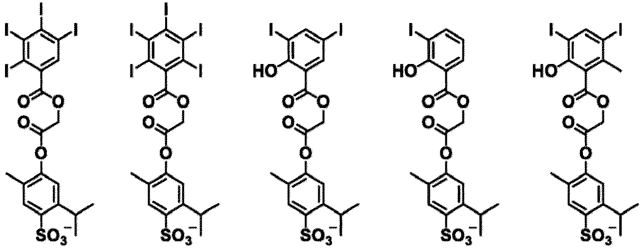

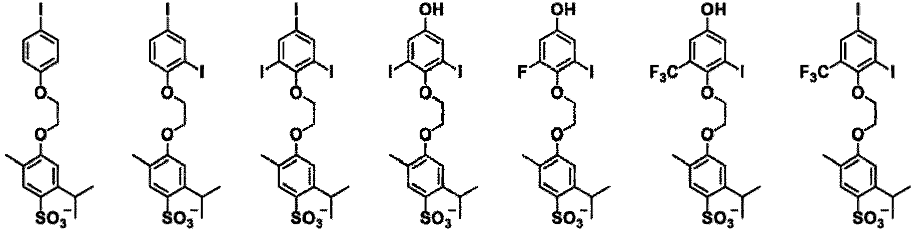

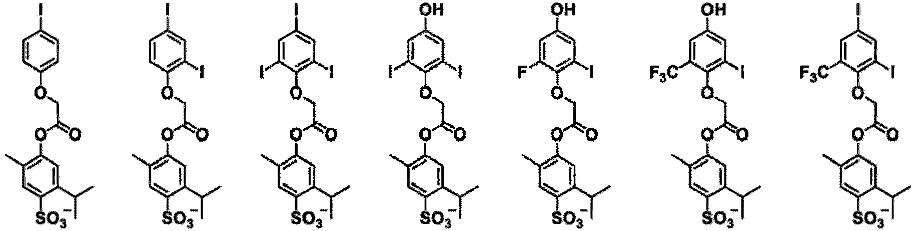

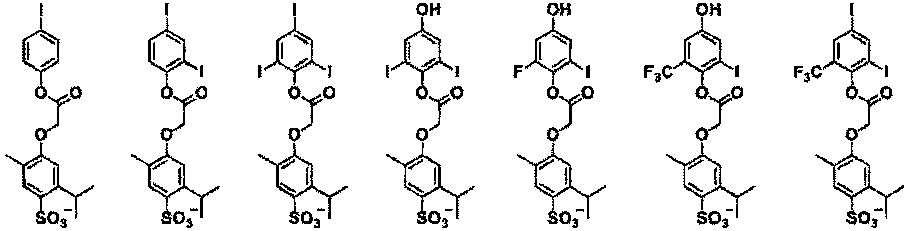

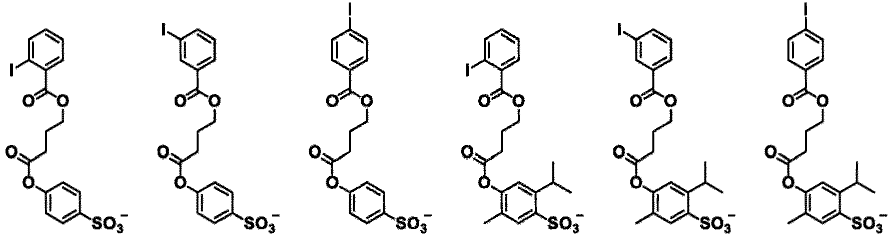

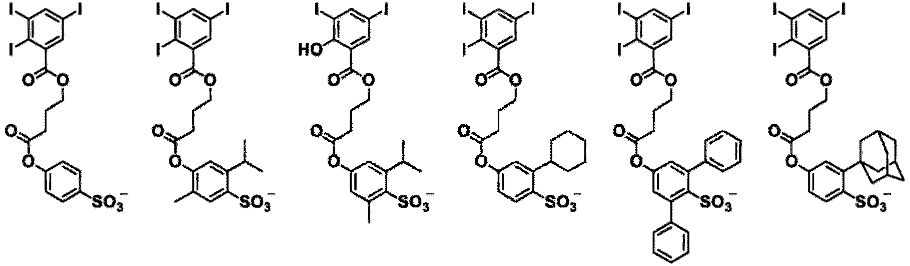

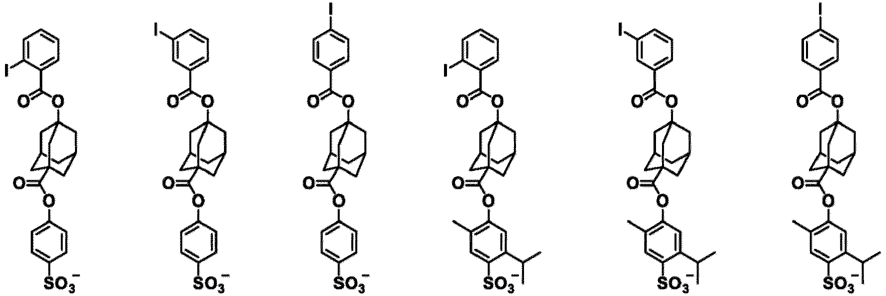

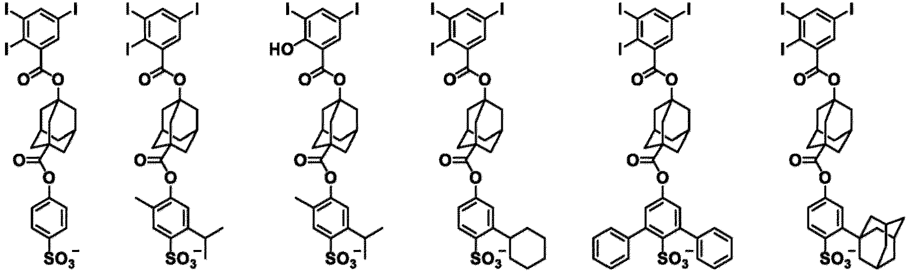

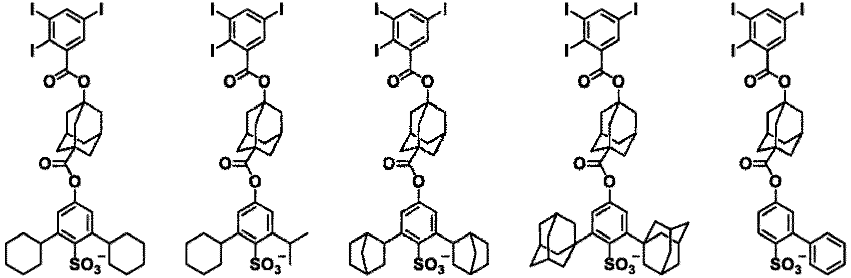

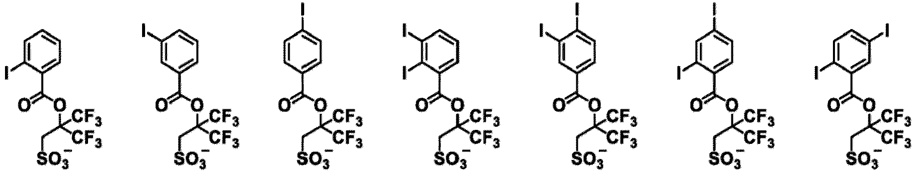

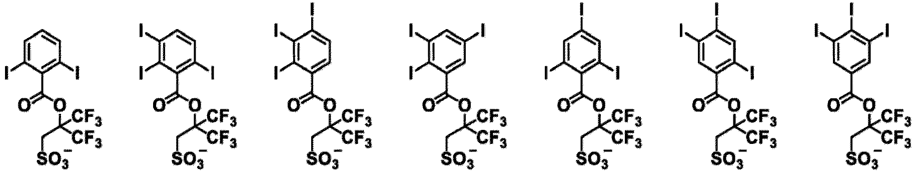

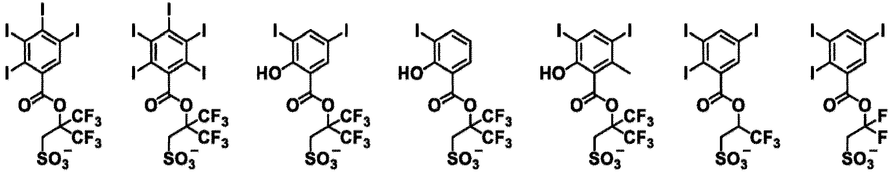

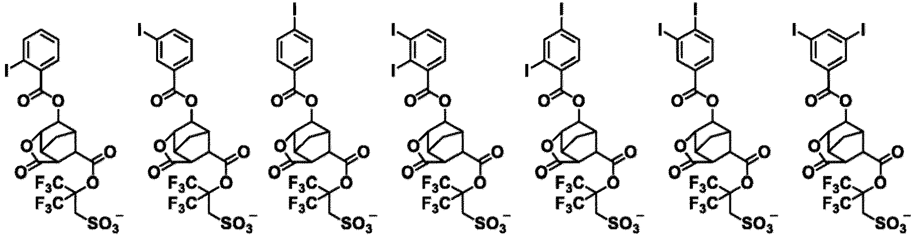

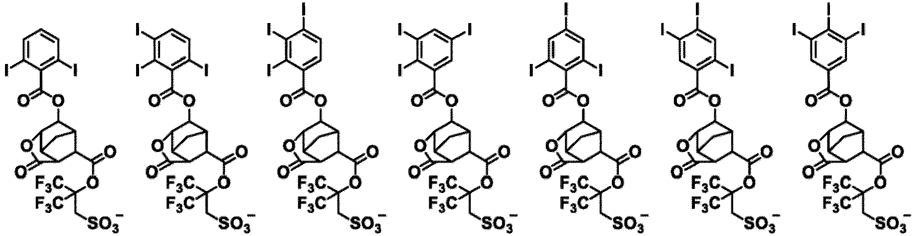

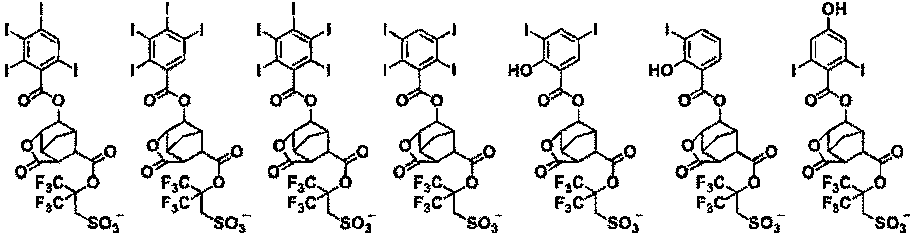

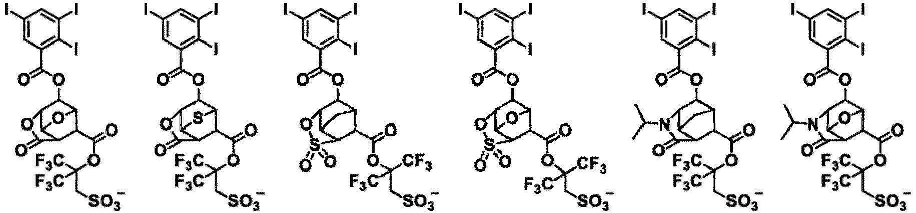

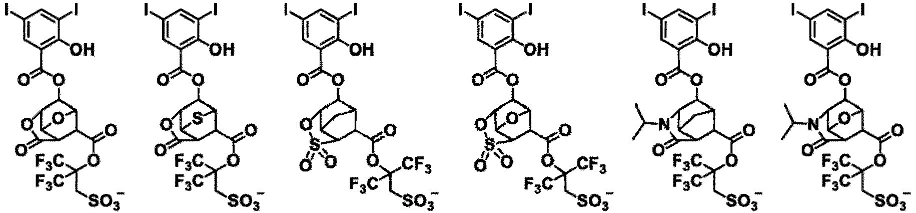

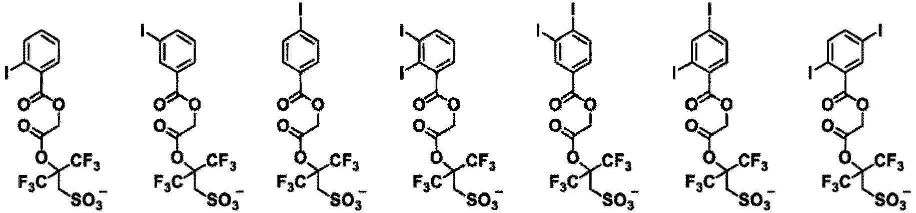

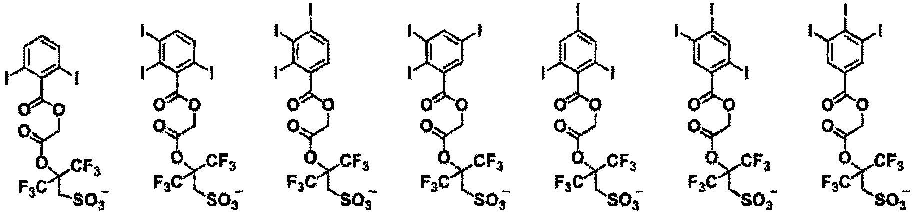

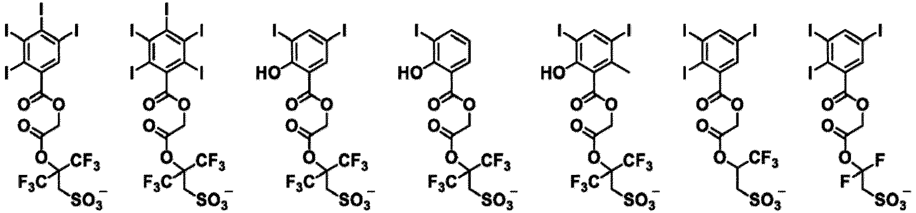

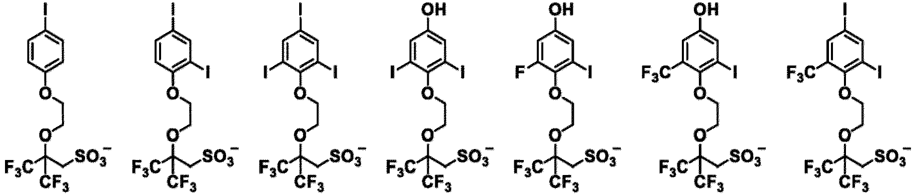

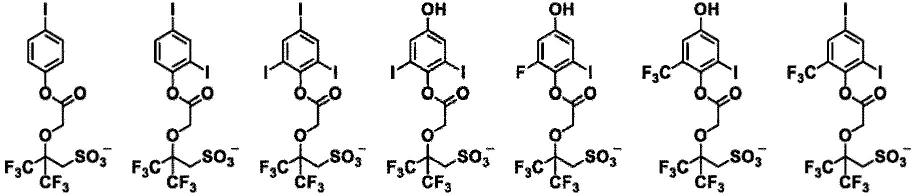

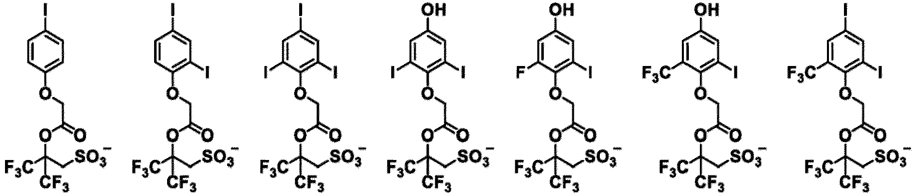

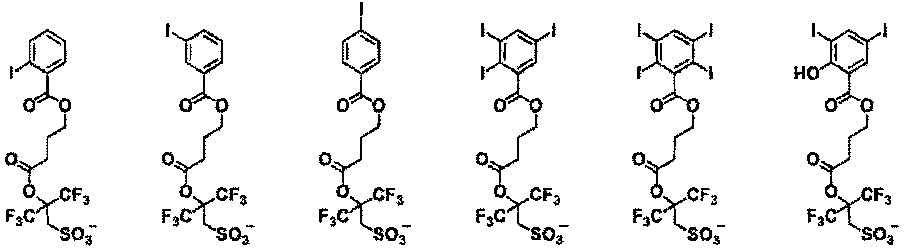

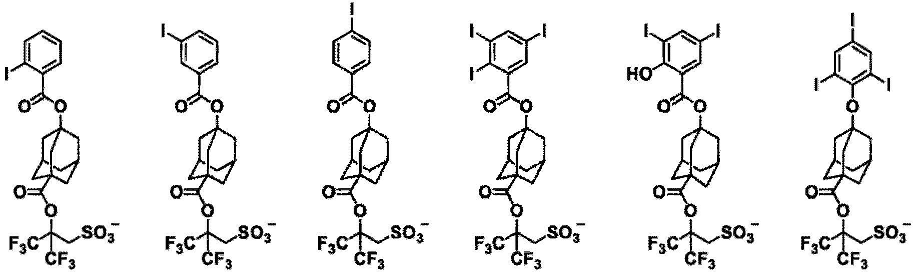

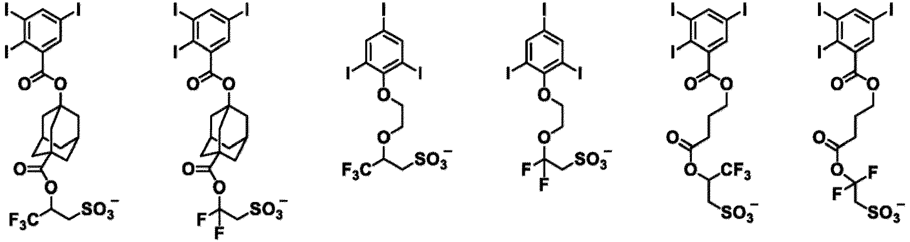

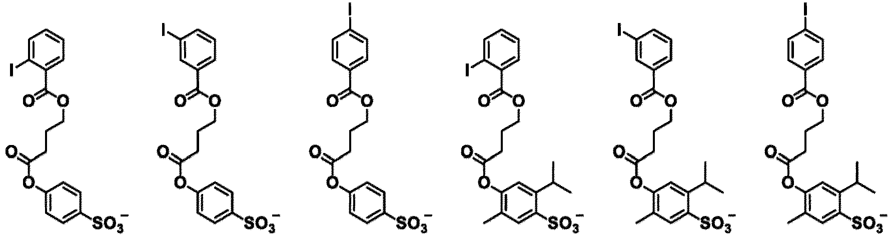

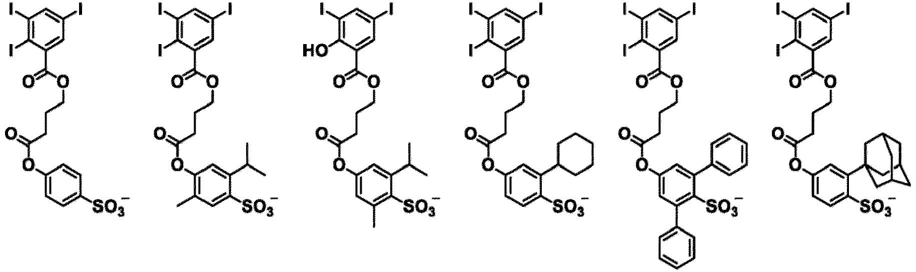

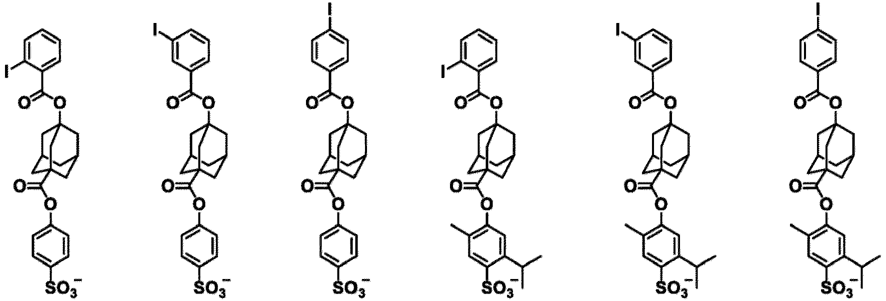

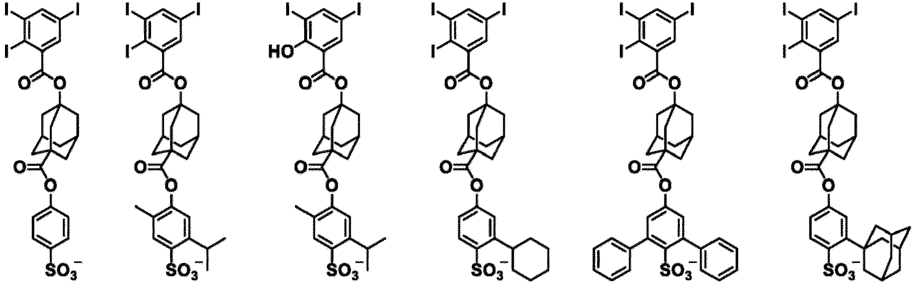

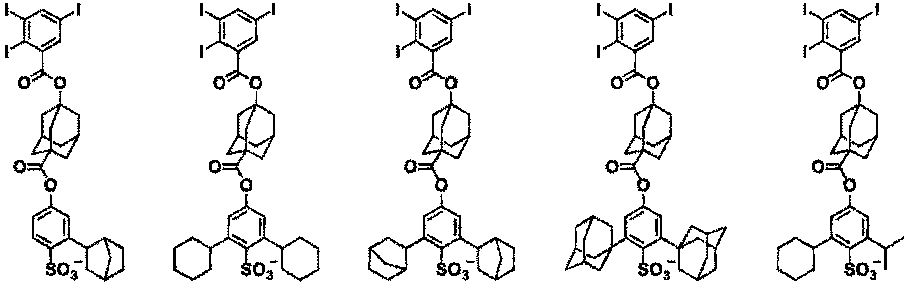

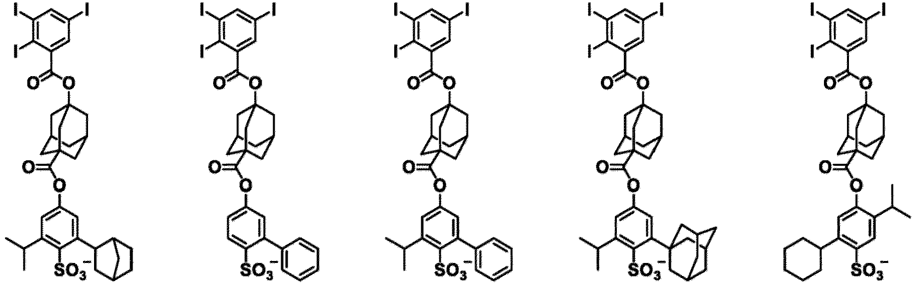

Preferred examples of the cation that pairs with the anion include sulfonium and iodonium cations. Examples of the sulfonium cation are as exemplified above for the sulfonium cation having formula (cation-1), but not limited thereto. Examples of the iodonium cation are as exemplified above for the iodonium cation having formula (cation-2), but not limited thereto.

The other PAG generates an acid having a pKa value of preferably -2.0 or larger, more preferably -1.0 or larger. The upper limit of pKa is preferably 2.0. Notably, the pKa value is computed using pKa DB in software ACD/Chemsketch ver: 9.04 of Advanced Chemistry Development Inc.

When the resist composition contains the other PAG (F), the amount of the PAG (F) used is preferably 1 to 10 parts, more preferably 1 to 5 parts by weight per 80 parts by weight of the base polymer (B). The other PAG may be used alone or in admixture. The inclusion of the other PAG provides for appropriate adjustment of the amount of acid generated in the exposed region and the degree of dissolution inhibition in the unexposed region.

### (G) Surfactant

The resist composition may contain any conventional surfactants for facilitating to coat the composition to the substrate. A number of surfactants are known in the art as described in WO 2006/121096, JP-A 2008-102383, JP-A 2008-304590, JP-A 2004-115630, and JP-A 2005-008766, and any suitable one may be chosen therefrom.

When the resist composition contains the surfactant (G), the amount of the surfactant (G) added is preferably up to 2 parts by weight, more preferably up to 1 part by weight and preferably at least 0.01 part by weight per 80 parts by weight of the base polymer (B).

### Process

A further embodiment of the invention is a pattern forming process comprising the steps of applying the chemically amplified positive resist composition defined above onto a substrate to form a resist film thereon, exposing the resist film to a pattern of high-energy radiation, and developing the exposed resist film in an alkaline developer.

The substrate used herein may be selected from, for example, substrates for IC fabrication, e.g., Si, SiO, SiO₂, SiN, SiON, TiN, WSi, BPSG, SOG, and organic antireflective coating, and substrates for mask circuit fabrication, e.g., Cr, CrO, CrON, MoSi₂, Si, SiO, and SiO₂.

The resist composition is first applied onto a substrate by a suitable coating technique such as spin coating. The coating is prebaked on a hotplate preferably at a temperature of 60 to 150°C for 1 to 20 minutes, more preferably at 80 to 140°C for 1 to 10 minutes to form a resist film of 0.03 to 2 µm thick.

Then the resist film is exposed patternwise to high-energy radiation. Examples of the high-energy radiation include UV, deep UV, excimer laser radiation (typically, KrF and ArF), EB, EUV, X-ray, γ-ray, and synchrotron radiation.

On use of UV, deep UV, excimer laser radiation, EUV, X-ray, γ-ray, and synchrotron radiation, the resist film is exposed through a mask having the desired pattern, preferably in a dose of 1 to 300 mJ/cm², more preferably 10 to 200 mJ/cm². On use of EB, a pattern may be directly written preferably in a dose of 1 to 300 pC/cm², more preferably 10 to 200 pC/cm². The resist composition of the invention is particularly useful in the EUV and EB lithography processes.

The exposure may be performed by conventional lithography whereas the immersion lithography of holding a liquid, typically water between the resist film and the mask may be employed if desired. In the case of immersion lithography, a protective film which is insoluble in water may be formed on the resist film.

After the exposure, the resist film may be baked (PEB), for example, on a hotplate preferably at 60 to 150°C for 1 to 20 minutes, and more preferably at 80 to 140°C for 1 to 10 minutes.

Finally, development is carried out using as the developer an aqueous alkaline solution, such as a 0.1 to 5 wt%, preferably 2 to 3 wt%, aqueous solution of tetramethylammonium hydroxide (TMAH), this being done by a conventional method such as dip, puddle, or spray development for a period of 0.1 to 3 minutes, and preferably 0.5 to 2 minutes. In this way the exposed region of resist film is dissolved away, forming the desired pattern on the substrate.

The resist composition of the invention is advantageous particularly on use under the situation that requires high etching resistance, and a minimal change of pattern line width and minimal LER even when the time duration from exposure to PEB is prolonged. It is also advantageous for pattern formation on a substrate having a surface layer of material to which the resist pattern is less adherent with a likelihood of pattern stripping or pattern collapse, specifically a substrate having sputter deposited thereon a layer of metallic chromium or a chromium compound containing one or more light elements such as oxygen, nitrogen and carbon. The resist composition is particularly useful in forming a pattern on a photomask blank as the substrate.

### EXAMPLES

Examples of the invention are given below by way of illustration and not by way of limitation. The abbreviation "pbw" is parts by weight. Analysis is made by time-of-flight mass spectrometry (TOF-MS) using an analyzer: MALDI TOF-MS: S3000 by JEOL Ltd.

### [1] Synthesis of onium salts

### Example 1-1

### Synthesis of PAG-1

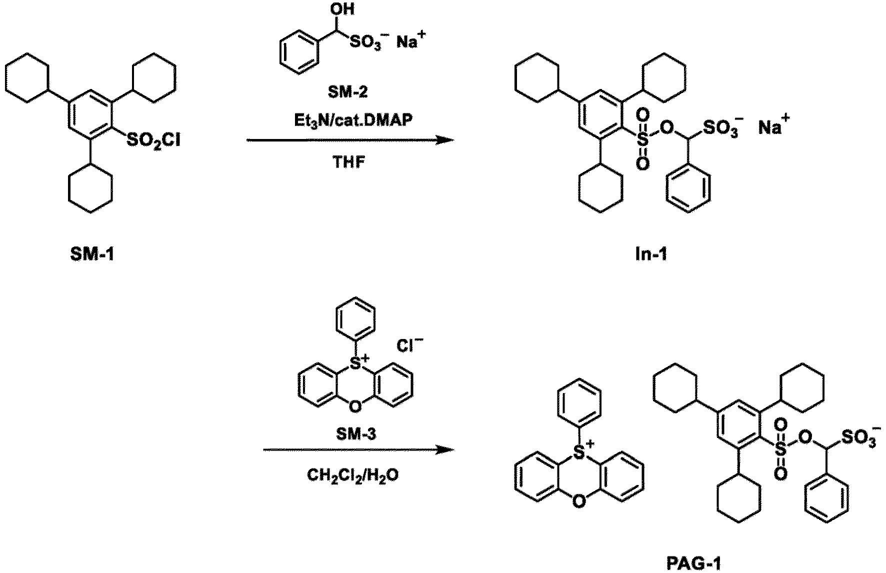

In nitrogen atmosphere, 10.6 g of SM-1, 6.3 g of SM-2 and 0.3 g of 4-dimethylaminopyridine were suspended in 50 g of THF. The suspension was cooled in an ice bath, after which 3.5 g of triethylamine was added dropwise to the suspension, which was stirred at room temperature for 10 hours. After aging, the reaction solution was cooled down and 50 g of water was added to quench the reaction. Thereafter, 9.4 g of SM-3 was added. The target compound was extracted with 100 g of methylene chloride and subjected to ordinary aqueous workup. The solvent was distilled off, obtaining 15.5 g of PAG-1 as oily matter (yield 73%).

PAG-1 was analyzed by TOF-MS.

### MALDI TOF-MS:

positive M⁺ 277 (corresponding to C₁₈H₁₃OS⁺)
negative M⁻ 573 (corresponding to C₃₁H₄₁O₆S⁻)

### Examples 1-2 to 1-7

### Synthesis of PAG-2 to PAG-7

Onium Salts PAG-2 to PAG-7, shown below, were synthesized through well-known organic synthesis reaction using the corresponding reactants.

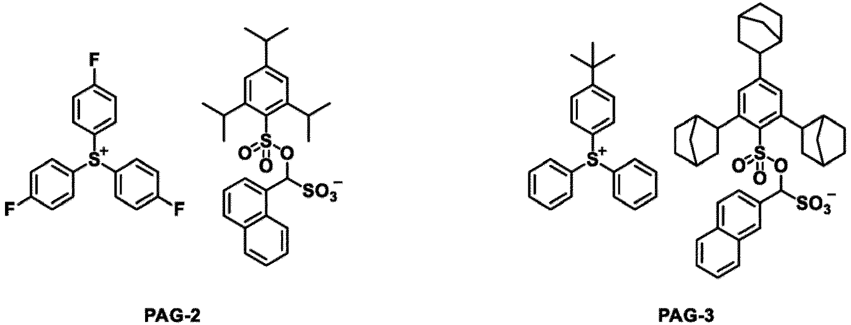

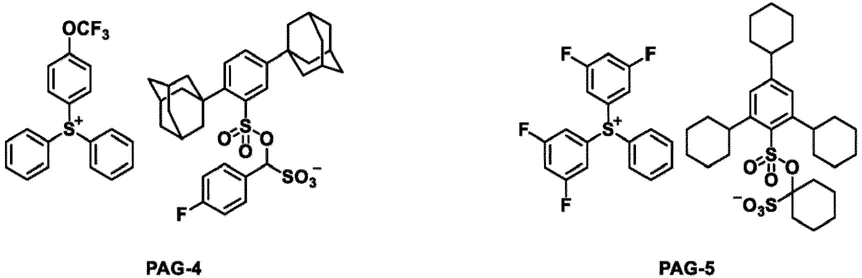

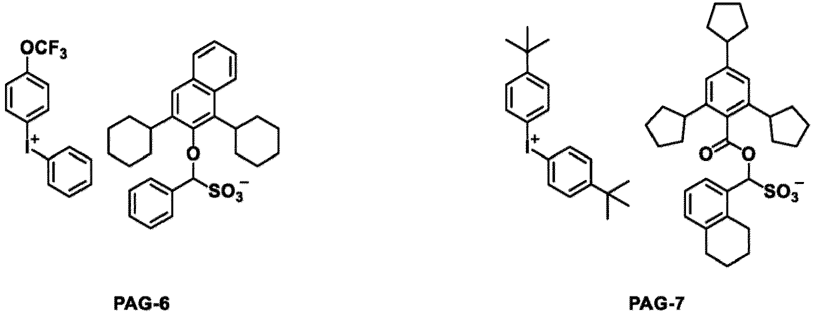

### [2] Synthesis of base polymer

### Synthesis Example 1

### Synthesis of Polymer P-1

A 100-ml flask was charged with 20 g of a hydroxystyrene-acenaphthylene copolymer and 46.7 g of THF solvent. In nitrogen atmosphere, 0.5 g of methanesulfonic acid was added to the solution at room temperature (~25°C), and 4.4 g of 1-methoxy-2-methylpropene was added dropwise thereto, after which reaction ran at room temperature for 4.5 hours. At the end of reaction, 1.0 g of triethylamine was added to the reaction solution, which was added dropwise to 500 g of hexane for precipitation. The solid precipitate was collected by filtration and washed twice with 120 g of hexane. The solid was dissolved in a mixture of 60 g of ethyl acetate and 20 g of water. The solution was transferred to a separatory funnel, to which 0.7 g of acetic acid was added, followed by separatory operation. After the lower layer was removed, 20 g of water and 0.9 g of pyridine were added to the organic layer, followed by separatory operation. After the lower layer was removed, 20 g of water was added to the organic layer, followed by water washing and separatory operation. The water washing and separatory operation was repeated 5 times in total. Thereafter, the organic layer was concentrated and dissolved in 40 g of PGME. The solution was added dropwise to 600 g of water for precipitation. The solid precipitate was collected by filtration, washed with water, and dried, obtaining 20.3 g of the target Polymer P-1 as white solids. Polymer P-1 was analyzed by ¹H-NMR, ¹³C-NMR and GPC, with the analysis results shown below.
P-1
**(a=0.62, b=0.11, c=0.27, Mw=5200)**

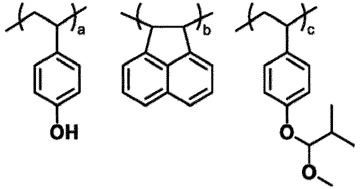

### Synthesis Examples 2 to 9

### Synthesis of Polymers P-2 to P-9

Polymers P-2 to P-9 shown below were synthesized with reference to Synthesis Example 1 and the well-known method except that the type and amount (blending ratio) of monomers were changed.

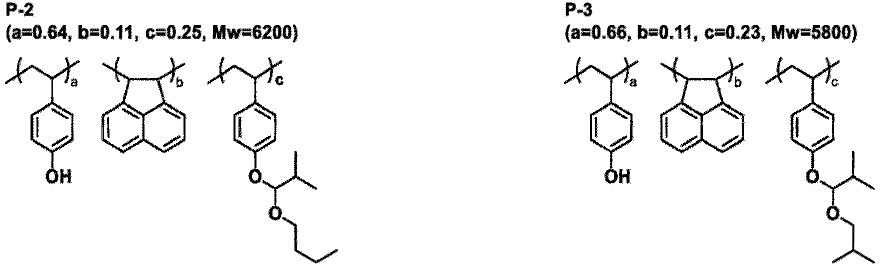

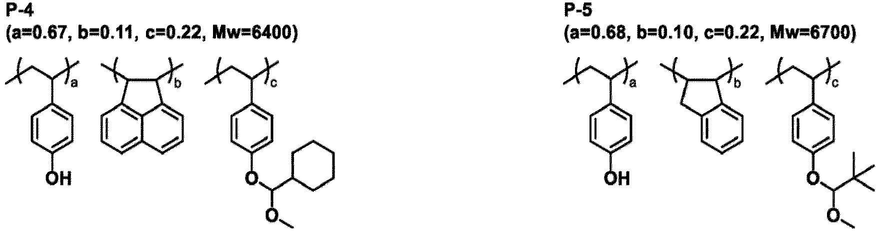

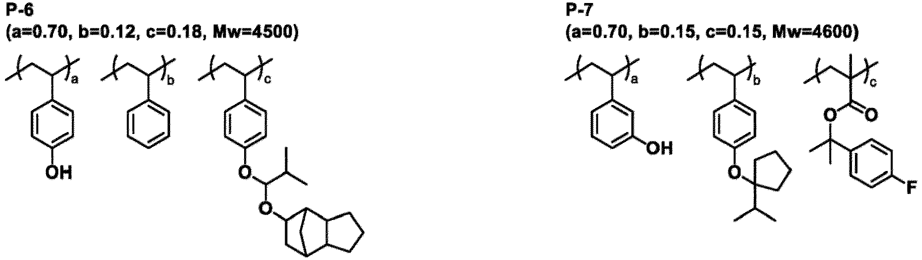

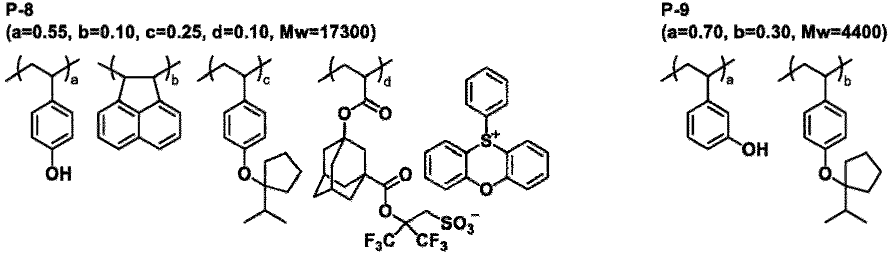

### [3] Preparation of resist composition

### Examples 2-1 to 2-32 and Comparative Examples 1-1 to 1-21

A chemically amplified positive resist composition was prepared by dissolving selected components in an organic solvent in accordance with the formulation shown in Tables 1 to 3, and filtering the solution through a nylon filter with a pore size of 5 nm and a UPE filter with a pore size of 1 nm. The organic solvent was a mixture of 940 pbw of PGMEA, 1,870 pbw of EL and 1,870 pbw of PGME.

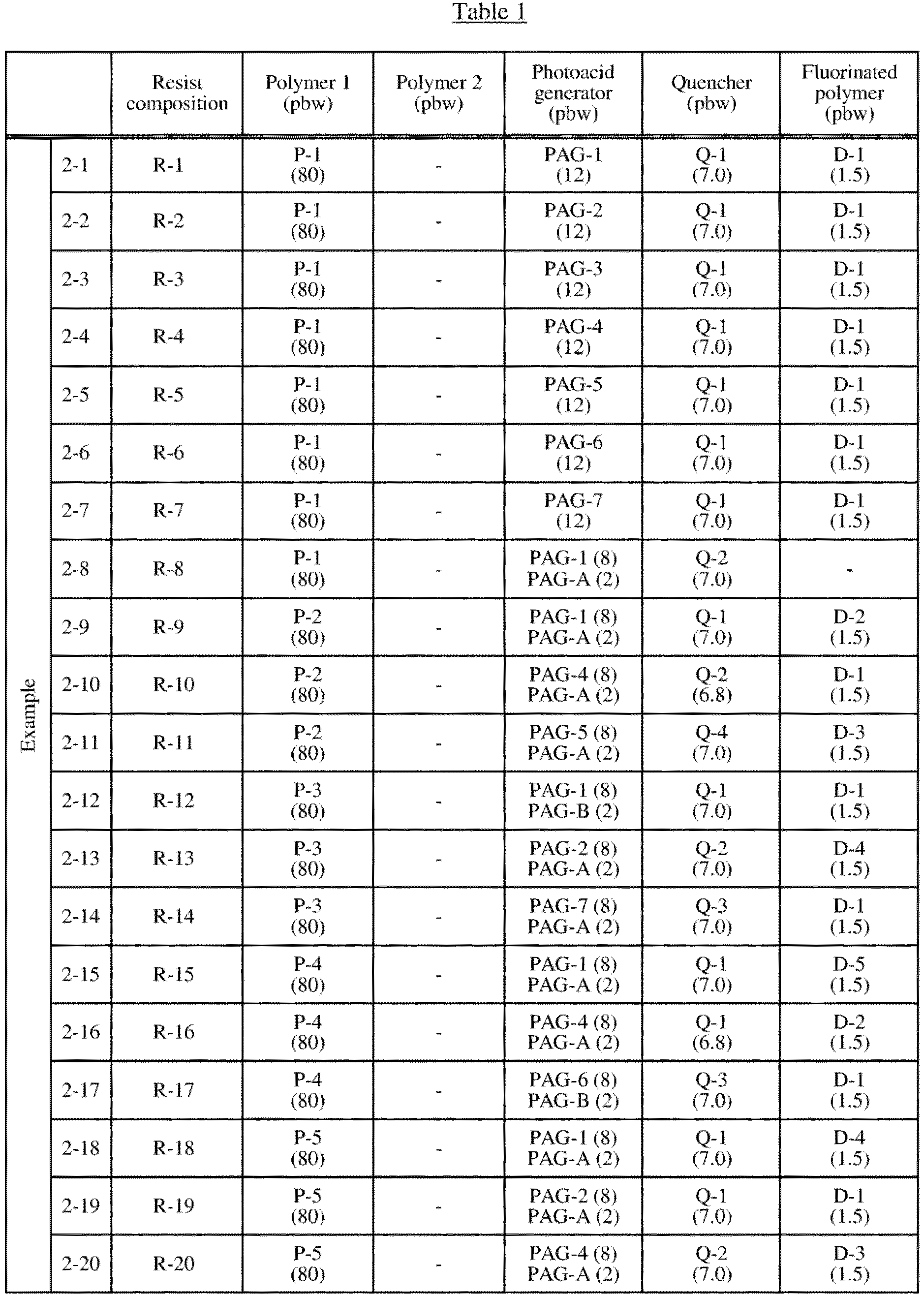

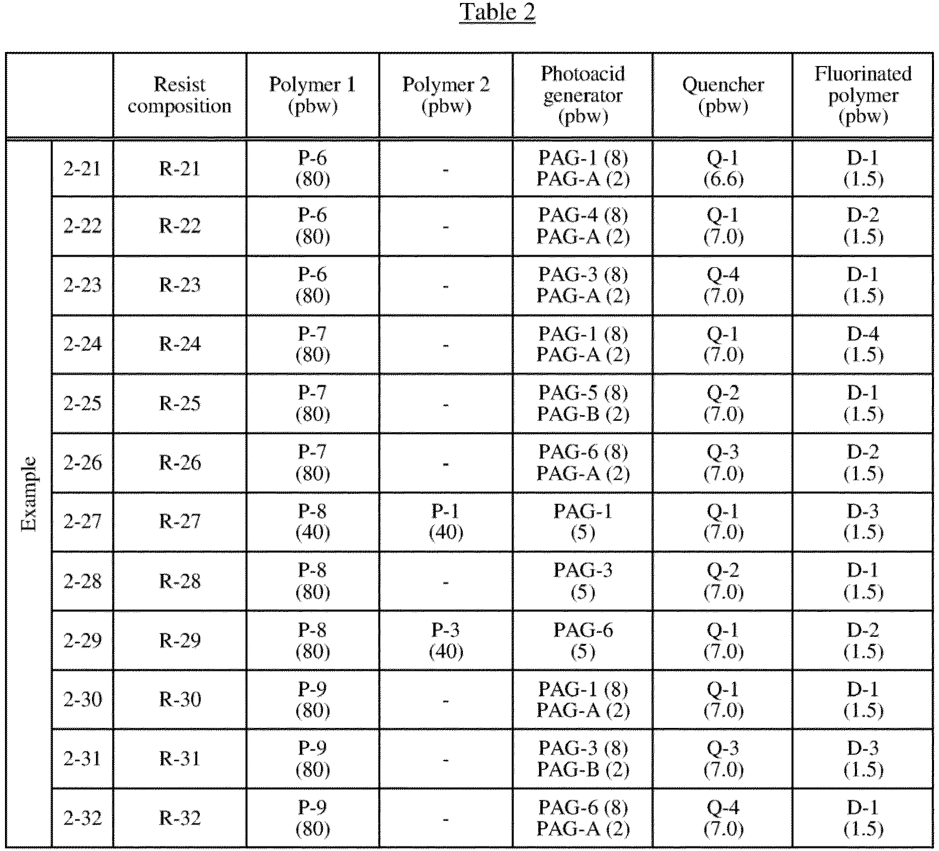

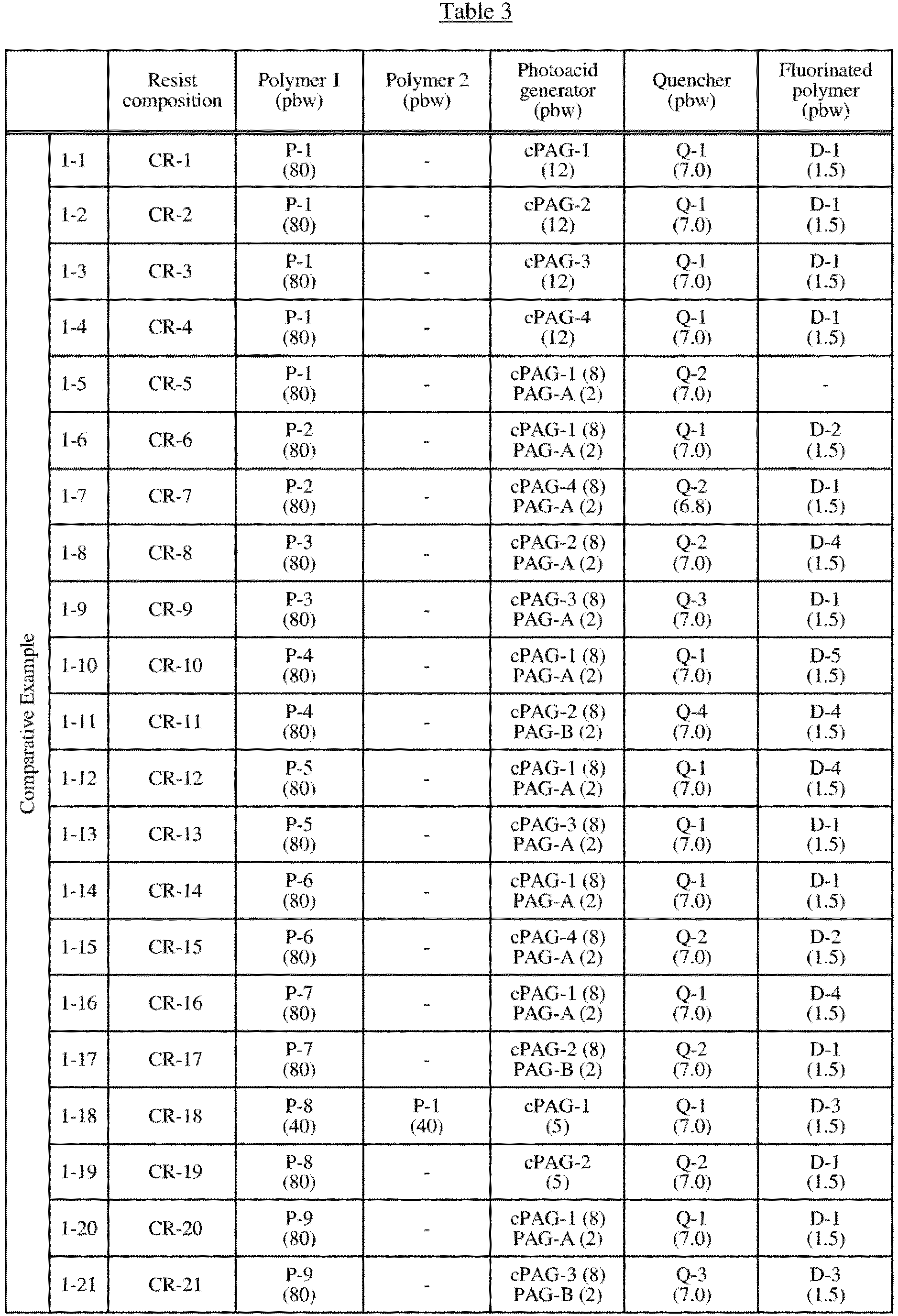

Comparative photoacid generators cPAG-1 to cPAG-4, Photoacid generators PAG-A and PAG-B, Quenchers Q-1 to Q-4, and Fluorinated Polymers D-1 to D-5 in Tables 1 to 3 are identified below.

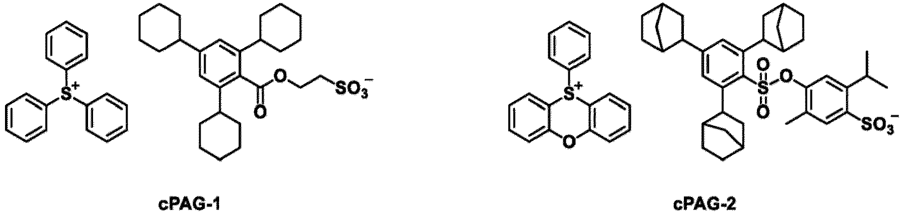

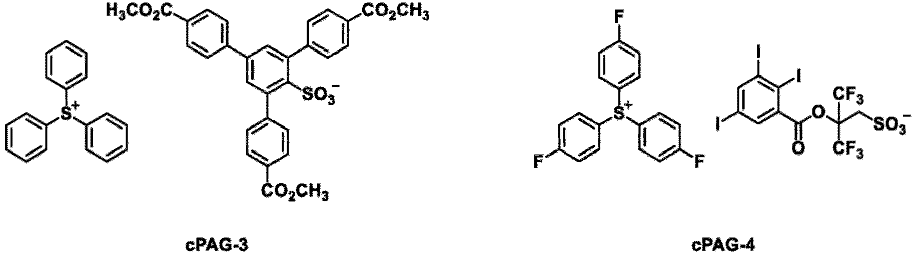

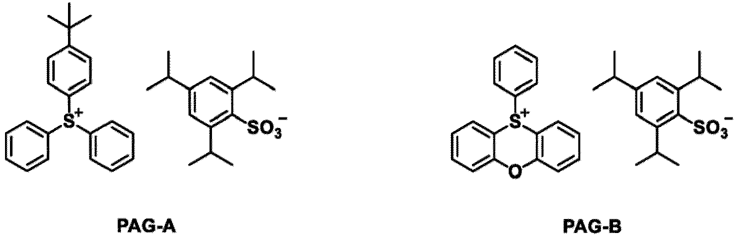

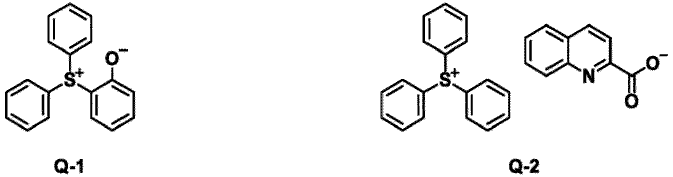

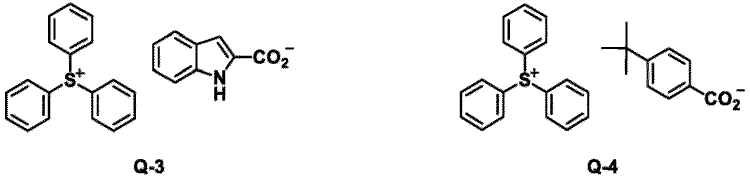

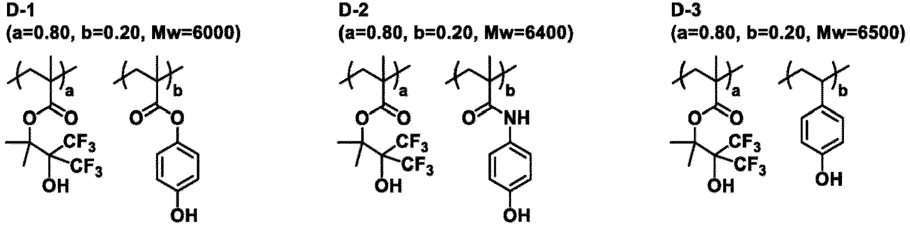

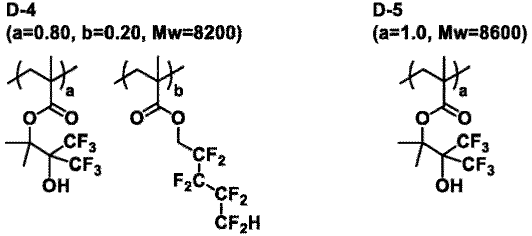

### [4] EB lithography test

### Examples 3-1 to 3-32 and Comparative Examples 2-1 to 2-21

A photomask blank of reflection type for an EUV lithography mask was furnished by starting with a low-coefficient-of-thermal-expansion glass substrate of 6 inches squares and depositing thereon a multilayer reflective film of 40 Mo/Si layers with a thickness of 284 nm, a Ru film of 3.5 nm thick as protective film, a TaN film of 70 nm thick as absorbing layer, and a CrN film of 6 nm thick as hard mask. Using a coater/developer system ACT-M (Tokyo Electron Ltd.), each of the resist compositions (R-1 to R-32, CR-1 to CR-21) was spin coated onto the photomask blank, and prebaked on a hotplate at 110°C for 600 seconds to form a resist film of 80 nm thick. The thickness of the resist film was measured by an optical film thickness measurement system Nanospec (Nanometrics Inc.). Measurement was made at 81 points in the plane of the blank substrate excluding a peripheral band extending 10 mm inward from the blank periphery, and an average film thickness and a film thickness range were computed therefrom.

The resist film was exposed to EB using an EB writer system EBM-5000Plus (NuFlare Technology Inc., accelerating voltage 50 kV), then baked (PEB) at 110°C for 600 seconds, and developed in a 2.38 wt% TMAH aqueous solution, thereby yielding a positive pattern.

The resist pattern was evaluated as follows. The patterned mask blank was observed under a top-down scanning electron microscope (TD-SEM). The optimum dose (Eop) was defined as the exposure dose (µC/cm²) which provided a 1:1 resolution at the top and bottom of a 200-nm 1:1 line-and-space (LS) pattern. The resolution (or maximum IS resolution) was defined as the minimum size at the dose which provided a 9:1 resolution for an isolated space (IS) of 200 nm. The edge roughness (LER) of a 200-nm LS pattern was measured under SEM. The develop loading was evaluated by forming a 200-nm LS pattern at the dose (µC/cm2) capable of resolving a 1:1 LS pattern of 200 nm design at a ratio 1:1 and a 200-nm LS pattern including dummy patterns having a density of 15%, 25%, 33%, 45%, 50%, 55%, 66%, 75%, 85%, and 95% arranged around the center pattern, measuring the size of spaces under SEM, and comparing the size difference among grouped and isolated patterns. Also, the pattern was visually observed to judge whether or not the profile was rectangular.

The dissolution rate of an exposed region is computed by spin coating the resist solution onto a 8-inch silicon wafer, baking at 110°C for 60 seconds to form a resist film of 90 nm thick, exposing the resist film to KrF excimer laser radiation in a dose (mJ/cm²) capable of resolving a 200-nm 1:1 LS pattern at a ratio 1:1, baking at 110°C for 60 seconds, developing the film in a 2.38 wt% TMAH aqueous solution at 23°C, and measuring a loss of film thickness by means of a resist development rate analyzer (RDA-800 by Litho Tech Japan Corp.). The results are shown in Tables 4 and 5.

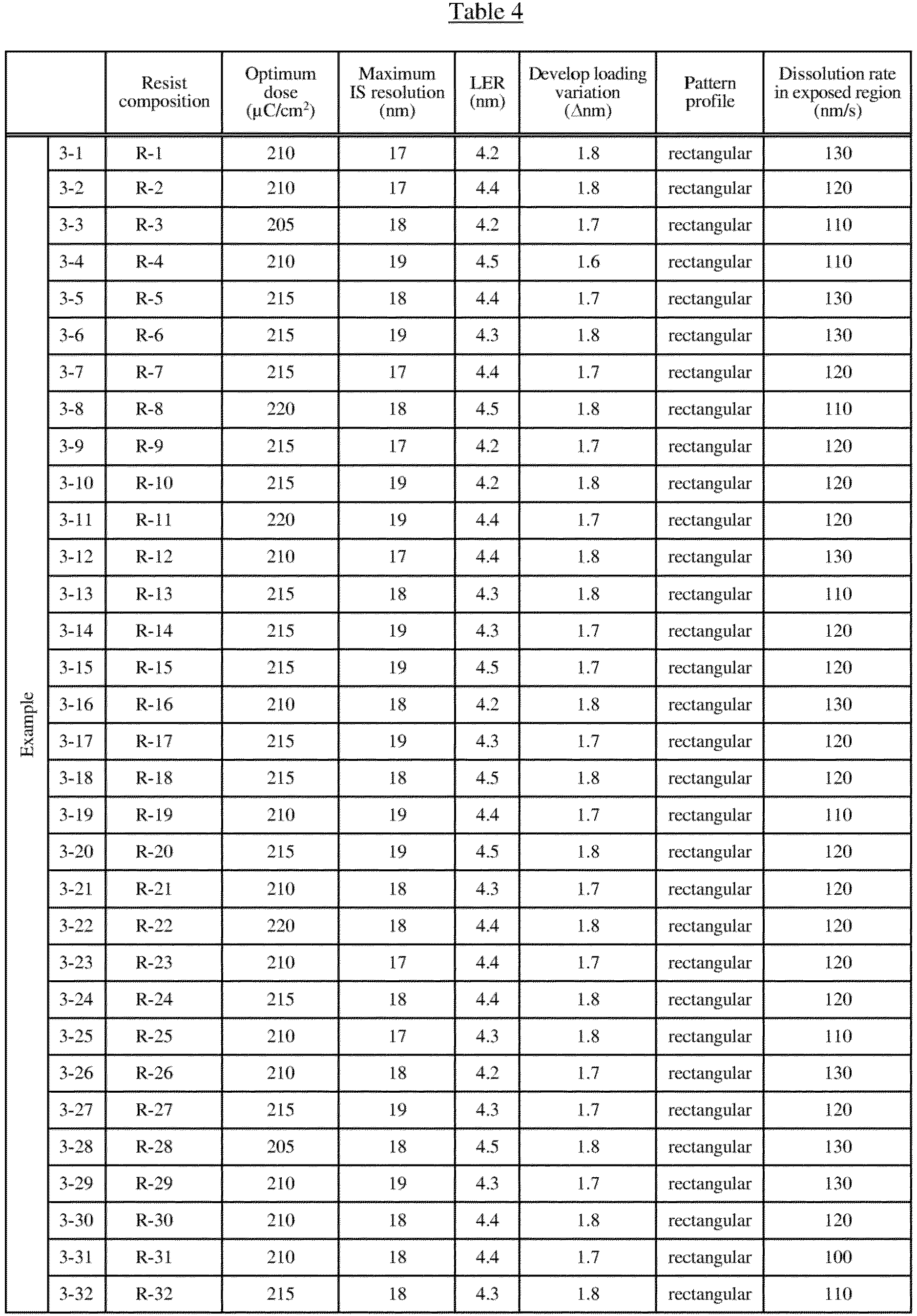

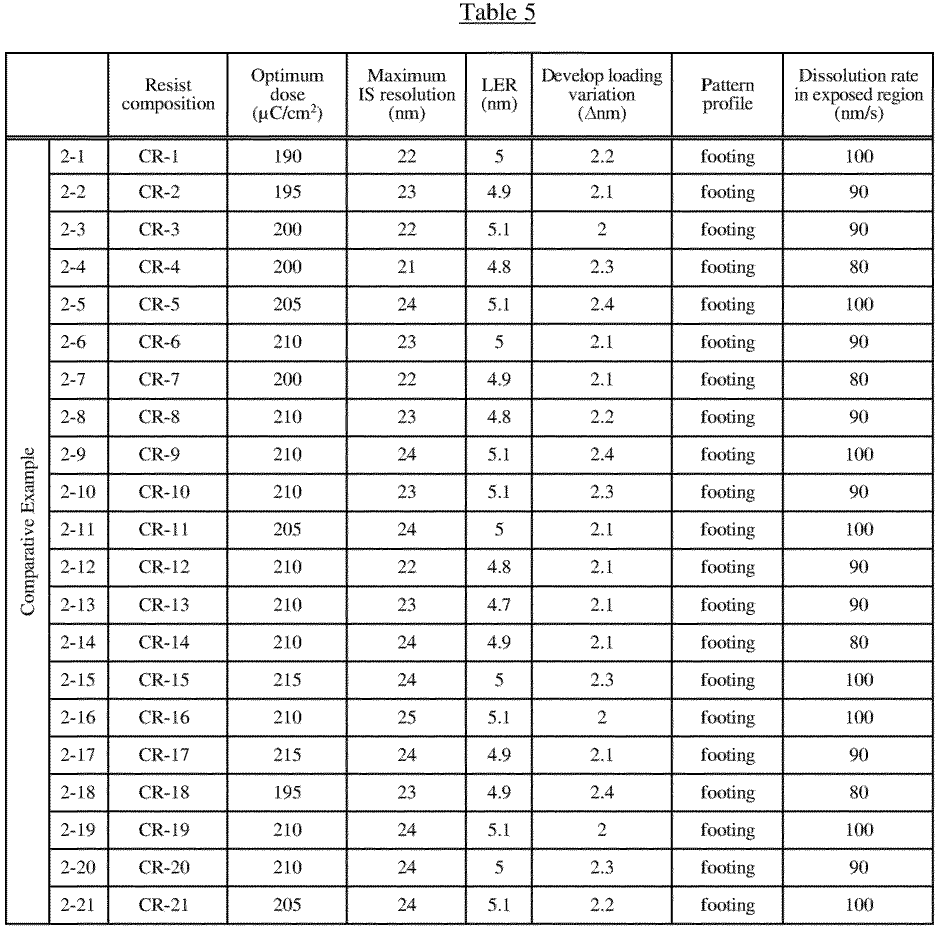

As is evident from Tables 4 and 5, the photoacid generator, the chemically amplified positive resist composition and the resist pattern forming process are effective in photolithography for the fabrication of semiconductor devices and the processing of photomask blanks of transmission and reflection types.

## Claims

1. An onium salt having the formula (A): 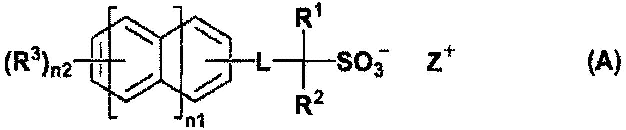 wherein n1 is an integer of 0 to 2, n2 is an integer of 2 to 5 in case of n1=0, an integer of 2 7 in case of n1=1, and an integer of 2 to 9 in case of n1=2,
L is a single bond, ether bond, ester bond, sulfonic ester bond, carbonate bond or carbamate bond,
R¹ and R² are each independently hydrogen or a C₃-C₂₀ branched or cyclic hydrocarbyl group which may contain a heteroatom, excluding that both R¹ and R² are hydrogen, R¹ and R² may bond together to form a ring with the carbon atom to which they are attached,
R³ is each independently iodine or a C₃-C₂₀ branched or cyclic hydrocarbyl group which may contain a heteroatom, at least one R³ is attached to a carbon atom adjoining the carbon atom to which L is attached, and
Z⁺ is an onium cation.

2. The onium salt of claim 1 having the formula (A1): 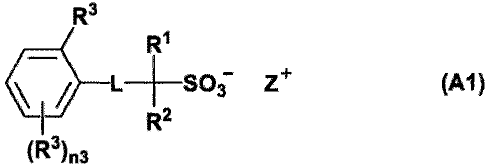 wherein L, R¹, R², R³, and Z⁺ are as defined above, and n3 is an integer of 1 to 4.

3. The onium salt of claim 1 wherein Z⁺ is an onium cation having the formula (cation-1) or (cation-2): 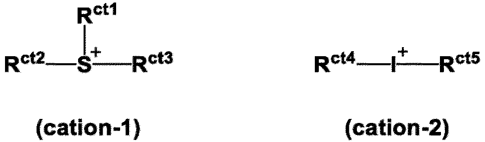 wherein R^{ct1} to R^{ct5} are each independently a C₁-C₃₀ hydrocarbyl group which may contain a heteroatom, and R^{ct1} and R^{ct2} may bond together to form a ring with the sulfur atom to which they are attached.

4. A chemically amplified positive resist composition comprising a photoacid generator of any one of claims 1 to 3.

5. The chemically amplified positive resist composition of claim 4, further comprising a base polymer containing a polymer which is decomposed under the action of acid to increase its solubility in alkaline developer.

6. The chemically amplified positive resist composition of claim 5 wherein the polymer comprises repeat units having the formula (B1): 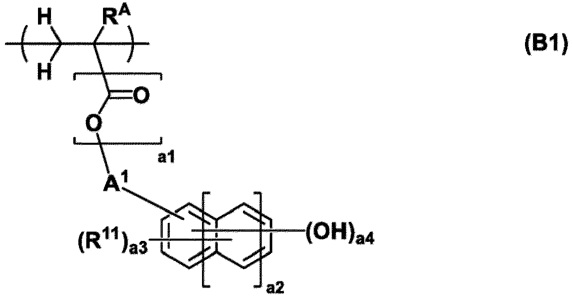 wherein a1 is 0 or 1, a2 is an integer of 0 to 2, a3 is an integer satisfying 0 ≤ a3 ≤ 5+2(a2)-a4, a4 is an integer of 1 to 3,
R^{A} is hydrogen, fluorine, methyl or trifluoromethyl,
R¹¹ is halogen, an optionally halogenated C₂-C₈ saturated hydrocarbylcarbonyloxy group, optionally halogenated C₁-C₆ saturated hydrocarbyl group, or optionally halogenated C₁-C₆ saturated hydrocarbyloxy group, and
A¹ is a single bond or C₁-C₁₀ saturated hydrocarbylene group in which some constituent - CH₂- may be replaced by -O-.

7. The chemically amplified positive resist composition of claim 5 or 6 wherein the polymer further comprises repeat units having the formula (B2-1) or (B2-2): 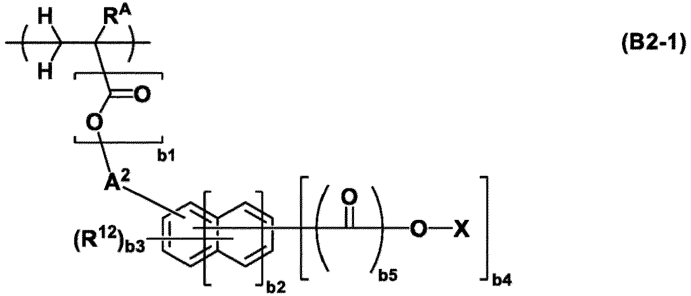 wherein b1 is 0 or 1, b2 is an integer of 0 to 2, b3 is an integer satisfying 0 ≤ b3 ≤ 5+2(b2)-b4, b4 is an integer of 1 to 3, b5 is 0 or 1,
R^{A} is hydrogen, fluorine, methyl or trifluoromethyl,
R¹² is halogen, an optionally halogenated C₂-C₈ saturated hydrocarbylcarbonyloxy group, optionally halogenated C₁-C₆ saturated hydrocarbyl group, or optionally halogenated C₁-C₆ saturated hydrocarbyloxy group,
A² is a single bond or C₁-C₁₀ saturated hydrocarbylene group in which some constituent - CH₂- may be replaced by -O-,
X is an acid labile group when b4 is 1, and X is each independently hydrogen or an acid labile group, at least one being an acid labile group, when b4 is 2 or 3, 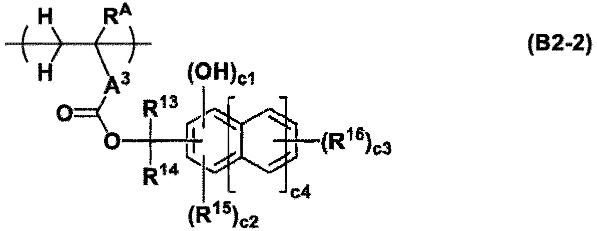 wherein c1 is an integer of 0 to 2, c2 is an integer of 0 to 2, c3 is an integer of 0 to 5, c4 is an integer of 0 to 2,
R^{A} is hydrogen, fluorine, methyl or trifluoromethyl,
R¹³ and R¹⁴ are each independently a C₁-C₁₀ hydrocarbyl group which may contain a heteroatom, R¹³ and R¹⁴ may bond together to form a ring with the carbon atom to which they are attached,
R¹⁵ is each independently fluorine, C₁-C₅ fluorinated alkyl group or C₁-C₅ fluorinated alkoxy group,
R¹⁶ is each independently a C₁-C₁₀ hydrocarbyl group which may contain a heteroatom, and
A³ is a single bond, phenylene group, naphthylene group, or *-C(=O)-O-A³¹-, wherein A³¹ is a C₁-C₂₀ aliphatic hydrocarbylene group which may contain hydroxy, ether bond, ester bond or lactone ring, or a phenylene or naphthylene group, * designates a point of attachment to the carbon atom in the backbone.

8. The chemically amplified positive resist composition of any one of claims 5 to 7 wherein the polymer further comprises repeat units of at least one type selected from repeat units having the formula (B3), repeat units having the formula (B4), and repeat units having the formula (B5): 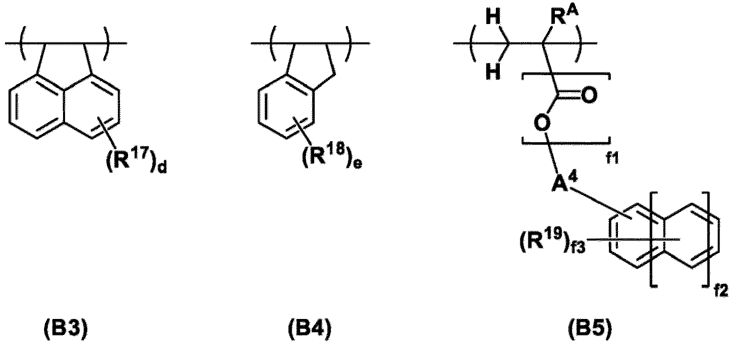 wherein d is an integer of 0 to 6, e is an integer of 0 to 4, f1 is 0 or 1, f2 is an integer of 0 to 2, and f3 is an integer of 0 to 5,
R^{A} is hydrogen, fluorine, methyl or trifluoromethyl,
R¹⁷ and R¹⁸ are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
R¹⁹ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro group, or cyano group, R¹⁹ may be a hydroxy group when f2 is 1 or 2, and
A⁴ is a single bond or C₁-C₁₀ saturated hydrocarbylene group in which some constituent - CH₂- may be replaced by -O-.

9. The chemically amplified positive resist composition of any one of claims 5 to 8, further comprising an organic solvent.

10. The chemically amplified positive resist composition of any one of claims 5 to 9, further comprising a fluorinated polymer comprising repeat units of at least one type selected from repeat units having the formula (D1), repeat units having the formula (D2), repeat units having the formula (D3) and repeat units having the formula (D4) and optionally repeat units of at least one type selected from repeat units having the formula (D5) and repeat units having the formula (D6): 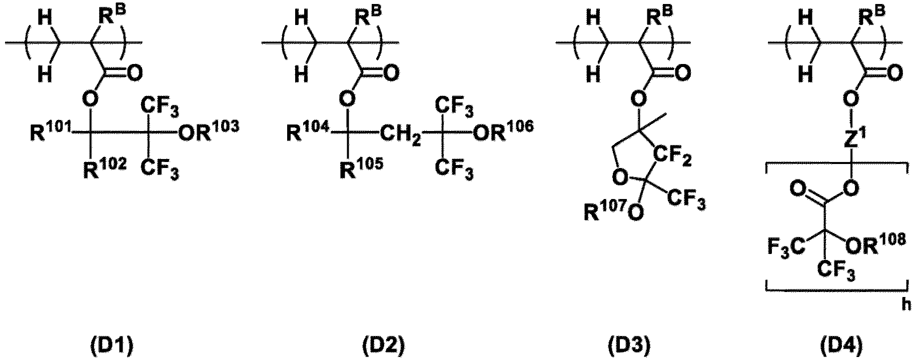 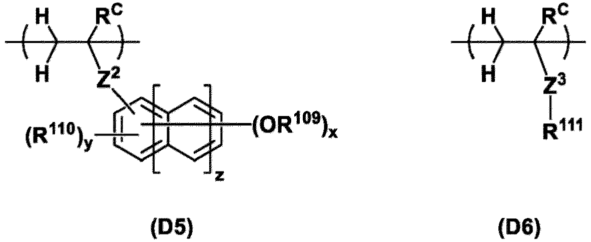 wherein x is an integer of 1 to 3, y is an integer satisfying 0 ≤ y ≤ 5+2z-x, z is 0 or 1, h is an integer of 1 to 3,
R^{B} is each independently hydrogen, fluorine, methyl or trifluoromethyl,
R^{C} is each independently hydrogen or methyl,
R¹⁰¹, R¹⁰², R¹⁰⁴ and R¹⁰⁵ are each independently hydrogen or a C₁-C₁₀ saturated hydrocarbyl group,
R¹⁰³, R¹⁰⁶, R¹⁰⁷ and R¹⁰⁸ are each independently hydrogen, a C₁-C₁₅ hydrocarbyl group, C₁-C₁₅ fluorinated hydrocarbyl group, or acid labile group, and when R¹⁰³, R¹⁰⁶, R¹⁰⁷ and R¹⁰⁸ each are a hydrocarbyl or fluorinated hydrocarbyl group, an ether bond or carbonyl moiety may intervene in a carbon-carbon bond,
R¹⁰⁹ is hydrogen or a C₁-C₅ straight or branched hydrocarbyl group in which a heteroatom-containing moiety may intervene in a carbon-carbon bond,
R¹¹⁰ is a C₁-C₅ straight or branched hydrocarbyl group in which a heteroatom-containing moiety may intervene in a carbon-carbon bond,
R¹¹¹ is a C₁-C₂₀ saturated hydrocarbyl group in which at least one hydrogen is substituted by fluorine, and in which some constituent -CH₂- may be replaced by an ester bond or ether bond,
Z¹ is a C₁-C₂₀ (h+1)-valent hydrocarbon group or C₁-C₂₀ (h+1)-valent fluorinated hydrocarbon group,
Z² is a single bond, *-C(=O)-O- or *-C(=O)-NH-, * designates a point of attachment to the carbon atom in the backbone,
Z³ is a single bond, -O-, *-C(=O)=O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond, or sulfonamide bond, and * designates a point of attachment to the carbon atom in the backbone.

11. The chemically amplified positive resist composition of any one of claims 5 to 10, further comprising a quencher.

12. A resist pattern forming process comprising the steps of:
applying the chemically amplified positive resist composition of any one of claims 4 to 11 onto a substrate to form a resist film thereon,
exposing the resist film to high-energy radiation, and
developing the exposed resist film in an alkaline developer.

13. The process of claim 12 wherein the high-energy radiation is EUV or EB.

14. The process of claim 12 or 13 wherein the substrate has the outermost surface of a chromium-containing material.

15. The process of any one of claims 12 to 14 wherein the substrate is a photomask blank.
